# EUROPEAN PATENT APPLICATION

(11) **EP 2 292 750 A2**
(43) Date of publication of application: **09.03.2011**
(21) Application number: 10191975.1
(22) Date of filing: 30.07.2004
(51) Int. Cl.: C12N 9/90, C12N 15/61, C12N 15/82, C07K 5/00, C07K 5/04, C12N 9/00

(54) **Alanine 2, 3 aminomutases**

(62) Divisional of application: 04786144.8
(71) Applicant: Novozymes A/S, 2880 Bagsvaerd (DK)
(72) Inventor: Jessen, Holly J., Chanhassen, MN 55317 (US); Gokarn, Ravi R., Oskaloosa, IA 52577 (US); Gort, Steven J., Apple Valley, MN 55124 (US); Selifonova, Olga V., Plymouth, MN 55446 (US); Liao, Hans H., Eden Prairie, MN 55347 (US); Brazeau, Brian J., Oskaloosa, IA 52577 (US)
(74) Representative: Gowshall, Jonathan Vallance

(57) **Abstract**

Alanine 2,3-aminomutase sequences are disclosed, as are cells having alanine 2,3-aminomutase activity and methods of selecting for such cells. Methods for producing beta-alanine, panthothenate, 3-hydroxypropionic acid, as well as other organic compounds, are disclosed.

SEQUENCE LISTING

<110>  Cargill Incorporated

<120>  Alanine 2,3 aminomutases

<130>  69200

<160>  59

<170>  PatentIn version 3.2

<210>  1
<211>  1416
<212>  DNA
<213>  Bacillus subtilis

<220>
<221>  CDS
<222>  (1)..(1416)

<400>  1
atg aaa aac aaa tgg tat aaa ccg aaa cgg cat tgg aag gag atc gag       48
Met Lys Asn Lys Trp Tyr Lys Pro Lys Arg His Trp Lys Glu Ile Glu
1               5                   10                  15

tta tgg aag gac gtt ccg gaa gag aaa tgg aac gat tgg ctt tgg cag       96
Leu Trp Lys Asp Val Pro Glu Glu Lys Trp Asn Asp Trp Leu Trp Gln
            20                  25                  30

ctg aca cac act gta aga acg tta gat gat tta aag aaa gtc att aat      144
Leu Thr His Thr Val Arg Thr Leu Asp Asp Leu Lys Lys Val Ile Asn
        35                  40                  45

ctg acc gag gat gaa gag gaa ggc gtc aga att tct acc aaa acg atc      192
Leu Thr Glu Asp Glu Glu Glu Gly Val Arg Ile Ser Thr Lys Thr Ile
    50                  55                  60

ccc tta aat att aca cct tac tat gct tct tta atg gac ccc gac aat      240
Pro Leu Asn Ile Thr Pro Tyr Tyr Ala Ser Leu Met Asp Pro Asp Asn
65                  70                  75                  80

ccg aga tgc ccg gta cgc atg cag tct gtg ccg ctt tct gaa gaa atg      288
Pro Arg Cys Pro Val Arg Met Gln Ser Val Pro Leu Ser Glu Glu Met
                85                  90                  95

cac aaa aca aaa tac gat atg gaa gac ccg ctt cat gag gat gaa gat      336
His Lys Thr Lys Tyr Asp Met Glu Asp Pro Leu His Glu Asp Glu Asp
            100                 105                 110

tca ccg gta ccc ggt ctg aca cac cgc tat ccc gac cgt gtg ctg ttt      384
Ser Pro Val Pro Gly Leu Thr His Arg Tyr Pro Asp Arg Val Leu Phe
        115                 120                 125

ctt gtc acg aat caa tgt tcc gtg tac tgc cgc tac tgc aca aga agg      432
Leu Val Thr Asn Gln Cys Ser Val Tyr Cys Arg Tyr Cys Thr Arg Arg
    130                 135                 140

cgc ttt tcc gga caa atc gga atg ggc gtc ccc aaa aaa cag ctt gat      480
Arg Phe Ser Gly Gln Ile Gly Met Gly Val Pro Lys Lys Gln Leu Asp

145                 150                 155                 160

gct gca att gct tat atc cgg gaa aca ccc gaa atc cgc gat tgt tta      528
Ala Ala Ile Ala Tyr Ile Arg Glu Thr Pro Glu Ile Arg Asp Cys Leu
                165                 170                 175

att tca ggc ggt gat ggg ctg ctc atc aac gac caa att tta gaa tat      576
Ile Ser Gly Gly Asp Gly Leu Leu Ile Asn Asp Gln Ile Leu Glu Tyr
            180                 185                 190

att tta aaa gag ctg cgc agc att ccg cat ctg gaa gtc atc aga atc      624
Ile Leu Lys Glu Leu Arg Ser Ile Pro His Leu Glu Val Ile Arg Ile
        195                 200                 205

gga aca aga gct ccc gtc gtc ttt ccg cag cgc att acc gat cat ctg      672
Gly Thr Arg Ala Pro Val Val Phe Pro Gln Arg Ile Thr Asp His Leu
    210                 215                 220

tgc gag ata ttg aaa aaa tat cat ccg gtc tgg ctg aac acc cat ttt      720
Cys Glu Ile Leu Lys Lys Tyr His Pro Val Trp Leu Asn Thr His Phe
225                 230                 235                 240

aac aca agc atc gaa atg aca gaa gaa tcc gtt gag gca tgt gaa aag      768
Asn Thr Ser Ile Glu Met Thr Glu Glu Ser Val Glu Ala Cys Glu Lys
                245                 250                 255

ctg gtg aac gcg gga gtg ccg gtc gga aat cag gct gtc gta tta gca      816
Leu Val Asn Ala Gly Val Pro Val Gly Asn Gln Ala Val Val Leu Ala
            260                 265                 270

ggt att aat gat tcg gtt cca att atg aaa aag ctc atg cat gac ttg      864
Gly Ile Asn Asp Ser Val Pro Ile Met Lys Lys Leu Met His Asp Leu
        275                 280                 285

gta aaa atc aga gtc cgt cct tat tat att tac caa tgt gat ctg tca      912
Val Lys Ile Arg Val Arg Pro Tyr Tyr Ile Tyr Gln Cys Asp Leu Ser
    290                 295                 300

gaa gga ata ggg cat ttc aga gct cct gtt tcc aaa ggt ttg gag atc      960
Glu Gly Ile Gly His Phe Arg Ala Pro Val Ser Lys Gly Leu Glu Ile
305                 310                 315                 320

att gaa ggg ctg aga ggt cat acc tca ggc tat gcg gtt cct acc ttt     1008
Ile Glu Gly Leu Arg Gly His Thr Ser Gly Tyr Ala Val Pro Thr Phe
                325                 330                 335

gtc gtt cac gca cca ggc gga gga ggt aaa atc gcc ctg cag ccg aac     1056
Val Val His Ala Pro Gly Gly Gly Gly Lys Ile Ala Leu Gln Pro Asn
            340                 345                 350

tat gtc ctg tca caa agt cct gac aaa gtg atc tta aga aat ttt gaa     1104
Tyr Val Leu Ser Gln Ser Pro Asp Lys Val Ile Leu Arg Asn Phe Glu
        355                 360                 365

ggt gtg att acg tca tat ccg gaa cca gag aat tat atc ccc aat cag     1152
Gly Val Ile Thr Ser Tyr Pro Glu Pro Glu Asn Tyr Ile Pro Asn Gln
    370                 375                 380

gca gac gcc tat ttt gag tcc gtt ttc cct gaa acc gct gac aaa aag     1200
Ala Asp Ala Tyr Phe Glu Ser Val Phe Pro Glu Thr Ala Asp Lys Lys
385                 390                 395                 400

gag ccg atc ggg ctg agt gcc att ttt gct gac aaa gaa gtt tcg ttt      1248
Glu Pro Ile Gly Leu Ser Ala Ile Phe Ala Asp Lys Glu Val Ser Phe
                405                 410                 415

aca cct gaa aat gta gac aga atc aaa agg aga gag gca tac atc gca      1296
Thr Pro Glu Asn Val Asp Arg Ile Lys Arg Arg Glu Ala Tyr Ile Ala
            420                 425                 430

aat ccg gag cat gaa aca tta aaa gat cgg cgt gag aaa aga gat cag      1344
Asn Pro Glu His Glu Thr Leu Lys Asp Arg Arg Glu Lys Arg Asp Gln
        435                 440                 445

ctc aaa gaa aag aaa ttt ttg gcg cag cag aaa aaa cag aaa gag act      1392
Leu Lys Glu Lys Lys Phe Leu Ala Gln Gln Lys Lys Gln Lys Glu Thr
    450                 455                 460

gaa tgc gga ggg gat tct tca tga                                      1416
Glu Cys Gly Gly Asp Ser Ser
465                 470

<210>  2
<211>  471
<212>  PRT
<213>  Bacillus subtilis

<400>  2

Met Lys Asn Lys Trp Tyr Lys Pro Lys Arg His Trp Lys Glu Ile Glu
1               5                   10                  15

Leu Trp Lys Asp Val Pro Glu Glu Lys Trp Asn Asp Trp Leu Trp Gln
            20                  25                  30

Leu Thr His Thr Val Arg Thr Leu Asp Asp Leu Lys Lys Val Ile Asn
        35                  40                  45

Leu Thr Glu Asp Glu Glu Glu Gly Val Arg Ile Ser Thr Lys Thr Ile
    50                  55                  60

Pro Leu Asn Ile Thr Pro Tyr Tyr Ala Ser Leu Met Asp Pro Asp Asn
65                  70                  75                  80

Pro Arg Cys Pro Val Arg Met Gln Ser Val Pro Leu Ser Glu Glu Met
                85                  90                  95

His Lys Thr Lys Tyr Asp Met Glu Asp Pro Leu His Glu Asp Glu Asp
            100                 105                 110

Ser Pro Val Pro Gly Leu Thr His Arg Tyr Pro Asp Arg Val Leu Phe
        115                 120                 125

Leu Val Thr Asn Gln Cys Ser Val Tyr Cys Arg Tyr Cys Thr Arg Arg
    130                 135                 140

Arg Phe Ser Gly Gln Ile Gly Met Gly Val Pro Lys Lys Gln Leu Asp
145                 150                 155                 160

Ala Ala Ile Ala Tyr Ile Arg Glu Thr Pro Glu Ile Arg Asp Cys Leu
                165                 170                 175

Ile Ser Gly Gly Asp Gly Leu Leu Ile Asn Asp Gln Ile Leu Glu Tyr
            180                 185                 190

Ile Leu Lys Glu Leu Arg Ser Ile Pro His Leu Glu Val Ile Arg Ile
        195                 200                 205

Gly Thr Arg Ala Pro Val Val Phe Pro Gln Arg Ile Thr Asp His Leu
    210                 215                 220

Cys Glu Ile Leu Lys Lys Tyr His Pro Val Trp Leu Asn Thr His Phe
225                 230                 235                 240

Asn Thr Ser Ile Glu Met Thr Glu Glu Ser Val Glu Ala Cys Glu Lys
                245                 250                 255

Leu Val Asn Ala Gly Val Pro Val Gly Asn Gln Ala Val Val Leu Ala
            260                 265                 270

Gly Ile Asn Asp Ser Val Pro Ile Met Lys Lys Leu Met His Asp Leu
        275                 280                 285

Val Lys Ile Arg Val Arg Pro Tyr Tyr Ile Tyr Gln Cys Asp Leu Ser
    290                 295                 300

Glu Gly Ile Gly His Phe Arg Ala Pro Val Ser Lys Gly Leu Glu Ile
305                 310                 315                 320

Ile Glu Gly Leu Arg Gly His Thr Ser Gly Tyr Ala Val Pro Thr Phe
                325                 330                 335

Val Val His Ala Pro Gly Gly Gly Gly Lys Ile Ala Leu Gln Pro Asn
            340                 345                 350

Tyr Val Leu Ser Gln Ser Pro Asp Lys Val Ile Leu Arg Asn Phe Glu
        355                 360                 365

Gly Val Ile Thr Ser Tyr Pro Glu Pro Glu Asn Tyr Ile Pro Asn Gln

370                 375                 380

Ala Asp Ala Tyr Phe Glu Ser Val Phe Pro Glu Thr Ala Asp Lys Lys
385                 390                 395                 400

Glu Pro Ile Gly Leu Ser Ala Ile Phe Ala Asp Lys Glu Val Ser Phe
                405                 410                 415

Thr Pro Glu Asn Val Asp Arg Ile Lys Arg Arg Glu Ala Tyr Ile Ala
            420                 425                 430

Asn Pro Glu His Glu Thr Leu Lys Asp Arg Arg Glu Lys Arg Asp Gln
        435                 440                 445

Leu Lys Glu Lys Lys Phe Leu Ala Gln Gln Lys Lys Gln Lys Glu Thr
    450                 455                 460

Glu Cys Gly Gly Asp Ser Ser
465                 470

<210>  3
<211>  1416
<212>  DNA
<213>  Bacillus subtilis

<220>
<221>  CDS
<222>  (1)..(1416)

<400>  3
atg aaa aac aaa tgg tat aaa ccg aaa cgg cat tgg aag gag atc gag       48
Met Lys Asn Lys Trp Tyr Lys Pro Lys Arg His Trp Lys Glu Ile Glu
1               5                   10                  15

tta tgg aag gac gtt ccg gaa gag aaa tgg aac gat tgg ctt tgg cag       96
Leu Trp Lys Asp Val Pro Glu Glu Lys Trp Asn Asp Trp Leu Trp Gln
            20                  25                  30

ctg aca cac act gta aga acg tta gat gat tta aag aaa gtc att aat      144
Leu Thr His Thr Val Arg Thr Leu Asp Asp Leu Lys Lys Val Ile Asn
        35                  40                  45

ctg acc gag gat gaa gag gaa ggc gtc cgt att tct acc aaa acg atc      192
Leu Thr Glu Asp Glu Glu Glu Gly Val Arg Ile Ser Thr Lys Thr Ile
    50                  55                  60

ccc tta aat att aca cct tac tat gct tct tta atg gac ccc gac aat      240
Pro Leu Asn Ile Thr Pro Tyr Tyr Ala Ser Leu Met Asp Pro Asp Asn
65                  70                  75                  80

ccg aga tgc ccg gta cgc atg cag tct gtg ccg ctt tct gaa gaa atg      288
Pro Arg Cys Pro Val Arg Met Gln Ser Val Pro Leu Ser Glu Glu Met
                85                  90                  95

cac aaa aca aaa tac gat atg gaa gac ccg ctt cat gag gat gaa gat 336
His Lys Thr Lys Tyr Asp Met Glu Asp Pro Leu His Glu Asp Glu Asp
            100                 105                 110

tca ccg gta ccc ggt ctg aca cac cgc tat ccc gac cgt gtg ctg ttt 384
Ser Pro Val Pro Gly Leu Thr His Arg Tyr Pro Asp Arg Val Leu Phe
        115                 120                 125

ctt gtc acg aat caa tgt tcc gtg tac tgc cgc cac tgc aca cgc cgg 432
Leu Val Thr Asn Gln Cys Ser Val Tyr Cys Arg His Cys Thr Arg Arg
    130                 135                 140

cgc ttt tcc gga caa atc gga atg ggc gtc ccc aaa aaa cag ctt gat 480
Arg Phe Ser Gly Gln Ile Gly Met Gly Val Pro Lys Lys Gln Leu Asp
145                 150                 155                 160

gct gca att gct tat atc cgg gaa aca ccc gaa atc cgc gat tgt tta 528
Ala Ala Ile Ala Tyr Ile Arg Glu Thr Pro Glu Ile Arg Asp Cys Leu
                165                 170                 175

att tca ggc ggt gat ggg ctg ctc atc aac gac caa att tta gaa tat 576
Ile Ser Gly Gly Asp Gly Leu Leu Ile Asn Asp Gln Ile Leu Glu Tyr
            180                 185                 190

att tta aaa gag ctg cgc agc att ccg cat ctg gaa gtc atc cgc atc 624
Ile Leu Lys Glu Leu Arg Ser Ile Pro His Leu Glu Val Ile Arg Ile
        195                 200                 205

gga aca cgt gct ccc gtc gtc ttt ccg cag cgc att acc gat cat ctg 672
Gly Thr Arg Ala Pro Val Val Phe Pro Gln Arg Ile Thr Asp His Leu
    210                 215                 220

tgc gag ata ttg aaa aaa tat cat ccg gtc tgg ctg aac acc cat ttt 720
Cys Glu Ile Leu Lys Lys Tyr His Pro Val Trp Leu Asn Thr His Phe
225                 230                 235                 240

aac aca agc atc gaa atg aca gaa gaa tcc gtt gag gca tgt gaa aag 768
Asn Thr Ser Ile Glu Met Thr Glu Glu Ser Val Glu Ala Cys Glu Lys
                245                 250                 255

ctg gtg aac gcg gga gtg ccg gtc gga aat cag gct gtc gta tta gca 816
Leu Val Asn Ala Gly Val Pro Val Gly Asn Gln Ala Val Val Leu Ala
            260                 265                 270

ggt att aat gat tcg gtt cca att atg aaa aag ctc atg cat gac ttg 864
Gly Ile Asn Asp Ser Val Pro Ile Met Lys Lys Leu Met His Asp Leu
        275                 280                 285

gta aaa atc aga gtc cgt cct tat tat att tac caa tgt gat ctg tca 912
Val Lys Ile Arg Val Arg Pro Tyr Tyr Ile Tyr Gln Cys Asp Leu Ser
    290                 295                 300

gaa gga ata ggg cat ttc cgt gct cct gtt tcc aaa ggt ttg gag atc 960
Glu Gly Ile Gly His Phe Arg Ala Pro Val Ser Lys Gly Leu Glu Ile
305                 310                 315                 320

att gaa ggg ctg aga ggt cat acc tca ggc tat gcg gtt cct acc ttt 1008
Ile Glu Gly Leu Arg Gly His Thr Ser Gly Tyr Ala Val Pro Thr Phe
                325                 330                 335

gtc gtt cac gca cca ggc gga gga ggt aaa atc gcc ctg cag ccg aac     1056
Val Val His Ala Pro Gly Gly Gly Gly Lys Ile Ala Leu Gln Pro Asn
            340                 345                 350

tat gtc ctg tca caa agt cct gac aaa gtg atc tta aga aat ttt gaa     1104
Tyr Val Leu Ser Gln Ser Pro Asp Lys Val Ile Leu Arg Asn Phe Glu
        355                 360                 365

ggt gtg att acg tca tat ccg gaa cca gag aat tat atc ccc aat cag     1152
Gly Val Ile Thr Ser Tyr Pro Glu Pro Glu Asn Tyr Ile Pro Asn Gln
    370                 375                 380

gca gac gcc tat ttt gag tcc gtt ttc cct gaa acc gct gac aaa aag     1200
Ala Asp Ala Tyr Phe Glu Ser Val Phe Pro Glu Thr Ala Asp Lys Lys
385                 390                 395                 400

gag ccg atc ggg ctg agt gcc att ttt gct gac aaa gaa gtt tcg ttt     1248
Glu Pro Ile Gly Leu Ser Ala Ile Phe Ala Asp Lys Glu Val Ser Phe
                405                 410                 415

aca cct gaa aat gta gac aga atc aaa cgg cgt gag gca tac atc gca     1296
Thr Pro Glu Asn Val Asp Arg Ile Lys Arg Arg Glu Ala Tyr Ile Ala
            420                 425                 430

aat ccg gag cat gaa aca tta aaa gat cgg cgt gag aaa aga gat cag     1344
Asn Pro Glu His Glu Thr Leu Lys Asp Arg Arg Glu Lys Arg Asp Gln
        435                 440                 445

ctc aaa gaa aag aaa ttt ttg gcg cag cag aaa aaa cag aaa gag act     1392
Leu Lys Glu Lys Lys Phe Leu Ala Gln Gln Lys Lys Gln Lys Glu Thr
    450                 455                 460

gaa tgc gga ggg gat tct tca tga                                     1416
Glu Cys Gly Gly Asp Ser Ser
465                 470


<210>  4
<211>  471
<212>  PRT
<213>  Bacillus subtilis

<400>  4

Met Lys Asn Lys Trp Tyr Lys Pro Lys Arg His Trp Lys Glu Ile Glu
1               5                   10                  15


Leu Trp Lys Asp Val Pro Glu Glu Lys Trp Asn Asp Trp Leu Trp Gln
            20                  25                  30


Leu Thr His Thr Val Arg Thr Leu Asp Asp Leu Lys Lys Val Ile Asn
        35                  40                  45


Leu Thr Glu Asp Glu Glu Glu Gly Val Arg Ile Ser Thr Lys Thr Ile
    50                  55                  60


Pro Leu Asn Ile Thr Pro Tyr Tyr Ala Ser Leu Met Asp Pro Asp Asn

65                  70                  75                  80

Pro Arg Cys Pro Val Arg Met Gln Ser Val Pro Leu Ser Glu Glu Met
                85                  90                  95

His Lys Thr Lys Tyr Asp Met Glu Asp Pro Leu His Glu Asp Glu Asp
            100                 105                 110

Ser Pro Val Pro Gly Leu Thr His Arg Tyr Pro Asp Arg Val Leu Phe
        115                 120                 125

Leu Val Thr Asn Gln Cys Ser Val Tyr Cys Arg His Cys Thr Arg Arg
    130                 135                 140

Arg Phe Ser Gly Gln Ile Gly Met Gly Val Pro Lys Lys Gln Leu Asp
145                 150                 155                 160

Ala Ala Ile Ala Tyr Ile Arg Glu Thr Pro Glu Ile Arg Asp Cys Leu
                165                 170                 175

Ile Ser Gly Gly Asp Gly Leu Leu Ile Asn Asp Gln Ile Leu Glu Tyr
            180                 185                 190

Ile Leu Lys Glu Leu Arg Ser Ile Pro His Leu Glu Val Ile Arg Ile
        195                 200                 205

Gly Thr Arg Ala Pro Val Val Phe Pro Gln Arg Ile Thr Asp His Leu
    210                 215                 220

Cys Glu Ile Leu Lys Lys Tyr His Pro Val Trp Leu Asn Thr His Phe
225                 230                 235                 240

Asn Thr Ser Ile Glu Met Thr Glu Glu Ser Val Glu Ala Cys Glu Lys
                245                 250                 255

Leu Val Asn Ala Gly Val Pro Val Gly Asn Gln Ala Val Val Leu Ala
            260                 265                 270

Gly Ile Asn Asp Ser Val Pro Ile Met Lys Lys Leu Met His Asp Leu
        275                 280                 285

Val Lys Ile Arg Val Arg Pro Tyr Tyr Ile Tyr Gln Cys Asp Leu Ser
    290                 295                 300

Glu Gly Ile Gly His Phe Arg Ala Pro Val Ser Lys Gly Leu Glu Ile
305                 310                 315                 320

Ile Glu Gly Leu Arg Gly His Thr Ser Gly Tyr Ala Val Pro Thr Phe
                325                 330                 335

Val Val His Ala Pro Gly Gly Gly Gly Lys Ile Ala Leu Gln Pro Asn
            340                 345                 350

Tyr Val Leu Ser Gln Ser Pro Asp Lys Val Ile Leu Arg Asn Phe Glu
        355                 360                 365

Gly Val Ile Thr Ser Tyr Pro Glu Pro Glu Asn Tyr Ile Pro Asn Gln
    370                 375                 380

Ala Asp Ala Tyr Phe Glu Ser Val Phe Pro Glu Thr Ala Asp Lys Lys
385                 390                 395                 400

Glu Pro Ile Gly Leu Ser Ala Ile Phe Ala Asp Lys Glu Val Ser Phe
                405                 410                 415

Thr Pro Glu Asn Val Asp Arg Ile Lys Arg Arg Glu Ala Tyr Ile Ala
            420                 425                 430

Asn Pro Glu His Glu Thr Leu Lys Asp Arg Arg Glu Lys Arg Asp Gln
        435                 440                 445

Leu Lys Glu Lys Lys Phe Leu Ala Gln Gln Lys Lys Gln Lys Glu Thr
    450                 455                 460

Glu Cys Gly Gly Asp Ser Ser
465                 470

<210>  5
<211>  1251
<212>  DNA
<213>  Porphyromonas gingivalis

<220>
<221>  CDS
<222>  (1)..(1251)

<400>  5
atg gca gaa agt cgt aga aag tat tat ttc cct gat gtc acc gat gag       48
Met Ala Glu Ser Arg Arg Lys Tyr Tyr Phe Pro Asp Val Thr Asp Glu
1               5                   10                  15

caa tgg tac gac tgg cat tgg cag gtc ctc aat cga att gag acg ctc       96
Gln Trp Tyr Asp Trp His Trp Gln Val Leu Asn Arg Ile Glu Thr Leu
            20                  25                  30

gac cag ctg aaa aag tac gtt aca ctc acc gct gaa gaa gaa gag gga 144
Asp Gln Leu Lys Lys Tyr Val Thr Leu Thr Ala Glu Glu Glu Glu Gly
        35                  40                  45

gta aaa gaa tcg ccc aaa gta ctc cga atg gct atc aca cct tat tat 192
Val Lys Glu Ser Pro Lys Val Leu Arg Met Ala Ile Thr Pro Tyr Tyr
    50                  55                  60

ttg agt ttg ata gac ccc gag aat cct aat tgt ccg att cgt aaa caa 240
Leu Ser Leu Ile Asp Pro Glu Asn Pro Asn Cys Pro Ile Arg Lys Gln
65                  70                  75                  80

gcc att cct act caa cag gaa ctg gta cgt gct cct gaa gat cag gta 288
Ala Ile Pro Thr Gln Gln Glu Leu Val Arg Ala Pro Glu Asp Gln Val
                85                  90                  95

gac cca ctt agt gaa gat gaa gat tcg ccc gta ccc gga ctg act cat 336
Asp Pro Leu Ser Glu Asp Glu Asp Ser Pro Val Pro Gly Leu Thr His
            100                 105                 110

cgt tat ccg gat cgt gta ttg ttc ctt atc acg gac aaa tgt tcg atg 384
Arg Tyr Pro Asp Arg Val Leu Phe Leu Ile Thr Asp Lys Cys Ser Met
        115                 120                 125

tac tgt cgt cat tgt act cgc cgt cgc ttc gca gga cag aaa gat gct 432
Tyr Cys Arg His Cys Thr Arg Arg Arg Phe Ala Gly Gln Lys Asp Ala
    130                 135                 140

tct tct cct tct gag cgc atc gat cga tgc att gac tat ata gcc aat 480
Ser Ser Pro Ser Glu Arg Ile Asp Arg Cys Ile Asp Tyr Ile Ala Asn
145                 150                 155                 160

aca ccg aca gtc cgc gat gtt ttg cta tcg gga ggc gat gcc ctc ctt 528
Thr Pro Thr Val Arg Asp Val Leu Leu Ser Gly Gly Asp Ala Leu Leu
                165                 170                 175

gtc agc gac gaa cgc ttg gaa tac ata ttg aag cgt ctg cgc gaa ata 576
Val Ser Asp Glu Arg Leu Glu Tyr Ile Leu Lys Arg Leu Arg Glu Ile
            180                 185                 190

cct cat gtg gag att gtt cgt ata gga agc cgt acg ccg gta gtc ctc 624
Pro His Val Glu Ile Val Arg Ile Gly Ser Arg Thr Pro Val Val Leu
        195                 200                 205

cct cag cgt ata acg cct caa ttg gtg gat atg ctc aaa aaa tat cat 672
Pro Gln Arg Ile Thr Pro Gln Leu Val Asp Met Leu Lys Lys Tyr His
    210                 215                 220

ccg gtg tgg ctg aac act cac ttc aac cac ccg aat gaa gtt acc gaa 720
Pro Val Trp Leu Asn Thr His Phe Asn His Pro Asn Glu Val Thr Glu
225                 230                 235                 240

gaa gca gta gag gct tgt gaa aga atg gcc aat gcc ggt att ccg ttg 768
Glu Ala Val Glu Ala Cys Glu Arg Met Ala Asn Ala Gly Ile Pro Leu
                245                 250                 255

ggt aac caa acg gtt tta ttg cgt gga atc aat gat tgt aca cat gtg 816
Gly Asn Gln Thr Val Leu Leu Arg Gly Ile Asn Asp Cys Thr His Val
            260                 265                 270

atg aag aga ttg gta cat ttg ctg gta aag atg cgt gtg cgt cct tac 864

Met Lys Arg Leu Val His Leu Leu Val Lys Met Arg Val Arg Pro Tyr
        275                 280                 285

tat ata tat gta tgc gat ctt tcg ctt gga ata ggt cat ttc cgc acg      912
Tyr Ile Tyr Val Cys Asp Leu Ser Leu Gly Ile Gly His Phe Arg Thr
    290                 295                 300

ccg gta tct aaa gga atc gaa att atc gaa aat ttg cgc gga cac acc      960
Pro Val Ser Lys Gly Ile Glu Ile Ile Glu Asn Leu Arg Gly His Thr
305                 310                 315                 320

tcg ggc tat gca gtt cct acc ttt gtg gta ggt gct ccg ggg ggt ggt     1008
Ser Gly Tyr Ala Val Pro Thr Phe Val Val Gly Ala Pro Gly Gly Gly
                325                 330                 335

ggt aag ata cct gta acg ccg aac tat gtt gta tct cag tcc cca cga     1056
Gly Lys Ile Pro Val Thr Pro Asn Tyr Val Val Ser Gln Ser Pro Arg
            340                 345                 350

cat gtg gtt ctt cgc aat tat gaa ggt gtt atc aca acc tat acg gag     1104
His Val Val Leu Arg Asn Tyr Glu Gly Val Ile Thr Thr Tyr Thr Glu
        355                 360                 365

ccg gag aat tat cat gag gag tgc gat tgt gag gac tgt cga gcc ggt     1152
Pro Glu Asn Tyr His Glu Glu Cys Asp Cys Glu Asp Cys Arg Ala Gly
    370                 375                 380

aag cat aaa gag ggt gta gct gca ctt tcc gga ggt cag cag ttg gct     1200
Lys His Lys Glu Gly Val Ala Ala Leu Ser Gly Gly Gln Gln Leu Ala
385                 390                 395                 400

atc gag cct tcc gac tta gct cgc aaa aaa cgc aag ttt gat aag aac     1248
Ile Glu Pro Ser Asp Leu Ala Arg Lys Lys Arg Lys Phe Asp Lys Asn
                405                 410                 415

tga                                                                 1251


<210>  6
<211>  416
<212>  PRT
<213>  Porphyromonas gingivalis

<400>  6

Met Ala Glu Ser Arg Arg Lys Tyr Tyr Phe Pro Asp Val Thr Asp Glu
1               5                   10                  15


Gln Trp Tyr Asp Trp His Trp Gln Val Leu Asn Arg Ile Glu Thr Leu
            20                  25                  30


Asp Gln Leu Lys Lys Tyr Val Thr Leu Thr Ala Glu Glu Glu Glu Gly
        35                  40                  45


Val Lys Glu Ser Pro Lys Val Leu Arg Met Ala Ile Thr Pro Tyr Tyr
    50                  55                  60

Leu Ser Leu Ile Asp Pro Glu Asn Pro Asn Cys Pro Ile Arg Lys Gln
65                  70                  75                  80

Ala Ile Pro Thr Gln Gln Glu Leu Val Arg Ala Pro Glu Asp Gln Val
                85                  90                  95

Asp Pro Leu Ser Glu Asp Glu Asp Ser Pro Val Pro Gly Leu Thr His
            100                 105                 110

Arg Tyr Pro Asp Arg Val Leu Phe Leu Ile Thr Asp Lys Cys Ser Met
        115                 120                 125

Tyr Cys Arg His Cys Thr Arg Arg Arg Phe Ala Gly Gln Lys Asp Ala
    130                 135                 140

Ser Ser Pro Ser Glu Arg Ile Asp Arg Cys Ile Asp Tyr Ile Ala Asn
145                 150                 155                 160

Thr Pro Thr Val Arg Asp Val Leu Leu Ser Gly Gly Asp Ala Leu Leu
                165                 170                 175

Val Ser Asp Glu Arg Leu Glu Tyr Ile Leu Lys Arg Leu Arg Glu Ile
            180                 185                 190

Pro His Val Glu Ile Val Arg Ile Gly Ser Arg Thr Pro Val Val Leu
        195                 200                 205

Pro Gln Arg Ile Thr Pro Gln Leu Val Asp Met Leu Lys Lys Tyr His
    210                 215                 220

Pro Val Trp Leu Asn Thr His Phe Asn His Pro Asn Glu Val Thr Glu
225                 230                 235                 240

Glu Ala Val Glu Ala Cys Glu Arg Met Ala Asn Ala Gly Ile Pro Leu
                245                 250                 255

Gly Asn Gln Thr Val Leu Leu Arg Gly Ile Asn Asp Cys Thr His Val
            260                 265                 270

Met Lys Arg Leu Val His Leu Leu Val Lys Met Arg Val Arg Pro Tyr
        275                 280                 285

Tyr Ile Tyr Val Cys Asp Leu Ser Leu Gly Ile Gly His Phe Arg Thr
    290                 295                 300

Pro Val Ser Lys Gly Ile Glu Ile Ile Glu Asn Leu Arg Gly His Thr

305                 310                 315                 320

Ser Gly Tyr Ala Val Pro Thr Phe Val Val Gly Ala Pro Gly Gly Gly
                325                 330                 335

Gly Lys Ile Pro Val Thr Pro Asn Tyr Val Val Ser Gln Ser Pro Arg
            340                 345                 350

His Val Val Leu Arg Asn Tyr Glu Gly Val Ile Thr Thr Tyr Thr Glu
        355                 360                 365

Pro Glu Asn Tyr His Glu Glu Cys Asp Cys Glu Asp Cys Arg Ala Gly
    370                 375                 380

Lys His Lys Glu Gly Val Ala Ala Leu Ser Gly Gly Gln Gln Leu Ala
385                 390                 395                 400

Ile Glu Pro Ser Asp Leu Ala Arg Lys Lys Arg Lys Phe Asp Lys Asn
                405                 410                 415

<210>  7
<211>  29
<212>  DNA
<213>  Artificial

<220>
<223>  PCR primer

<400>  7
ccggcccata tgaatacagt taatactag                                        29

<210>  8
<211>  37
<212>  DNA
<213>  Artificial

<220>
<223>  PCR primer

<400>  8
cgccgcggat ccttatttaa acaatctctc cctgtcg                               37

<210>  9
<211>  1278
<212>  DNA
<213>  Fusobacterium nucleatum

<220>
<221>  CDS
<222>  (1)..(1278)

<400> 9
atg aat aca gtt aat act aga aaa aaa ttt ttc cca aat gta act gat 48
Met Asn Thr Val Asn Thr Arg Lys Lys Phe Phe Pro Asn Val Thr Asp
1               5                   10                  15

gaa gaa tgg aat gat tgg aca tgg caa gta aaa aac aga ctt gaa agt 96
Glu Glu Trp Asn Asp Trp Thr Trp Gln Val Lys Asn Arg Leu Glu Ser
            20                  25                  30

gtt gaa gat tta aaa aaa tat gtt gat tta agt gaa gaa gaa aca gaa 144
Val Glu Asp Leu Lys Lys Tyr Val Asp Leu Ser Glu Glu Glu Thr Glu
        35                  40                  45

ggg gtt gta aga act ctt gaa act tta aga atg gca atc act cca tat 192
Gly Val Val Arg Thr Leu Glu Thr Leu Arg Met Ala Ile Thr Pro Tyr
    50                  55                  60

tac ttc tca ttg ata gat ttg aat agt gat aga tgc cca ata aga aag 240
Tyr Phe Ser Leu Ile Asp Leu Asn Ser Asp Arg Cys Pro Ile Arg Lys
65                  70                  75                  80

caa gct ata cct act ata caa gaa ata cat caa tct gat gct gat ttg 288
Gln Ala Ile Pro Thr Ile Gln Glu Ile His Gln Ser Asp Ala Asp Leu
                85                  90                  95

tta gat cct cta cat gaa gat gaa gac tct cca gta cca gga tta act 336
Leu Asp Pro Leu His Glu Asp Glu Asp Ser Pro Val Pro Gly Leu Thr
            100                 105                 110

cat aga tat cca gat aga gtt tta ctt cta ata aca gac atg tgt tct 384
His Arg Tyr Pro Asp Arg Val Leu Leu Leu Ile Thr Asp Met Cys Ser
        115                 120                 125

atg tat tgt aga cac tgc act cgt aga aga ttt gct ggg tca agt gat 432
Met Tyr Cys Arg His Cys Thr Arg Arg Arg Phe Ala Gly Ser Ser Asp
    130                 135                 140

gat gct atg cct atg gat aga att gac aaa gca ata gaa tat att gca 480
Asp Ala Met Pro Met Asp Arg Ile Asp Lys Ala Ile Glu Tyr Ile Ala
145                 150                 155                 160

aaa act cca caa gta agg gat gta ttg tta tca gga gga gat gca ctt 528
Lys Thr Pro Gln Val Arg Asp Val Leu Leu Ser Gly Gly Asp Ala Leu
                165                 170                 175

cta gtt tct gat aaa aaa tta gaa agc ata atc caa aaa cta aga gca 576
Leu Val Ser Asp Lys Lys Leu Glu Ser Ile Ile Gln Lys Leu Arg Ala
            180                 185                 190

ata cct cat gtt gaa ata ata aga ata gga agt aga aca cca gtt gtt 624
Ile Pro His Val Glu Ile Ile Arg Ile Gly Ser Arg Thr Pro Val Val
        195                 200                 205

tta cct caa aga att act cct gaa tta tgt aat atg tta aag aaa tat 672
Leu Pro Gln Arg Ile Thr Pro Glu Leu Cys Asn Met Leu Lys Lys Tyr
    210                 215                 220

cat cca att tgg ttg aat act cat ttt aac cac cct caa gaa gta aca 720
His Pro Ile Trp Leu Asn Thr His Phe Asn His Pro Gln Glu Val Thr
225                 230                 235                 240

cca gaa gct aaa aaa gct tgt gaa atg ttg gca gat gca gga gtt cca      768
Pro Glu Ala Lys Lys Ala Cys Glu Met Leu Ala Asp Ala Gly Val Pro
                245                 250                 255

tta gga aat caa act gta cta tta aga gga ata aat gac agt gta cct      816
Leu Gly Asn Gln Thr Val Leu Leu Arg Gly Ile Asn Asp Ser Val Pro
            260                 265                 270

gta atg aaa agg tta gta cat gat tta gta atg atg aga gta aga cct      864
Val Met Lys Arg Leu Val His Asp Leu Val Met Met Arg Val Arg Pro
        275                 280                 285

tat tat att tac caa tgt gac tta tct atg gga ctt gaa cac ttc aga      912
Tyr Tyr Ile Tyr Gln Cys Asp Leu Ser Met Gly Leu Glu His Phe Arg
    290                 295                 300

aca cca gtt tct aaa ggt ata gaa att att gaa gga tta aga gga cat      960
Thr Pro Val Ser Lys Gly Ile Glu Ile Ile Glu Gly Leu Arg Gly His
305                 310                 315                 320

aca tct gga tat gca gta cca aca ttt gtt gtt gat gca cct ggt ggt     1008
Thr Ser Gly Tyr Ala Val Pro Thr Phe Val Val Asp Ala Pro Gly Gly
                325                 330                 335

gga gga aaa act cca gta atg cct caa tat gta att tct caa tct cct     1056
Gly Gly Lys Thr Pro Val Met Pro Gln Tyr Val Ile Ser Gln Ser Pro
            340                 345                 350

cat aga gta gtt tta aga aac ttt gaa gga gtt ata aca act tat aca     1104
His Arg Val Val Leu Arg Asn Phe Glu Gly Val Ile Thr Thr Tyr Thr
        355                 360                 365

gaa cca gaa aat tat aca cat gaa cct tgt tat gat gaa gaa aaa ttt     1152
Glu Pro Glu Asn Tyr Thr His Glu Pro Cys Tyr Asp Glu Glu Lys Phe
    370                 375                 380

gaa aaa atg tat gaa ata agt gga gtt tat atg cta gat gaa gga tta     1200
Glu Lys Met Tyr Glu Ile Ser Gly Val Tyr Met Leu Asp Glu Gly Leu
385                 390                 395                 400

aaa atg tca cta gaa cct agc cac tta gca aga cat gaa aga aat aaa     1248
Lys Met Ser Leu Glu Pro Ser His Leu Ala Arg His Glu Arg Asn Lys
                405                 410                 415

aag aga gca gaa gct gaa ggg aaa aaa taa                             1278
Lys Arg Ala Glu Ala Glu Gly Lys Lys
            420                 425


<210>  10
<211>  425
<212>  PRT
<213>  Fusobacterium nucleatum

<400>  10

Met Asn Thr Val Asn Thr Arg Lys Lys Phe Phe Pro Asn Val Thr Asp
1               5                   10                  15


Glu Glu Trp Asn Asp Trp Thr Trp Gln Val Lys Asn Arg Leu Glu Ser

20                  25                  30

Val Glu Asp Leu Lys Lys Tyr Val Asp Leu Ser Glu Glu Glu Thr Glu
        35                  40                  45

Gly Val Val Arg Thr Leu Glu Thr Leu Arg Met Ala Ile Thr Pro Tyr
    50                  55                  60

Tyr Phe Ser Leu Ile Asp Leu Asn Ser Asp Arg Cys Pro Ile Arg Lys
65                  70                  75                  80

Gln Ala Ile Pro Thr Ile Gln Glu Ile His Gln Ser Asp Ala Asp Leu
                85                  90                  95

Leu Asp Pro Leu His Glu Asp Glu Asp Ser Pro Val Pro Gly Leu Thr
            100                 105                 110

His Arg Tyr Pro Asp Arg Val Leu Leu Leu Ile Thr Asp Met Cys Ser
        115                 120                 125

Met Tyr Cys Arg His Cys Thr Arg Arg Arg Phe Ala Gly Ser Ser Asp
    130                 135                 140

Asp Ala Met Pro Met Asp Arg Ile Asp Lys Ala Ile Glu Tyr Ile Ala
145                 150                 155                 160

Lys Thr Pro Gln Val Arg Asp Val Leu Leu Ser Gly Gly Asp Ala Leu
                165                 170                 175

Leu Val Ser Asp Lys Lys Leu Glu Ser Ile Ile Gln Lys Leu Arg Ala
            180                 185                 190

Ile Pro His Val Glu Ile Ile Arg Ile Gly Ser Arg Thr Pro Val Val
        195                 200                 205

Leu Pro Gln Arg Ile Thr Pro Glu Leu Cys Asn Met Leu Lys Lys Tyr
    210                 215                 220

His Pro Ile Trp Leu Asn Thr His Phe Asn His Pro Gln Glu Val Thr
225                 230                 235                 240

Pro Glu Ala Lys Lys Ala Cys Glu Met Leu Ala Asp Ala Gly Val Pro
                245                 250                 255

Leu Gly Asn Gln Thr Val Leu Leu Arg Gly Ile Asn Asp Ser Val Pro
            260                 265                 270

Val Met Lys Arg Leu Val His Asp Leu Val Met Met Arg Val Arg Pro
        275                 280                 285

Tyr Tyr Ile Tyr Gln Cys Asp Leu Ser Met Gly Leu Glu His Phe Arg
    290                 295                 300

Thr Pro Val Ser Lys Gly Ile Glu Ile Ile Glu Gly Leu Arg Gly His
305                 310                 315                 320

Thr Ser Gly Tyr Ala Val Pro Thr Phe Val Val Asp Ala Pro Gly Gly
                325                 330                 335

Gly Gly Lys Thr Pro Val Met Pro Gln Tyr Val Ile Ser Gln Ser Pro
            340                 345                 350

His Arg Val Val Leu Arg Asn Phe Glu Gly Val Ile Thr Thr Tyr Thr
        355                 360                 365

Glu Pro Glu Asn Tyr Thr His Glu Pro Cys Tyr Asp Glu Glu Lys Phe
    370                 375                 380

Glu Lys Met Tyr Glu Ile Ser Gly Val Tyr Met Leu Asp Glu Gly Leu
385                 390                 395                 400

Lys Met Ser Leu Glu Pro Ser His Leu Ala Arg His Glu Arg Asn Lys
                405                 410                 415

Lys Arg Ala Glu Ala Glu Gly Lys Lys
            420                 425

<210>  11
<211>  1278
<212>  DNA
<213>  Fusobacterium nucleatum

<220>
<221>  CDS
<222>  (1)..(1278)

<400>  11
atg aat aca gtt aat act cgt aaa aaa ttt ttc cca aat gta act gat       48
Met Asn Thr Val Asn Thr Arg Lys Lys Phe Phe Pro Asn Val Thr Asp
1               5                   10                  15

gaa gaa tgg aat gat tgg aca tgg caa gta aaa aac cgc ctt gaa agt       96
Glu Glu Trp Asn Asp Trp Thr Trp Gln Val Lys Asn Arg Leu Glu Ser
            20                  25                  30

gtt gaa gat tta aaa aaa tat gtt gat tta agt gaa gaa gaa aca gaa      144
Val Glu Asp Leu Lys Lys Tyr Val Asp Leu Ser Glu Glu Glu Thr Glu
        35                  40                  45

ggg gtt gta cgc act ctt gaa act tta cgt atg gca atc act cca tat      192
Gly Val Val Arg Thr Leu Glu Thr Leu Arg Met Ala Ile Thr Pro Tyr
    50                  55                  60

tac ttc tca ttg ata gat ttg aat agt gat cgc tgc cca ata cgt aag      240
Tyr Phe Ser Leu Ile Asp Leu Asn Ser Asp Arg Cys Pro Ile Arg Lys
65                  70                  75                  80

caa gct ata cct act ata caa gaa ata cat caa tct gat gct gat ttg      288
Gln Ala Ile Pro Thr Ile Gln Glu Ile His Gln Ser Asp Ala Asp Leu
                85                  90                  95

tta gat cct cta cat gaa gat gaa gac tct cca gta cca gga tta act      336
Leu Asp Pro Leu His Glu Asp Glu Asp Ser Pro Val Pro Gly Leu Thr
            100                 105                 110

cat cgc tat cca gat cgt gtt tta ctt cta ata aca gac atg tgt tct      384
His Arg Tyr Pro Asp Arg Val Leu Leu Leu Ile Thr Asp Met Cys Ser
        115                 120                 125

atg tat tgt cgc cac tgc act cgt cgc aga ttt gct ggg tca agt gat      432
Met Tyr Cys Arg His Cys Thr Arg Arg Arg Phe Ala Gly Ser Ser Asp
    130                 135                 140

gat gct atg cct atg gat aga att gac aaa gca ata gaa tat att gca      480
Asp Ala Met Pro Met Asp Arg Ile Asp Lys Ala Ile Glu Tyr Ile Ala
145                 150                 155                 160

aaa act cca caa gta agg gat gta ttg tta tca gga gga gat gca ctt      528
Lys Thr Pro Gln Val Arg Asp Val Leu Leu Ser Gly Gly Asp Ala Leu
                165                 170                 175

cta gtt tct gat aaa aaa tta gaa agc ata atc caa aaa cta cgc gca      576
Leu Val Ser Asp Lys Lys Leu Glu Ser Ile Ile Gln Lys Leu Arg Ala
            180                 185                 190

ata cct cat gtt gaa ata atc aga ata gga agt cgt aca cca gtt gtt      624
Ile Pro His Val Glu Ile Ile Arg Ile Gly Ser Arg Thr Pro Val Val
        195                 200                 205

tta cct caa aga att act cct gaa tta tgt aat atg tta aag aaa tat      672
Leu Pro Gln Arg Ile Thr Pro Glu Leu Cys Asn Met Leu Lys Lys Tyr
    210                 215                 220

cat cca att tgg ttg aat act cat ttt aac cac cct caa gaa gta acg      720
His Pro Ile Trp Leu Asn Thr His Phe Asn His Pro Gln Glu Val Thr
225                 230                 235                 240

cca gaa gct aaa aaa gct tgt gaa atg ttg gca gat gca gga gtt cca      768
Pro Glu Ala Lys Lys Ala Cys Glu Met Leu Ala Asp Ala Gly Val Pro
                245                 250                 255

tta gga aat caa act gta cta tta aga gga ata aat gac agt gta cct      816
Leu Gly Asn Gln Thr Val Leu Leu Arg Gly Ile Asn Asp Ser Val Pro
            260                 265                 270

gta atg aaa agg tta gta cat gat tta gta atg atg cgt gta cgc cct      864

Val Met Lys Arg Leu Val His Asp Leu Val Met Met Arg Val Arg Pro
        275                 280                 285

tat tat att tac caa tgt gac tta tct atg gga ctc gaa cac ttc cgc      912
Tyr Tyr Ile Tyr Gln Cys Asp Leu Ser Met Gly Leu Glu His Phe Arg
    290                 295                 300

aca cca gtt tct aaa ggt ata gaa att att gaa gga tta cgt gga cat      960
Thr Pro Val Ser Lys Gly Ile Glu Ile Ile Glu Gly Leu Arg Gly His
305                 310                 315                 320

aca tct gga tat gca gta cca aca ttt gtt gtt gat gca cct ggt ggt     1008
Thr Ser Gly Tyr Ala Val Pro Thr Phe Val Val Asp Ala Pro Gly Gly
                325                 330                 335

gga gga aaa act cca gta atg cct caa tat gta att tct caa tct cct     1056
Gly Gly Lys Thr Pro Val Met Pro Gln Tyr Val Ile Ser Gln Ser Pro
            340                 345                 350

cat cgt gta gtt tta cgc aac ttt gaa gga gtt ata aca act tat aca     1104
His Arg Val Val Leu Arg Asn Phe Glu Gly Val Ile Thr Thr Tyr Thr
        355                 360                 365

gaa cca gaa aat tat aca cat gaa cct tgt tat gat gaa gaa aaa ttt     1152
Glu Pro Glu Asn Tyr Thr His Glu Pro Cys Tyr Asp Glu Glu Lys Phe
    370                 375                 380

gaa aaa atg tat gaa ata agt gga gtt tat atg cta gat gaa gga tta     1200
Glu Lys Met Tyr Glu Ile Ser Gly Val Tyr Met Leu Asp Glu Gly Leu
385                 390                 395                 400

aaa atg tca cta gaa cct agc cac tta gca cgt cat gaa cgc aat aaa     1248
Lys Met Ser Leu Glu Pro Ser His Leu Ala Arg His Glu Arg Asn Lys
                405                 410                 415

aag aga gca gaa gct gaa ggg aaa aaa taa                             1278
Lys Arg Ala Glu Ala Glu Gly Lys Lys
            420                 425


<210>  12
<211>  425
<212>  PRT
<213>  Fusobacterium nucleatum

<400>  12

Met Asn Thr Val Asn Thr Arg Lys Lys Phe Phe Pro Asn Val Thr Asp
1               5                   10                  15


Glu Glu Trp Asn Asp Trp Thr Trp Gln Val Lys Asn Arg Leu Glu Ser
            20                  25                  30


Val Glu Asp Leu Lys Lys Tyr Val Asp Leu Ser Glu Glu Glu Thr Glu
        35                  40                  45


Gly Val Val Arg Thr Leu Glu Thr Leu Arg Met Ala Ile Thr Pro Tyr
    50                  55                  60

Tyr Phe Ser Leu Ile Asp Leu Asn Ser Asp Arg Cys Pro Ile Arg Lys
65                  70                  75                  80

Gln Ala Ile Pro Thr Ile Gln Glu Ile His Gln Ser Asp Ala Asp Leu
                85                  90                  95

Leu Asp Pro Leu His Glu Asp Glu Asp Ser Pro Val Pro Gly Leu Thr
            100                 105                 110

His Arg Tyr Pro Asp Arg Val Leu Leu Leu Ile Thr Asp Met Cys Ser
        115                 120                 125

Met Tyr Cys Arg His Cys Thr Arg Arg Arg Phe Ala Gly Ser Ser Asp
    130                 135                 140

Asp Ala Met Pro Met Asp Arg Ile Asp Lys Ala Ile Glu Tyr Ile Ala
145                 150                 155                 160

Lys Thr Pro Gln Val Arg Asp Val Leu Leu Ser Gly Gly Asp Ala Leu
                165                 170                 175

Leu Val Ser Asp Lys Lys Leu Glu Ser Ile Ile Gln Lys Leu Arg Ala
            180                 185                 190

Ile Pro His Val Glu Ile Ile Arg Ile Gly Ser Arg Thr Pro Val Val
        195                 200                 205

Leu Pro Gln Arg Ile Thr Pro Glu Leu Cys Asn Met Leu Lys Lys Tyr
    210                 215                 220

His Pro Ile Trp Leu Asn Thr His Phe Asn His Pro Gln Glu Val Thr
225                 230                 235                 240

Pro Glu Ala Lys Lys Ala Cys Glu Met Leu Ala Asp Ala Gly Val Pro
                245                 250                 255

Leu Gly Asn Gln Thr Val Leu Leu Arg Gly Ile Asn Asp Ser Val Pro
            260                 265                 270

Val Met Lys Arg Leu Val His Asp Leu Val Met Met Arg Val Arg Pro
        275                 280                 285

Tyr Tyr Ile Tyr Gln Cys Asp Leu Ser Met Gly Leu Glu His Phe Arg
    290                 295                 300

Thr Pro Val Ser Lys Gly Ile Glu Ile Ile Glu Gly Leu Arg Gly His
305                 310                 315                 320

Thr Ser Gly Tyr Ala Val Pro Thr Phe Val Val Asp Ala Pro Gly Gly
                325                 330                 335

Gly Gly Lys Thr Pro Val Met Pro Gln Tyr Val Ile Ser Gln Ser Pro
            340                 345                 350

His Arg Val Val Leu Arg Asn Phe Glu Gly Val Ile Thr Thr Tyr Thr
        355                 360                 365

Glu Pro Glu Asn Tyr Thr His Glu Pro Cys Tyr Asp Glu Glu Lys Phe
    370                 375                 380

Glu Lys Met Tyr Glu Ile Ser Gly Val Tyr Met Leu Asp Glu Gly Leu
385                 390                 395                 400

Lys Met Ser Leu Glu Pro Ser His Leu Ala Arg His Glu Arg Asn Lys
                405                 410                 415

Lys Arg Ala Glu Ala Glu Gly Lys Lys
            420                 425

<210>  13
<211>  1278
<212>  DNA
<213>  Fusobacterium nucleatum

<220>
<221>  CDS
<222>  (1)..(1278)

<400>  13
atg aat aca gtt aat act cgt aaa aaa ttt ttc cca aat gta act gat       48
Met Asn Thr Val Asn Thr Arg Lys Lys Phe Phe Pro Asn Val Thr Asp
1               5                   10                  15

gaa gaa tgg aat gat tgg aca tgg caa gta aaa aac cgc ctt gaa agt       96
Glu Glu Trp Asn Asp Trp Thr Trp Gln Val Lys Asn Arg Leu Glu Ser
            20                  25                  30

gtt gaa gat tta aaa aaa tat gtt gat tta agt gaa gaa gaa aca gaa      144
Val Glu Asp Leu Lys Lys Tyr Val Asp Leu Ser Glu Glu Glu Thr Glu
        35                  40                  45

ggg gtt gta cgc act ctt gaa act tta cgt atg gca atc act cca tat      192
Gly Val Val Arg Thr Leu Glu Thr Leu Arg Met Ala Ile Thr Pro Tyr
    50                  55                  60

tac ttc tca ttg ata gat ttg aat agt gat cgc tgc cca ata cgt aag      240

Tyr Phe Ser Leu Ile Asp Leu Asn Ser Asp Arg Cys Pro Ile Arg Lys
65                  70                  75                  80

caa gct ata cct act ata caa gaa ata cat caa tct gat gct gat atg      288
Gln Ala Ile Pro Thr Ile Gln Glu Ile His Gln Ser Asp Ala Asp Met
                85                  90                  95

ttg gat cct cta cat gaa gat gaa gac tct cca gta cca gga tta act      336
Leu Asp Pro Leu His Glu Asp Glu Asp Ser Pro Val Pro Gly Leu Thr
            100                 105                 110

cat cgc tat cca gat cgt gtt tta ctt cta ata aca gac atg tgt tct      384
His Arg Tyr Pro Asp Arg Val Leu Leu Leu Ile Thr Asp Met Cys Ser
        115                 120                 125

gta tac tgt cgc cac tgc act cgt cgc aga ttt gct ggg tca agt gat      432
Val Tyr Cys Arg His Cys Thr Arg Arg Arg Phe Ala Gly Ser Ser Asp
    130                 135                 140

gat gct atg cct atg gat aga att gac aaa gca ata gaa tat att gca      480
Asp Ala Met Pro Met Asp Arg Ile Asp Lys Ala Ile Glu Tyr Ile Ala
145                 150                 155                 160

aaa act cca caa gta agg gat gta ttg tta tca gga gga gat gca ctt      528
Lys Thr Pro Gln Val Arg Asp Val Leu Leu Ser Gly Gly Asp Ala Leu
                165                 170                 175

cta gtt tct gat aaa aaa tta gaa agc ata atc caa aaa cta cgc gca      576
Leu Val Ser Asp Lys Lys Leu Glu Ser Ile Ile Gln Lys Leu Arg Ala
            180                 185                 190

ata cct cat gtt gaa ata atc aga ata gga agt cgt aca cca gtt gtt      624
Ile Pro His Val Glu Ile Ile Arg Ile Gly Ser Arg Thr Pro Val Val
        195                 200                 205

tta cct caa aga att act cct gaa tta tgt aat atg tta aag aaa tat      672
Leu Pro Gln Arg Ile Thr Pro Glu Leu Cys Asn Met Leu Lys Lys Tyr
    210                 215                 220

cat cca att tgg ttg aat act cat ttt aac cac cct caa gaa gta acg      720
His Pro Ile Trp Leu Asn Thr His Phe Asn His Pro Gln Glu Val Thr
225                 230                 235                 240

cca gaa gct aaa aaa gct tgt gaa atg ttg gca gat gca gga gtt cca      768
Pro Glu Ala Lys Lys Ala Cys Glu Met Leu Ala Asp Ala Gly Val Pro
                245                 250                 255

tta gga aat caa act gta cta tta aga gga ata aat gac agt gta cct      816
Leu Gly Asn Gln Thr Val Leu Leu Arg Gly Ile Asn Asp Ser Val Pro
            260                 265                 270

gta atg aaa agg tta gta cat gat tta gta atg atg cgt gta cgc cct      864
Val Met Lys Arg Leu Val His Asp Leu Val Met Met Arg Val Arg Pro
        275                 280                 285

tat tat att tac caa tgt gac tta tct atg gga ctc gaa cac ttc cgc      912
Tyr Tyr Ile Tyr Gln Cys Asp Leu Ser Met Gly Leu Glu His Phe Arg
    290                 295                 300

aca cca gtt tct aaa ggt ata gaa att att gaa gga tta cgt gga cat      960
Thr Pro Val Ser Lys Gly Ile Glu Ile Ile Glu Gly Leu Arg Gly His

305                 310                 315                 320

aca tct gga tat gca gta cca aca ttt gtt gtg cat gca cct ggt ggt      1008
Thr Ser Gly Tyr Ala Val Pro Thr Phe Val Val His Ala Pro Gly Gly
                325                 330                 335

gga gga aaa act cca gta atg cct caa tat gta att tct caa tct cct      1056
Gly Gly Lys Thr Pro Val Met Pro Gln Tyr Val Ile Ser Gln Ser Pro
            340                 345                 350

cat cgt gta gtt tta cgc aac ttt gaa gga gtt ata aca act tat aca      1104
His Arg Val Val Leu Arg Asn Phe Glu Gly Val Ile Thr Thr Tyr Thr
        355                 360                 365

gaa cca gaa aat tat aca cat gaa cct tgt tat gat gaa gaa aaa ttt      1152
Glu Pro Glu Asn Tyr Thr His Glu Pro Cys Tyr Asp Glu Glu Lys Phe
    370                 375                 380

gaa aaa atg tat gaa ata agt gga gtt tat atg cta gat gaa gga tta      1200
Glu Lys Met Tyr Glu Ile Ser Gly Val Tyr Met Leu Asp Glu Gly Leu
385                 390                 395                 400

aaa atg tca cta gaa cct agc cac tta gca cgt cat gaa cgc aat aaa      1248
Lys Met Ser Leu Glu Pro Ser His Leu Ala Arg His Glu Arg Asn Lys
                405                 410                 415

aag aga gca gaa gct gaa ggg aaa aaa taa                              1278
Lys Arg Ala Glu Ala Glu Gly Lys Lys
            420                 425


<210>  14
<211>  425
<212>  PRT
<213>  Fusobacterium nucleatum

<400>  14

Met Asn Thr Val Asn Thr Arg Lys Lys Phe Phe Pro Asn Val Thr Asp
1               5                   10                  15


Glu Glu Trp Asn Asp Trp Thr Trp Gln Val Lys Asn Arg Leu Glu Ser
            20                  25                  30


Val Glu Asp Leu Lys Lys Tyr Val Asp Leu Ser Glu Glu Glu Thr Glu
        35                  40                  45


Gly Val Val Arg Thr Leu Glu Thr Leu Arg Met Ala Ile Thr Pro Tyr
    50                  55                  60


Tyr Phe Ser Leu Ile Asp Leu Asn Ser Asp Arg Cys Pro Ile Arg Lys
65                  70                  75                  80


Gln Ala Ile Pro Thr Ile Gln Glu Ile His Gln Ser Asp Ala Asp Met
                85                  90                  95

Leu Asp Pro Leu His Glu Asp Glu Asp Ser Pro Val Pro Gly Leu Thr
            100                 105                 110

His Arg Tyr Pro Asp Arg Val Leu Leu Leu Ile Thr Asp Met Cys Ser
        115                 120                 125

Val Tyr Cys Arg His Cys Thr Arg Arg Arg Phe Ala Gly Ser Ser Asp
    130                 135                 140

Asp Ala Met Pro Met Asp Arg Ile Asp Lys Ala Ile Glu Tyr Ile Ala
145                 150                 155                 160

Lys Thr Pro Gln Val Arg Asp Val Leu Leu Ser Gly Gly Asp Ala Leu
                165                 170                 175

Leu Val Ser Asp Lys Lys Leu Glu Ser Ile Ile Gln Lys Leu Arg Ala
            180                 185                 190

Ile Pro His Val Glu Ile Ile Arg Ile Gly Ser Arg Thr Pro Val Val
        195                 200                 205

Leu Pro Gln Arg Ile Thr Pro Glu Leu Cys Asn Met Leu Lys Lys Tyr
    210                 215                 220

His Pro Ile Trp Leu Asn Thr His Phe Asn His Pro Gln Glu Val Thr
225                 230                 235                 240

Pro Glu Ala Lys Lys Ala Cys Glu Met Leu Ala Asp Ala Gly Val Pro
                245                 250                 255

Leu Gly Asn Gln Thr Val Leu Leu Arg Gly Ile Asn Asp Ser Val Pro
            260                 265                 270

Val Met Lys Arg Leu Val His Asp Leu Val Met Met Arg Val Arg Pro
        275                 280                 285

Tyr Tyr Ile Tyr Gln Cys Asp Leu Ser Met Gly Leu Glu His Phe Arg
    290                 295                 300

Thr Pro Val Ser Lys Gly Ile Glu Ile Ile Glu Gly Leu Arg Gly His
305                 310                 315                 320

Thr Ser Gly Tyr Ala Val Pro Thr Phe Val Val His Ala Pro Gly Gly
                325                 330                 335

Gly Gly Lys Thr Pro Val Met Pro Gln Tyr Val Ile Ser Gln Ser Pro
            340                 345                 350

His Arg Val Val Leu Arg Asn Phe Glu Gly Val Ile Thr Thr Tyr Thr
        355                 360                 365

Glu Pro Glu Asn Tyr Thr His Glu Pro Cys Tyr Asp Glu Glu Lys Phe
    370                 375                 380

Glu Lys Met Tyr Glu Ile Ser Gly Val Tyr Met Leu Asp Glu Gly Leu
385                 390                 395                 400

Lys Met Ser Leu Glu Pro Ser His Leu Ala Arg His Glu Arg Asn Lys
                405                 410                 415

Lys Arg Ala Glu Ala Glu Gly Lys Lys
            420                 425

<210>  15
<211>  40
<212>  DNA
<213>  Artificial

<220>
<223>  mutagenesis primer

<400>  15
catcaatctg atgctgatat gttggatcct ctacatgaag                              40

<210>  16
<211>  38
<212>  DNA
<213>  Artificial

<220>
<223>  mutagenesis primer

<400>  16
aacagacatg tgttctgtat actgtcgcca ctgcactc                                38

<210>  17
<211>  34
<212>  DNA
<213>  Artificial

<220>
<223>  mutagenesis primer

<400>  17
gtaccaacat ttgttgtgca tgcacctggt ggtg                                    34

<210>  18

<211>  1278
<212>  DNA
<213>  Fusobacterium nucleatum

<220>
<221>  CDS
<222>  (1)..(1278)

<400>  18
atg aat aca gtt aat act cgt aaa aaa ttt ttc cca aat gta act gat        48
Met Asn Thr Val Asn Thr Arg Lys Lys Phe Phe Pro Asn Val Thr Asp
1               5                   10                  15

gaa gaa tgg aat gat tgg aca tgg caa gta aaa aac cgc ctt aaa agt        96
Glu Glu Trp Asn Asp Trp Thr Trp Gln Val Lys Asn Arg Leu Lys Ser
            20                  25                  30

gtt gaa gat tta aaa aaa tat gtt gat tta agt gaa gaa gaa aca gaa       144
Val Glu Asp Leu Lys Lys Tyr Val Asp Leu Ser Glu Glu Glu Thr Glu
        35                  40                  45

ggg gtt gta cgc act ctt gaa act tta cgt atg gca atc act cca ttt       192
Gly Val Val Arg Thr Leu Glu Thr Leu Arg Met Ala Ile Thr Pro Phe
    50                  55                  60

tac ttc tca ttg ata gat ttg aat agt gat cgc tgc cca ata cgt aag       240
Tyr Phe Ser Leu Ile Asp Leu Asn Ser Asp Arg Cys Pro Ile Arg Lys
65                  70                  75                  80

caa gct ata cct act ata cga gaa ata cat caa tct gat gct gat atg       288
Gln Ala Ile Pro Thr Ile Arg Glu Ile His Gln Ser Asp Ala Asp Met
                85                  90                  95

ttg gat cct cta cat gaa gat gaa gac tct cca gta cca gga tta act       336
Leu Asp Pro Leu His Glu Asp Glu Asp Ser Pro Val Pro Gly Leu Thr
            100                 105                 110

cat cgc tat cca gat cgt gtt tta ctt cta ata aca gac atg tgt tct       384
His Arg Tyr Pro Asp Arg Val Leu Leu Leu Ile Thr Asp Met Cys Ser
        115                 120                 125

gta tac tgt cgc cac tgc act cgt cgc aga ttt gct ggg tca agt gat       432
Val Tyr Cys Arg His Cys Thr Arg Arg Arg Phe Ala Gly Ser Ser Asp
    130                 135                 140

ggt gct atg cct atg gat aga att gac aaa gca ata gaa tat att gca       480
Gly Ala Met Pro Met Asp Arg Ile Asp Lys Ala Ile Glu Tyr Ile Ala
145                 150                 155                 160

aaa act cca caa gta agg gat gta ttg tta tca gga gga gat gca ctt       528
Lys Thr Pro Gln Val Arg Asp Val Leu Leu Ser Gly Gly Asp Ala Leu
                165                 170                 175

cta gtt tct gat aaa aaa tta gaa agc ata atc caa aaa cta cgc gca       576
Leu Val Ser Asp Lys Lys Leu Glu Ser Ile Ile Gln Lys Leu Arg Ala
            180                 185                 190

ata cct cat gtt gaa ata atc aga ata gga agt cgt aca cca gtt gtt       624
Ile Pro His Val Glu Ile Ile Arg Ile Gly Ser Arg Thr Pro Val Val
        195                 200                 205

tta cct caa aga att act cct gaa tta tgt aat atg tta aag aaa tat 672
Leu Pro Gln Arg Ile Thr Pro Glu Leu Cys Asn Met Leu Lys Lys Tyr
    210                 215                 220

cat cca att tgg atg aat act cat ttt aac cac cct caa gaa gta acg 720
His Pro Ile Trp Met Asn Thr His Phe Asn His Pro Gln Glu Val Thr
225                 230                 235                 240

cca gaa gct aaa aaa gct tgt gaa atg ttg gca gat gca gga gtt cca 768
Pro Glu Ala Lys Lys Ala Cys Glu Met Leu Ala Asp Ala Gly Val Pro
                245                 250                 255

tta gga aat caa act gta cta tta aga gga ata aat gac agt gta cct 816
Leu Gly Asn Gln Thr Val Leu Leu Arg Gly Ile Asn Asp Ser Val Pro
            260                 265                 270

gta atg aaa agg tta gta cat gat tta gta atg atg cgt gta cgc cct 864
Val Met Lys Arg Leu Val His Asp Leu Val Met Met Arg Val Arg Pro
        275                 280                 285

tat tat att tac caa tgt gac tta tct atg gga ctc gaa cac ttc cgc 912
Tyr Tyr Ile Tyr Gln Cys Asp Leu Ser Met Gly Leu Glu His Phe Arg
    290                 295                 300

aca cca gtt tct aaa ggt ata gaa att att gaa gga tta cgt gga cat 960
Thr Pro Val Ser Lys Gly Ile Glu Ile Ile Glu Gly Leu Arg Gly His
305                 310                 315                 320

aca tct gga tat gca gta cca aca ttt gtt gtg cat gca cct ggt ggt 1008
Thr Ser Gly Tyr Ala Val Pro Thr Phe Val Val His Ala Pro Gly Gly
                325                 330                 335

gga gga aaa act cca gta atg cct caa tat gta att tct caa tct cct 1056
Gly Gly Lys Thr Pro Val Met Pro Gln Tyr Val Ile Ser Gln Ser Pro
            340                 345                 350

cat cgt gta gtt tta cgc aac ttt gaa gga gtt ata aca act tat aca 1104
His Arg Val Val Leu Arg Asn Phe Glu Gly Val Ile Thr Thr Tyr Thr
        355                 360                 365

gaa cca gaa aat tat aca cat gaa cct tgt tat gat gaa gaa aaa ttt 1152
Glu Pro Glu Asn Tyr Thr His Glu Pro Cys Tyr Asp Glu Glu Lys Phe
    370                 375                 380

gaa aaa atg tat gaa ata agt gga gtt tat atg cta gat gaa gga tta 1200
Glu Lys Met Tyr Glu Ile Ser Gly Val Tyr Met Leu Asp Glu Gly Leu
385                 390                 395                 400

gaa atg tca cta gaa cct agc cac tta gca cgt cat gaa cgc aat aaa 1248
Glu Met Ser Leu Glu Pro Ser His Leu Ala Arg His Glu Arg Asn Lys
                405                 410                 415

aag aga gca gaa gct gaa ggg aaa aaa taa 1278
Lys Arg Ala Glu Ala Glu Gly Lys Lys
            420                 425

<210> 19
<211> 425
<212> PRT

<213>  Fusobacterium nucleatum

<400>  19

Met Asn Thr Val Asn Thr Arg Lys Lys Phe Phe Pro Asn Val Thr Asp
1               5                   10                  15

Glu Glu Trp Asn Asp Trp Thr Trp Gln Val Lys Asn Arg Leu Lys Ser
            20                  25                  30

Val Glu Asp Leu Lys Lys Tyr Val Asp Leu Ser Glu Glu Glu Thr Glu
        35                  40                  45

Gly Val Val Arg Thr Leu Glu Thr Leu Arg Met Ala Ile Thr Pro Phe
    50                  55                  60

Tyr Phe Ser Leu Ile Asp Leu Asn Ser Asp Arg Cys Pro Ile Arg Lys
65                  70                  75                  80

Gln Ala Ile Pro Thr Ile Arg Glu Ile His Gln Ser Asp Ala Asp Met
                85                  90                  95

Leu Asp Pro Leu His Glu Asp Glu Asp Ser Pro Val Pro Gly Leu Thr
            100                 105                 110

His Arg Tyr Pro Asp Arg Val Leu Leu Leu Ile Thr Asp Met Cys Ser
        115                 120                 125

Val Tyr Cys Arg His Cys Thr Arg Arg Arg Phe Ala Gly Ser Ser Asp
    130                 135                 140

Gly Ala Met Pro Met Asp Arg Ile Asp Lys Ala Ile Glu Tyr Ile Ala
145                 150                 155                 160

Lys Thr Pro Gln Val Arg Asp Val Leu Leu Ser Gly Gly Asp Ala Leu
                165                 170                 175

Leu Val Ser Asp Lys Lys Leu Glu Ser Ile Ile Gln Lys Leu Arg Ala
            180                 185                 190

Ile Pro His Val Glu Ile Ile Arg Ile Gly Ser Arg Thr Pro Val Val
        195                 200                 205

Leu Pro Gln Arg Ile Thr Pro Glu Leu Cys Asn Met Leu Lys Lys Tyr
    210                 215                 220

His Pro Ile Trp Met Asn Thr His Phe Asn His Pro Gln Glu Val Thr

225                 230                 235                 240

Pro Glu Ala Lys Lys Ala Cys Glu Met Leu Ala Asp Ala Gly Val Pro
                245                 250                 255

Leu Gly Asn Gln Thr Val Leu Leu Arg Gly Ile Asn Asp Ser Val Pro
            260                 265                 270

Val Met Lys Arg Leu Val His Asp Leu Val Met Met Arg Val Arg Pro
        275                 280                 285

Tyr Tyr Ile Tyr Gln Cys Asp Leu Ser Met Gly Leu Glu His Phe Arg
    290                 295                 300

Thr Pro Val Ser Lys Gly Ile Glu Ile Ile Glu Gly Leu Arg Gly His
305                 310                 315                 320

Thr Ser Gly Tyr Ala Val Pro Thr Phe Val Val His Ala Pro Gly Gly
                325                 330                 335

Gly Gly Lys Thr Pro Val Met Pro Gln Tyr Val Ile Ser Gln Ser Pro
            340                 345                 350

His Arg Val Val Leu Arg Asn Phe Glu Gly Val Ile Thr Thr Tyr Thr
        355                 360                 365

Glu Pro Glu Asn Tyr Thr His Glu Pro Cys Tyr Asp Glu Glu Lys Phe
    370                 375                 380

Glu Lys Met Tyr Glu Ile Ser Gly Val Tyr Met Leu Asp Glu Gly Leu
385                 390                 395                 400

Glu Met Ser Leu Glu Pro Ser His Leu Ala Arg His Glu Arg Asn Lys
                405                 410                 415

Lys Arg Ala Glu Ala Glu Gly Lys Lys
            420                 425

<210>  20
<211>  1278
<212>  DNA
<213>  Fusobacterium nucleatum

<220>
<221>  CDS
<222>  (1)..(1278)

<400>  20
atg aat aca gtt aat act cgt aaa aaa ttt ttc cca aat gta act gat       48
Met Asn Thr Val Asn Thr Arg Lys Lys Phe Phe Pro Asn Val Thr Asp
1               5                   10                  15

gaa gaa tgg aat gat tgg aca tgg caa gta aaa aac cgc ctt aaa agt       96
Glu Glu Trp Asn Asp Trp Thr Trp Gln Val Lys Asn Arg Leu Lys Ser
            20                  25                  30

gtt gaa gat tta gaa aaa tat gtt gat tta agt gaa gaa gaa aca gaa      144
Val Glu Asp Leu Glu Lys Tyr Val Asp Leu Ser Glu Glu Glu Thr Glu
        35                  40                  45

ggg gtt gta cgc act ctt gaa act tta cgt atg gca atc act cca ttt      192
Gly Val Val Arg Thr Leu Glu Thr Leu Arg Met Ala Ile Thr Pro Phe
    50                  55                  60

tac ttc tca ttg ata gat ttg aat agt gat cgc tgc cca ata cgt aag      240
Tyr Phe Ser Leu Ile Asp Leu Asn Ser Asp Arg Cys Pro Ile Arg Lys
65                  70                  75                  80

caa gct ata cct act ata cga gaa ata cat caa tct gat gct gat atg      288
Gln Ala Ile Pro Thr Ile Arg Glu Ile His Gln Ser Asp Ala Asp Met
                85                  90                  95

ttg gat cct cta cat gaa gat gaa gac tct cca gta cca gga tta act      336
Leu Asp Pro Leu His Glu Asp Glu Asp Ser Pro Val Pro Gly Leu Thr
            100                 105                 110

cat cgc tat cca gat cgt gtt tta ctt cta ata aca gac atg tgt tct      384
His Arg Tyr Pro Asp Arg Val Leu Leu Leu Ile Thr Asp Met Cys Ser
        115                 120                 125

gta tac tgt cgc cac tgc act cgt cgc aga ttt gct ggg tca agt gat      432
Val Tyr Cys Arg His Cys Thr Arg Arg Arg Phe Ala Gly Ser Ser Asp
    130                 135                 140

ggt gct atg cct atg gat aga att gac aaa gca ata gaa tat att gca      480
Gly Ala Met Pro Met Asp Arg Ile Asp Lys Ala Ile Glu Tyr Ile Ala
145                 150                 155                 160

aaa act cca caa gta agg gat gta ttg tta tca gga gga gat gca ctt      528
Lys Thr Pro Gln Val Arg Asp Val Leu Leu Ser Gly Gly Asp Ala Leu
                165                 170                 175

cta gtt tct aat aaa aaa tta gaa agc ata atc caa aaa cta cgc gca      576
Leu Val Ser Asn Lys Lys Leu Glu Ser Ile Ile Gln Lys Leu Arg Ala
            180                 185                 190

ata cct cat gtt gaa ata atc aga ata gga agt cgt aca cca gtt gtt      624
Ile Pro His Val Glu Ile Ile Arg Ile Gly Ser Arg Thr Pro Val Val
        195                 200                 205

tta cct caa aga att act cct gaa tta tgt aat atg tta aag aaa tat      672
Leu Pro Gln Arg Ile Thr Pro Glu Leu Cys Asn Met Leu Lys Lys Tyr
    210                 215                 220

cat cca att tgg atg aat act cat ttt aac cac cct caa gaa gta acg      720
His Pro Ile Trp Met Asn Thr His Phe Asn His Pro Gln Glu Val Thr
225                 230                 235                 240

cca gaa gct aaa aaa gct tgt gaa atg ttg gca gat gca gga gtt cca      768
Pro Glu Ala Lys Lys Ala Cys Glu Met Leu Ala Asp Ala Gly Val Pro
                245                 250                 255

tta gga aat caa act gta cta tta aga gga ata aat gac agt gta cct      816
Leu Gly Asn Gln Thr Val Leu Leu Arg Gly Ile Asn Asp Ser Val Pro
            260                 265                 270

gta atg aaa agg tta gta cat gat tta gta atg atg cgt gta cgc cct      864
Val Met Lys Arg Leu Val His Asp Leu Val Met Met Arg Val Arg Pro
        275                 280                 285

tat tat att tac caa tgt gac tta tct atg gga ctc gaa cac ttc cgc      912
Tyr Tyr Ile Tyr Gln Cys Asp Leu Ser Met Gly Leu Glu His Phe Arg
    290                 295                 300

aca cca gtt tct aaa ggt ata gaa att att gaa gga tta cgt gga cat      960
Thr Pro Val Ser Lys Gly Ile Glu Ile Ile Glu Gly Leu Arg Gly His
305                 310                 315                 320

aca tct gga tat gca gta cca aca ttt gtt gtg cat gca cct ggt ggt     1008
Thr Ser Gly Tyr Ala Val Pro Thr Phe Val Val His Ala Pro Gly Gly
                325                 330                 335

gga gga aaa act cca gta atg cct caa tat gta att tct caa tct cct     1056
Gly Gly Lys Thr Pro Val Met Pro Gln Tyr Val Ile Ser Gln Ser Pro
            340                 345                 350

cat cgt gta gtt tta cgc aac ttt gaa gga gtt ata aca act tat aca     1104
His Arg Val Val Leu Arg Asn Phe Glu Gly Val Ile Thr Thr Tyr Thr
        355                 360                 365

gaa cca gaa aat tat aca cat gaa cct tgt tat gat gaa gaa aaa ttt     1152
Glu Pro Glu Asn Tyr Thr His Glu Pro Cys Tyr Asp Glu Glu Lys Phe
    370                 375                 380

gaa aaa atg tat gaa ata agt gga gtt tat atg cta gat gaa gga tta     1200
Glu Lys Met Tyr Glu Ile Ser Gly Val Tyr Met Leu Asp Glu Gly Leu
385                 390                 395                 400

gaa atg tca cta gaa cct agc cac tta gca cgt cat gaa cgc aat aaa     1248
Glu Met Ser Leu Glu Pro Ser His Leu Ala Arg His Glu Arg Asn Lys
                405                 410                 415

aag aga gca gaa gct gaa ggg aaa aaa taa                             1278
Lys Arg Ala Glu Ala Glu Gly Lys Lys
            420                 425


<210>  21
<211>  425
<212>  PRT
<213>  Fusobacterium nucleatum

<400>  21

Met Asn Thr Val Asn Thr Arg Lys Lys Phe Phe Pro Asn Val Thr Asp
1               5                   10                  15


Glu Glu Trp Asn Asp Trp Thr Trp Gln Val Lys Asn Arg Leu Lys Ser

20                  25                  30

Val Glu Asp Leu Glu Lys Tyr Val Asp Leu Ser Glu Glu Glu Thr Glu
        35                  40                  45

Gly Val Val Arg Thr Leu Glu Thr Leu Arg Met Ala Ile Thr Pro Phe
    50                  55                  60

Tyr Phe Ser Leu Ile Asp Leu Asn Ser Asp Arg Cys Pro Ile Arg Lys
65                  70                  75                  80

Gln Ala Ile Pro Thr Ile Arg Glu Ile His Gln Ser Asp Ala Asp Met
                85                  90                  95

Leu Asp Pro Leu His Glu Asp Glu Asp Ser Pro Val Pro Gly Leu Thr
            100                 105                 110

His Arg Tyr Pro Asp Arg Val Leu Leu Leu Ile Thr Asp Met Cys Ser
        115                 120                 125

Val Tyr Cys Arg His Cys Thr Arg Arg Arg Phe Ala Gly Ser Ser Asp
    130                 135                 140

Gly Ala Met Pro Met Asp Arg Ile Asp Lys Ala Ile Glu Tyr Ile Ala
145                 150                 155                 160

Lys Thr Pro Gln Val Arg Asp Val Leu Leu Ser Gly Gly Asp Ala Leu
                165                 170                 175

Leu Val Ser Asn Lys Lys Leu Glu Ser Ile Ile Gln Lys Leu Arg Ala
            180                 185                 190

Ile Pro His Val Glu Ile Ile Arg Ile Gly Ser Arg Thr Pro Val Val
        195                 200                 205

Leu Pro Gln Arg Ile Thr Pro Glu Leu Cys Asn Met Leu Lys Lys Tyr
    210                 215                 220

His Pro Ile Trp Met Asn Thr His Phe Asn His Pro Gln Glu Val Thr
225                 230                 235                 240

Pro Glu Ala Lys Lys Ala Cys Glu Met Leu Ala Asp Ala Gly Val Pro
                245                 250                 255

Leu Gly Asn Gln Thr Val Leu Leu Arg Gly Ile Asn Asp Ser Val Pro
            260                 265                 270

Val Met Lys Arg Leu Val His Asp Leu Val Met Met Arg Val Arg Pro
        275                 280                 285

Tyr Tyr Ile Tyr Gln Cys Asp Leu Ser Met Gly Leu Glu His Phe Arg
    290                 295                 300

Thr Pro Val Ser Lys Gly Ile Glu Ile Ile Glu Gly Leu Arg Gly His
305                 310                 315                 320

Thr Ser Gly Tyr Ala Val Pro Thr Phe Val Val His Ala Pro Gly Gly
                325                 330                 335

Gly Gly Lys Thr Pro Val Met Pro Gln Tyr Val Ile Ser Gln Ser Pro
            340                 345                 350

His Arg Val Val Leu Arg Asn Phe Glu Gly Val Ile Thr Thr Tyr Thr
        355                 360                 365

Glu Pro Glu Asn Tyr Thr His Glu Pro Cys Tyr Asp Glu Glu Lys Phe
    370                 375                 380

Glu Lys Met Tyr Glu Ile Ser Gly Val Tyr Met Leu Asp Glu Gly Leu
385                 390                 395                 400

Glu Met Ser Leu Glu Pro Ser His Leu Ala Arg His Glu Arg Asn Lys
                405                 410                 415

Lys Arg Ala Glu Ala Glu Gly Lys Lys
            420                 425

<210>  22
<211>  20
<212>  DNA
<213>  Artificial

<220>
<223>  primer

<220>
<221>  misc_feature
<222>  (1)..(20)
<223>  Y is t/u or c; w is a or t/u.

<400>  22
ytwagaatgg cwatwacwcc                                                   20

<210>  23

<211>  20
<212>  DNA
<213>  Artificial

<220>
<223>  primer


<220>
<221>  misc_feature
<222>  (1)..(20)
<223>  R is g or a, W is a or t/u.

<400>  23
agaaarcarg cwatwccwac                                                   20


<210>  24
<211>  20
<212>  DNA
<213>  Artificial

<220>
<223>  primer


<220>
<221>  misc_feature
<222>  (1)..(20)
<223>  Y is t/u or c; w is a or t/u.

<400>  24
ggwytwacwc ayagataycc                                                   20


<210>  25
<211>  20
<212>  DNA
<213>  Artificial

<220>
<223>  primer


<220>
<221>  misc_feature
<222>  (1)..(20)
<223>  Y is t/u or c; w is a or t/u.

<400>  25
tawgtwgtwa twacwccytc                                                   20


<210>  26
<211>  20
<212>  DNA
<213>  Artificial

<220>
<223>  primer

<220>
<221>  misc_feature
<222>  (1)..(20)
<223>  r is g or a; w is a or t/u.

<400>  26
tcwacwacra awgtwggwac                                                   20


<210>  27
<211>  20
<212>  DNA
<213>  Artificial

<220>
<223>  primer


<220>
<221>  misc_feature
<222>  (1)..(20)
<223>  r is g or a; w is a or t/u.

<400>  27
ccwccwccwg gwgcrtcwac                                                   20


<210>  28
<211>  30
<212>  DNA
<213>  Artificial

<220>
<223>  primer

<400>  28
cctttcagtt ggaattgagc actttagaac                                        30


<210>  29
<211>  30
<212>  DNA
<213>  Artificial

<220>
<223>  primer

<400>  29
gatactgcgt tcctacattt gttgtggatg                                        30


<210>  30
<211>  29
<212>  DNA
<213>  Artificial

<220>
<223>  primer

<400>  30

cgctgctcta tgtagctcta aagaaagag                                          29


<210>  31
<211>  28
<212>  DNA
<213>  Artificial

<220>
<223>  primer

<400>  31
cagcttgctt tcttacaggg tcatttgg                                           28


<210>  32
<211>  1245
<212>  DNA
<213>  Clostridium sticklandii


<220>
<221>  CDS
<222>  (1)..(1245)

<400>  32
atg agt tta aag gat aag ttt ttt tca cat gta agc caa gaa gat tgg       48
Met Ser Leu Lys Asp Lys Phe Phe Ser His Val Ser Gln Glu Asp Trp
1               5                   10                  15

aat gat tgg aaa tgg caa gta aga aat cgt ata gaa act gtt gaa gaa       96
Asn Asp Trp Lys Trp Gln Val Arg Asn Arg Ile Glu Thr Val Glu Glu
            20                  25                  30

ctt aaa aaa tat att cca ctt act cca gaa gaa gaa gaa ggg gta aaa      144
Leu Lys Lys Tyr Ile Pro Leu Thr Pro Glu Glu Glu Glu Gly Val Lys
        35                  40                  45

aga tgt ctt gat aca tta aga atg gct att act cca tac tat cta tcg      192
Arg Cys Leu Asp Thr Leu Arg Met Ala Ile Thr Pro Tyr Tyr Leu Ser
    50                  55                  60

cta att gat gta gaa aat cca aat gac cct gta aga aag caa gct gta      240
Leu Ile Asp Val Glu Asn Pro Asn Asp Pro Val Arg Lys Gln Ala Val
65                  70                  75                  80

cct ctt tct tta gag cta cat aga gca gcg tct gat caa gaa gac cca      288
Pro Leu Ser Leu Glu Leu His Arg Ala Ala Ser Asp Gln Glu Asp Pro
                85                  90                  95

ctt cat gaa gat gga gat tct cca gtt cca gga ctt aca cat aga tat      336
Leu His Glu Asp Gly Asp Ser Pro Val Pro Gly Leu Thr His Arg Tyr
            100                 105                 110

cct gat aga gtt ctt ctt tta atg act gat caa tgt tca atg tac tgc      384
Pro Asp Arg Val Leu Leu Leu Met Thr Asp Gln Cys Ser Met Tyr Cys
        115                 120                 125

aga cac tgt act aga aga aga ttc gct ggt caa aca gat tct gct gtt      432
Arg His Cys Thr Arg Arg Arg Phe Ala Gly Gln Thr Asp Ser Ala Val
    130                 135                 140

gat acg aag caa ata gat gct gcg att gaa tat atc aaa aat act cca      480
Asp Thr Lys Gln Ile Asp Ala Ala Ile Glu Tyr Ile Lys Asn Thr Pro
145                 150                 155                 160

caa gta aga gac gtt cta ctt tca gga gga gat gct cta tta atc tca      528
Gln Val Arg Asp Val Leu Leu Ser Gly Gly Asp Ala Leu Leu Ile Ser
                165                 170                 175

gat gaa aag ctt gag tac aca atc aaa aga ctt cgt gaa ata cca cac      576
Asp Glu Lys Leu Glu Tyr Thr Ile Lys Arg Leu Arg Glu Ile Pro His
            180                 185                 190

gtt gag gtt att cgt ata gga tca aga gta cca gtt gta atg cca caa      624
Val Glu Val Ile Arg Ile Gly Ser Arg Val Pro Val Val Met Pro Gln
        195                 200                 205

aga att aca cca gaa cta gtt tct atg ctt aaa aag tat cat cca gta      672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct      720
Trp Leu Asn Thr His Phe Asn His Pro Asn Glu Ile Thr Glu Glu Ser
225                 230                 235                 240

aaa aga gca tgt gag tta ctt gct gat gca ggt att cct ctt gga aat      768
Lys Arg Ala Cys Glu Leu Leu Ala Asp Ala Gly Ile Pro Leu Gly Asn
                245                 250                 255

caa agt gtg ctt ctt gca ggt gta aat gat tgc atg cac gtt atg aaa      816
Gln Ser Val Leu Leu Ala Gly Val Asn Asp Cys Met His Val Met Lys
            260                 265                 270

aaa cta gta aat gat tta gtt aaa ata aga gta aga cct tac tat att      864
Lys Leu Val Asn Asp Leu Val Lys Ile Arg Val Arg Pro Tyr Tyr Ile
        275                 280                 285

tat caa tgt gac ctt tca gtt gga att gag cac ttt aga act cca gtt      912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga      960
Ala Lys Gly Ile Glu Ile Ile Glu Gly Leu Arg Gly His Thr Ser Gly
305                 310                 315                 320

tac tgc gtt cct aca ttt gtt gtg gat gca cct ggt ggt gga gga aaa     1008
Tyr Cys Val Pro Thr Phe Val Val Asp Ala Pro Gly Gly Gly Gly Lys
                325                 330                 335

act cca gtt atg cca aac tat gtt att tca caa aat cac aat aaa gtt     1056
Thr Pro Val Met Pro Asn Tyr Val Ile Ser Gln Asn His Asn Lys Val
            340                 345                 350

att tta cgt aac ttt gaa ggt gta att aca act tac gat gag cct gat     1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat     1152
His Tyr Thr Phe His Cys Asp Cys Asp Val Cys Thr Gly Lys Thr Asn
    370                 375                 380

gtt cat aag gtt gga gta gct gga ctt ctt aat gga gag aca gcg aca      1200
Val His Lys Val Gly Val Ala Gly Leu Leu Asn Gly Glu Thr Ala Thr
385                 390                 395                 400

ctt gaa cca gag ggt ttg gaa aga aaa caa aga gga cat cac taa         1245
Leu Glu Pro Glu Gly Leu Glu Arg Lys Gln Arg Gly His His
                405                 410

<210>  33
<211>  414
<212>  PRT
<213>  Clostridium sticklandii

<400>  33

Met Ser Leu Lys Asp Lys Phe Phe Ser His Val Ser Gln Glu Asp Trp
1               5                   10                  15

Asn Asp Trp Lys Trp Gln Val Arg Asn Arg Ile Glu Thr Val Glu Glu
            20                  25                  30

Leu Lys Lys Tyr Ile Pro Leu Thr Pro Glu Glu Glu Glu Gly Val Lys
        35                  40                  45

Arg Cys Leu Asp Thr Leu Arg Met Ala Ile Thr Pro Tyr Tyr Leu Ser
    50                  55                  60

Leu Ile Asp Val Glu Asn Pro Asn Asp Pro Val Arg Lys Gln Ala Val
65                  70                  75                  80

Pro Leu Ser Leu Glu Leu His Arg Ala Ala Ser Asp Gln Glu Asp Pro
                85                  90                  95

Leu His Glu Asp Gly Asp Ser Pro Val Pro Gly Leu Thr His Arg Tyr
            100                 105                 110

Pro Asp Arg Val Leu Leu Leu Met Thr Asp Gln Cys Ser Met Tyr Cys
        115                 120                 125

Arg His Cys Thr Arg Arg Arg Phe Ala Gly Gln Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Lys Arg Leu Arg Glu Ile Pro His

180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys
            260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val Asp Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  34
<211>  1245

<212>  DNA
<213>  Clostridium sticklandii


<220>
<221>  CDS
<222>  (1)..(1245)

<400>  34
atg agt tta aag gat aag ttt ttt tca cat gta agc caa gaa gat tgg        48
Met Ser Leu Lys Asp Lys Phe Phe Ser His Val Ser Gln Glu Asp Trp
1               5                   10                  15

aat gat tgg aaa tgg caa gta aga aat cgt ata gaa act gtt gaa gaa        96
Asn Asp Trp Lys Trp Gln Val Arg Asn Arg Ile Glu Thr Val Glu Glu
            20                  25                  30

ctt aaa aaa tat att cca ctt act cca gaa gaa gaa gaa ggg gta aaa       144
Leu Lys Lys Tyr Ile Pro Leu Thr Pro Glu Glu Glu Glu Gly Val Lys
        35                  40                  45

cgc tgt ctt gat aca tta cgt atg gct att act cca tac tat cta tcg       192
Arg Cys Leu Asp Thr Leu Arg Met Ala Ile Thr Pro Tyr Tyr Leu Ser
    50                  55                  60

cta att gat gta gaa aat cca aat gac cct gta aga aag caa gct gta       240
Leu Ile Asp Val Glu Asn Pro Asn Asp Pro Val Arg Lys Gln Ala Val
65                  70                  75                  80

cct ctt tct tta gag ctg cat cgc gca gcg tct gat caa gaa gac cca       288
Pro Leu Ser Leu Glu Leu His Arg Ala Ala Ser Asp Gln Glu Asp Pro
                85                  90                  95

ctt cat gaa gat gga gat tct cca gtt cca gga ctt aca cat cgc tat       336
Leu His Glu Asp Gly Asp Ser Pro Val Pro Gly Leu Thr His Arg Tyr
            100                 105                 110

cct gat cgt gtt ctt ctt tta atg act gat caa tgt tca atg tac tgc       384
Pro Asp Arg Val Leu Leu Leu Met Thr Asp Gln Cys Ser Met Tyr Cys
        115                 120                 125

cgc tac tgt act cgt aga cgc ttc gct ggt caa aca gat tct gct gtt       432
Arg Tyr Cys Thr Arg Arg Arg Phe Ala Gly Gln Thr Asp Ser Ala Val
    130                 135                 140

gat acg aag caa ata gat gct gcg att gaa tat atc aaa aat act cca       480
Asp Thr Lys Gln Ile Asp Ala Ala Ile Glu Tyr Ile Lys Asn Thr Pro
145                 150                 155                 160

caa gta aga gac gtt cta ctt tca gga gga gat gct cta tta atc tca       528
Gln Val Arg Asp Val Leu Leu Ser Gly Gly Asp Ala Leu Leu Ile Ser
                165                 170                 175

gat gaa aag ctt gag tac aca atc aaa aga ctt cgt gaa ata cca cac       576
Asp Glu Lys Leu Glu Tyr Thr Ile Lys Arg Leu Arg Glu Ile Pro His
            180                 185                 190

gtt gag gtt att cgt att gga tca cgt gta cca gtt gta atg cca caa       624
Val Glu Val Ile Arg Ile Gly Ser Arg Val Pro Val Val Met Pro Gln
        195                 200                 205

cgt att aca cca gaa cta gtt tct atg ctt aaa aag tat cat cca gta      672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct      720
Trp Leu Asn Thr His Phe Asn His Pro Asn Glu Ile Thr Glu Glu Ser
225                 230                 235                 240

aaa cgt gca tgt gag tta ctt gct gat gca ggt att cct ctt gga aat      768
Lys Arg Ala Cys Glu Leu Leu Ala Asp Ala Gly Ile Pro Leu Gly Asn
                245                 250                 255

caa agt gtg ctt ctt gca ggt gta aat gat tgc atg cac gtt atg aaa      816
Gln Ser Val Leu Leu Ala Gly Val Asn Asp Cys Met His Val Met Lys
            260                 265                 270

aaa cta gta aat gat tta gtt aaa ata cgc gta cgt cct tac tat att      864
Lys Leu Val Asn Asp Leu Val Lys Ile Arg Val Arg Pro Tyr Tyr Ile
        275                 280                 285

tat caa tgt gac ctt tca gtt gga att gag cac ttt cgc act cca gtt      912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga      960
Ala Lys Gly Ile Glu Ile Ile Glu Gly Leu Arg Gly His Thr Ser Gly
305                 310                 315                 320

tac tgc gtt cct aca ttt gtt gtg gat gca cct ggt ggt gga gga aaa     1008
Tyr Cys Val Pro Thr Phe Val Val Asp Ala Pro Gly Gly Gly Gly Lys
                325                 330                 335

act cca gtt atg cca aac tat gtt att tca caa aat cac aat aaa gtt     1056
Thr Pro Val Met Pro Asn Tyr Val Ile Ser Gln Asn His Asn Lys Val
            340                 345                 350

att tta cgt aac ttt gaa ggt gta att aca act tac gat gag cct gat     1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat     1152
His Tyr Thr Phe His Cys Asp Cys Asp Val Cys Thr Gly Lys Thr Asn
    370                 375                 380

gtt cat aag gtt gga gta gct gga ctt cta aat gga gag aca gcg aca     1200
Val His Lys Val Gly Val Ala Gly Leu Leu Asn Gly Glu Thr Ala Thr
385                 390                 395                 400

ctt gaa cca gag ggt ttg gaa aga aaa caa aga gga cat cac taa         1245
Leu Glu Pro Glu Gly Leu Glu Arg Lys Gln Arg Gly His His
                405                 410


<210>  35
<211>  414
<212>  PRT
<213>  Clostridium sticklandii

<400>  35

Met Ser Leu Lys Asp Lys Phe Phe Ser His Val Ser Gln Glu Asp Trp

1               5                   10                  15

Asn Asp Trp Lys Trp Gln Val Arg Asn Arg Ile Glu Thr Val Glu Glu
            20                  25                  30

Leu Lys Lys Tyr Ile Pro Leu Thr Pro Glu Glu Glu Glu Gly Val Lys
        35                  40                  45

Arg Cys Leu Asp Thr Leu Arg Met Ala Ile Thr Pro Tyr Tyr Leu Ser
    50                  55                  60

Leu Ile Asp Val Glu Asn Pro Asn Asp Pro Val Arg Lys Gln Ala Val
65                  70                  75                  80

Pro Leu Ser Leu Glu Leu His Arg Ala Ala Ser Asp Gln Glu Asp Pro
                85                  90                  95

Leu His Glu Asp Gly Asp Ser Pro Val Pro Gly Leu Thr His Arg Tyr
            100                 105                 110

Pro Asp Arg Val Leu Leu Leu Met Thr Asp Gln Cys Ser Met Tyr Cys
        115                 120                 125

Arg Tyr Cys Thr Arg Arg Arg Phe Ala Gly Gln Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Lys Arg Leu Arg Glu Ile Pro His
            180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys
            260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val Asp Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  36
<211>  45
<212>  DNA
<213>  Artificial

<220>
<223>  mutagenesis primer

<400>  36
gaaatggcaa gtaagaaatc gtataaagac tgttgaagaa cttaa                    45

<210>  37
<211>  35
<212>  DNA
<213>  Artificial

<220>
<223>  mutagenesis primer

<400>  37
tcgcgcagcg tctgatatgg aagacccact tcatg                                  35


<210>  38
<211>  40
<212>  DNA
<213>  Artificial

<220>
<223>  mutagenesis primer

<400>  38
gactgatcaa tgttcagtat actgccgcca ctgtactcgt                             40


<210>  39
<211>  34
<212>  DNA
<213>  Artificial

<220>
<223>  mutagenesis primer

<400>  39
gttcctacat ttgttgtgca tgcacctggt ggtg                                   34


<210>  40
<211>  1245
<212>  DNA
<213>  Clostridium sticklandii


<220>
<221>  CDS
<222>  (1)..(1245)

<400>  40
atg agt tta aag gat aag ttt ttt tca cat gta agc caa gaa gat tgg       48
Met Ser Leu Lys Asp Lys Phe Phe Ser His Val Ser Gln Glu Asp Trp
1               5                   10                  15

aat gat tgg aaa tgg caa gta aga aat cgt ata aag act gtt gaa gaa       96
Asn Asp Trp Lys Trp Gln Val Arg Asn Arg Ile Lys Thr Val Glu Glu
            20                  25                  30

ctt aaa aaa tat att cca ctt act cca gaa gaa gaa gaa ggg gta aaa      144
Leu Lys Lys Tyr Ile Pro Leu Thr Pro Glu Glu Glu Glu Gly Val Lys
        35                  40                  45

cgc tgt ctt gat aca tta cgt atg gct att act cca tac tat cta tcg      192
Arg Cys Leu Asp Thr Leu Arg Met Ala Ile Thr Pro Tyr Tyr Leu Ser
    50                  55                  60

cta att gat gta gaa aat cca aat gac cct gta aga aag caa gct gta      240
Leu Ile Asp Val Glu Asn Pro Asn Asp Pro Val Arg Lys Gln Ala Val
65                  70                  75                  80

cct ctt tct tta gag ctg cat cgc gca gcg tct gat atg gaa gac cca      288

Pro Leu Ser Leu Glu Leu His Arg Ala Ala Ser Asp Met Glu Asp Pro
                85                  90                  95

ctt cat gaa gat gga gat tct cca gtt cca gga ctt aca cat cgc tat      336
Leu His Glu Asp Gly Asp Ser Pro Val Pro Gly Leu Thr His Arg Tyr
            100                 105                 110

cct gat cgt gtt ctt ctt tta atg act gat caa tgt tca gta tac tgc      384
Pro Asp Arg Val Leu Leu Leu Met Thr Asp Gln Cys Ser Val Tyr Cys
        115                 120                 125

cgc cac tgt act cgt aga cgc ttc gct ggt caa aca gat tct gct gtt      432
Arg His Cys Thr Arg Arg Arg Phe Ala Gly Gln Thr Asp Ser Ala Val
    130                 135                 140

gat acg aag caa ata gat gct gcg att gaa tat atc aaa aat act cca      480
Asp Thr Lys Gln Ile Asp Ala Ala Ile Glu Tyr Ile Lys Asn Thr Pro
145                 150                 155                 160

caa gta aga gac gtt cta ctt tca gga gga gat gct cta tta atc tca      528
Gln Val Arg Asp Val Leu Leu Ser Gly Gly Asp Ala Leu Leu Ile Ser
                165                 170                 175

gat gaa aag ctt gag tac aca atc aaa aga ctt cgt gaa ata cca cac      576
Asp Glu Lys Leu Glu Tyr Thr Ile Lys Arg Leu Arg Glu Ile Pro His
            180                 185                 190

gtt gag gtt att cgt att gga tca cgt gta cca gtt gta atg cca caa      624
Val Glu Val Ile Arg Ile Gly Ser Arg Val Pro Val Val Met Pro Gln
        195                 200                 205

cgt att aca cca gaa cta gtt tct atg ctt aaa aag tat cat cca gta      672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct      720
Trp Leu Asn Thr His Phe Asn His Pro Asn Glu Ile Thr Glu Glu Ser
225                 230                 235                 240

aaa cgt gca tgt gag tta ctt gct gat gca ggt att cct ctt gga aat      768
Lys Arg Ala Cys Glu Leu Leu Ala Asp Ala Gly Ile Pro Leu Gly Asn
                245                 250                 255

caa agt gtg ctt ctt gca ggt gta aat gat tgc atg cac gtt atg aaa      816
Gln Ser Val Leu Leu Ala Gly Val Asn Asp Cys Met His Val Met Lys
            260                 265                 270

aaa cta gta aat gat tta gtt aaa ata cgc gta cgt cct tac tat att      864
Lys Leu Val Asn Asp Leu Val Lys Ile Arg Val Arg Pro Tyr Tyr Ile
        275                 280                 285

tat caa tgt gac ctt tca gtt gga att gag cac ttt cgc act cca gtt      912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga      960
Ala Lys Gly Ile Glu Ile Ile Glu Gly Leu Arg Gly His Thr Ser Gly
305                 310                 315                 320

tac tgc gtt cct aca ttt gtt gtg cat gca cct ggt ggt gga gga aaa     1008
Tyr Cys Val Pro Thr Phe Val Val His Ala Pro Gly Gly Gly Gly Lys

325                 330                 335

act cca gtt atg cca aac tat gtt att tca caa aat cac aat aaa gtt      1056
Thr Pro Val Met Pro Asn Tyr Val Ile Ser Gln Asn His Asn Lys Val
            340                 345                 350

att tta cgt aac ttt gaa ggt gta att aca act tac gat gag cct gat      1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat      1152
His Tyr Thr Phe His Cys Asp Cys Asp Val Cys Thr Gly Lys Thr Asn
    370                 375                 380

gtt cat aag gtt gga gta gct gga ctt cta aat gga gag aca gcg aca      1200
Val His Lys Val Gly Val Ala Gly Leu Leu Asn Gly Glu Thr Ala Thr
385                 390                 395                 400

ctt gaa cca gag ggt ttg gaa aga aaa caa aga gga cat cac taa          1245
Leu Glu Pro Glu Gly Leu Glu Arg Lys Gln Arg Gly His His
                405                 410


<210>  41
<211>  414
<212>  PRT
<213>  Clostridium sticklandii

<400>  41

Met Ser Leu Lys Asp Lys Phe Phe Ser His Val Ser Gln Glu Asp Trp
1               5                   10                  15


Asn Asp Trp Lys Trp Gln Val Arg Asn Arg Ile Lys Thr Val Glu Glu
            20                  25                  30


Leu Lys Lys Tyr Ile Pro Leu Thr Pro Glu Glu Glu Glu Gly Val Lys
        35                  40                  45


Arg Cys Leu Asp Thr Leu Arg Met Ala Ile Thr Pro Tyr Tyr Leu Ser
    50                  55                  60


Leu Ile Asp Val Glu Asn Pro Asn Asp Pro Val Arg Lys Gln Ala Val
65                  70                  75                  80


Pro Leu Ser Leu Glu Leu His Arg Ala Ala Ser Asp Met Glu Asp Pro
                85                  90                  95


Leu His Glu Asp Gly Asp Ser Pro Val Pro Gly Leu Thr His Arg Tyr
            100                 105                 110


Pro Asp Arg Val Leu Leu Leu Met Thr Asp Gln Cys Ser Val Tyr Cys
        115                 120                 125

Arg His Cys Thr Arg Arg Arg Phe Ala Gly Gln Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Lys Arg Leu Arg Glu Ile Pro His
            180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys
            260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val His Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  42
<211>  1245
<212>  DNA
<213>  Clostridium sticklandii

<220>
<221>  CDS
<222>  (1)..(1245)

<400>  42
atg agt tta aag gat aag ttt ttt aca cat gta agc caa gaa gat tgg       48
Met Ser Leu Lys Asp Lys Phe Phe Thr His Val Ser Gln Glu Asp Trp
1               5                   10                  15

aat gat tgg aaa tgg caa gta aga aat cgt ata aag act gtt gaa gaa       96
Asn Asp Trp Lys Trp Gln Val Arg Asn Arg Ile Lys Thr Val Glu Glu
            20                  25                  30

ctt aaa aaa tat att cca ctt act cca gaa gaa gaa gaa ggg gta aaa      144
Leu Lys Lys Tyr Ile Pro Leu Thr Pro Glu Glu Glu Glu Gly Val Lys
        35                  40                  45

cgc tgt ctt gat aca tta cgt atg gct att act cca tac tat cta tcg      192
Arg Cys Leu Asp Thr Leu Arg Met Ala Ile Thr Pro Tyr Tyr Leu Ser
    50                  55                  60

cta att gat gta gaa aat cca aat gac cct gta aga aag caa gct gta      240
Leu Ile Asp Val Glu Asn Pro Asn Asp Pro Val Arg Lys Gln Ala Val
65                  70                  75                  80

cct ctt tct tta gag ctg cat cgc gca gcg tct gat atg gaa gac cca      288
Pro Leu Ser Leu Glu Leu His Arg Ala Ala Ser Asp Met Glu Asp Pro
                85                  90                  95

ctt cat gaa gat gga gat tct cca gtt cca gga ctt aca cat cgc tat      336
Leu His Glu Asp Gly Asp Ser Pro Val Pro Gly Leu Thr His Arg Tyr
            100                 105                 110

cct gat cgc gtt ctt ctt tta atg act gat caa tgt tca gta tac tgc      384
Pro Asp Arg Val Leu Leu Leu Met Thr Asp Gln Cys Ser Val Tyr Cys
        115                 120                 125

cgc cac tgt act cgt aga cgc ttc gct ggt cga aca gat tct gct gtt      432
Arg His Cys Thr Arg Arg Arg Phe Ala Gly Arg Thr Asp Ser Ala Val
    130                 135                 140

gat acg aag caa ata gat gct gcg att gaa tat atc aaa aat act cca      480
Asp Thr Lys Gln Ile Asp Ala Ala Ile Glu Tyr Ile Lys Asn Thr Pro

145                 150                 155                 160

caa gta aga gac gtt cta ctt tca gga gga gat gct cta tta atc tca       528
Gln Val Arg Asp Val Leu Leu Ser Gly Gly Asp Ala Leu Leu Ile Ser
                165                 170                 175

gat gaa aag ctt gag tac aca atc aga aga ctt cgt gaa ata cca cac       576
Asp Glu Lys Leu Glu Tyr Thr Ile Arg Arg Leu Arg Glu Ile Pro His
            180                 185                 190

gtt gag gtt att cgt att gga tca cgt gta cca gtt gta atg cca caa       624
Val Glu Val Ile Arg Ile Gly Ser Arg Val Pro Val Val Met Pro Gln
        195                 200                 205

cgt att aca cca gaa cta gtt tct atg ctt aaa aag tat cat cca gta       672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct       720
Trp Leu Asn Thr His Phe Asn His Pro Asn Glu Ile Thr Glu Glu Ser
225                 230                 235                 240

aaa cgt gca tgt gag tta ctt gct gat gca ggt att cct ctt gga aat       768
Lys Arg Ala Cys Glu Leu Leu Ala Asp Ala Gly Ile Pro Leu Gly Asn
                245                 250                 255

caa agt gtg ctt ctt gca ggt gta aat gat tgc atg cac gtt atg aaa       816
Gln Ser Val Leu Leu Ala Gly Val Asn Asp Cys Met His Val Met Lys
            260                 265                 270

aaa cta gta aat gac tta gtt aaa ata cgc gta cgt cct tac tat att       864
Lys Leu Val Asn Asp Leu Val Lys Ile Arg Val Arg Pro Tyr Tyr Ile
        275                 280                 285

tat caa tgt gac ctt tca gtt gga att gag cac ttt cgc act cca gtt       912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga       960
Ala Lys Gly Ile Glu Ile Ile Glu Gly Leu Arg Gly His Thr Ser Gly
305                 310                 315                 320

tac tgc gtt cct aca ttt gtt gtg cat gca cct ggt ggt gga gga aaa      1008
Tyr Cys Val Pro Thr Phe Val Val His Ala Pro Gly Gly Gly Gly Lys
                325                 330                 335

act cca gtt atg cca aac tat gtt att tca caa aat cac aat aaa gtt      1056
Thr Pro Val Met Pro Asn Tyr Val Ile Ser Gln Asn His Asn Lys Val
            340                 345                 350

att tta cgt aac ttt gaa ggt gta att aca act tac gat gag cct gat      1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat      1152
His Tyr Thr Phe His Cys Asp Cys Asp Val Cys Thr Gly Lys Thr Asn
    370                 375                 380

gtt cat aag gtt gga gta gct gga ctt cta aat gga gag aca gcg aca      1200
Val His Lys Val Gly Val Ala Gly Leu Leu Asn Gly Glu Thr Ala Thr
385                 390                 395                 400

ctt gaa cct gag ggt ttg gaa aga aaa caa aga gga cat cac taa          1245
Leu Glu Pro Glu Gly Leu Glu Arg Lys Gln Arg Gly His His
                405                 410

<210>  43
<211>  414
<212>  PRT
<213>  Clostridium sticklandii

<400>  43

Met Ser Leu Lys Asp Lys Phe Phe Thr His Val Ser Gln Glu Asp Trp
1               5                   10                  15

Asn Asp Trp Lys Trp Gln Val Arg Asn Arg Ile Lys Thr Val Glu Glu
            20                  25                  30

Leu Lys Lys Tyr Ile Pro Leu Thr Pro Glu Glu Glu Glu Gly Val Lys
        35                  40                  45

Arg Cys Leu Asp Thr Leu Arg Met Ala Ile Thr Pro Tyr Tyr Leu Ser
    50                  55                  60

Leu Ile Asp Val Glu Asn Pro Asn Asp Pro Val Arg Lys Gln Ala Val
65                  70                  75                  80

Pro Leu Ser Leu Glu Leu His Arg Ala Ala Ser Asp Met Glu Asp Pro
                85                  90                  95

Leu His Glu Asp Gly Asp Ser Pro Val Pro Gly Leu Thr His Arg Tyr
            100                 105                 110

Pro Asp Arg Val Leu Leu Leu Met Thr Asp Gln Cys Ser Val Tyr Cys
        115                 120                 125

Arg His Cys Thr Arg Arg Arg Phe Ala Gly Arg Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Arg Arg Leu Arg Glu Ile Pro His
            180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys
            260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val His Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  44
<211>  1245
<212>  DNA
<213>  Clostridium sticklandii

<220>
<221>  CDS
<222>  (1)..(1245)

<400>  44
atg agt tta aag gat aag ttt ttt tca cat gta agc caa gaa gat tgg       48
Met Ser Leu Lys Asp Lys Phe Phe Ser His Val Ser Gln Glu Asp Trp
1               5                   10                  15

aat gat tgg aaa tgg caa gta aga aat cgt ata aag act gct gaa gaa       96
Asn Asp Trp Lys Trp Gln Val Arg Asn Arg Ile Lys Thr Ala Glu Glu
            20                  25                  30

ctt aaa aaa tat att cca ctt act cca gaa gaa gaa gaa ggg gta aaa      144
Leu Lys Lys Tyr Ile Pro Leu Thr Pro Glu Glu Glu Glu Gly Val Lys
        35                  40                  45

cgc tgt cat gat aca tta cgt atg gct att act cca tac tat cta tcg      192
Arg Cys His Asp Thr Leu Arg Met Ala Ile Thr Pro Tyr Tyr Leu Ser
    50                  55                  60

cta att gat gta gga aat cca aat gac cct gta aga aag caa gct gta      240
Leu Ile Asp Val Gly Asn Pro Asn Asp Pro Val Arg Lys Gln Ala Val
65                  70                  75                  80

cct ctt tct tta gag ctg cat cgc gca gcg tct gat atg gaa gac cca      288
Pro Leu Ser Leu Glu Leu His Arg Ala Ala Ser Asp Met Glu Asp Pro
                85                  90                  95

ctt cat gaa gat gga gat tct cca gtt cca gga ctt aca cat cgc tat      336
Leu His Glu Asp Gly Asp Ser Pro Val Pro Gly Leu Thr His Arg Tyr
            100                 105                 110

cct gat cgt gtt ctt ctt tta atg act gat cta tgt tca gta tac tgc      384
Pro Asp Arg Val Leu Leu Leu Met Thr Asp Leu Cys Ser Val Tyr Cys
        115                 120                 125

cgc cac tgt act cgt aga cgc ttc gct ggt caa aca gat tct gct gtt      432
Arg His Cys Thr Arg Arg Arg Phe Ala Gly Gln Thr Asp Ser Ala Val
    130                 135                 140

gat acg aag caa ata gat gct gcg att gaa tat atc aaa aat act cca      480
Asp Thr Lys Gln Ile Asp Ala Ala Ile Glu Tyr Ile Lys Asn Thr Pro
145                 150                 155                 160

caa gta aga gac gtt cta ctt tca gga gga gat gca cta tta atc tca      528
Gln Val Arg Asp Val Leu Leu Ser Gly Gly Asp Ala Leu Leu Ile Ser
                165                 170                 175

gat gaa aag ctt gag tac aca atc aaa aga ctt cgt gaa ata cca cac      576
Asp Glu Lys Leu Glu Tyr Thr Ile Lys Arg Leu Arg Glu Ile Pro His
            180                 185                 190

gtt gag gtt att cgt att gga tca cgt gta cca gtt gta atg cca caa      624
Val Glu Val Ile Arg Ile Gly Ser Arg Val Pro Val Val Met Pro Gln
        195                 200                 205

cgt att aca cca gaa cta gtt tct atg ctt aaa aag tat cat cca gta      672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct      720
Trp Leu Asn Thr His Phe Asn His Pro Asn Glu Ile Thr Glu Glu Ser
225                 230                 235                 240

aaa cgt gca tgt gag tta ctt gct gat gca ggt att cct ctt gga aat      768
Lys Arg Ala Cys Glu Leu Leu Ala Asp Ala Gly Ile Pro Leu Gly Asn
                245                 250                 255

caa agt gtg ctt cta gca ggt gta aat gat tgc atg cac gtt atg aaa      816
Gln Ser Val Leu Leu Ala Gly Val Asn Asp Cys Met His Val Met Lys
            260                 265                 270

aaa cta gta aat gat tta gtt aaa ata cgc gta cgt cct tac tat att      864
Lys Leu Val Asn Asp Leu Val Lys Ile Arg Val Arg Pro Tyr Tyr Ile
        275                 280                 285

tat caa tgt gac ctt tca gtt gga att gag cac ttt cgc act cca gtt      912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga      960
Ala Lys Gly Ile Glu Ile Ile Glu Gly Leu Arg Gly His Thr Ser Gly
305                 310                 315                 320

tac tgc gtt cct aca ttt gtt gtg cat gca cct ggt ggt gga gga aaa     1008
Tyr Cys Val Pro Thr Phe Val Val His Ala Pro Gly Gly Gly Gly Lys
                325                 330                 335

act cca gtt atg cca aac tat gtt att tca caa aat cac aat aaa gtt     1056
Thr Pro Val Met Pro Asn Tyr Val Ile Ser Gln Asn His Asn Lys Val
            340                 345                 350

att tta cgt aac ttt gaa ggt gta att aca act tac gat gag cct gat     1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat     1152
His Tyr Thr Phe His Cys Asp Cys Asp Val Cys Thr Gly Lys Thr Asn
    370                 375                 380

gtt cat aag gtt gga gta gct gga ctt cta aat gga gag aca gcg aca     1200
Val His Lys Val Gly Val Ala Gly Leu Leu Asn Gly Glu Thr Ala Thr
385                 390                 395                 400

ctt gaa cca gag ggt ttg gaa aga aaa caa aga gga cat cac taa         1245
Leu Glu Pro Glu Gly Leu Glu Arg Lys Gln Arg Gly His His
                405                 410

<210>  45
<211>  414
<212>  PRT
<213>  Clostridium sticklandii

<400>  45

Met Ser Leu Lys Asp Lys Phe Phe Ser His Val Ser Gln Glu Asp Trp
1               5                   10                  15

Asn Asp Trp Lys Trp Gln Val Arg Asn Arg Ile Lys Thr Ala Glu Glu
            20                  25                  30

Leu Lys Lys Tyr Ile Pro Leu Thr Pro Glu Glu Glu Glu Gly Val Lys
        35                  40                  45

Arg Cys His Asp Thr Leu Arg Met Ala Ile Thr Pro Tyr Tyr Leu Ser
    50                  55                  60

Leu Ile Asp Val Gly Asn Pro Asn Asp Pro Val Arg Lys Gln Ala Val
65                  70                  75                  80

Pro Leu Ser Leu Glu Leu His Arg Ala Ala Ser Asp Met Glu Asp Pro
                85                  90                  95

Leu His Glu Asp Gly Asp Ser Pro Val Pro Gly Leu Thr His Arg Tyr
            100                 105                 110

Pro Asp Arg Val Leu Leu Leu Met Thr Asp Leu Cys Ser Val Tyr Cys
        115                 120                 125

Arg His Cys Thr Arg Arg Arg Phe Ala Gly Gln Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Lys Arg Leu Arg Glu Ile Pro His
            180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys

260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val His Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  46
<211>  1245
<212>  DNA
<213>  Clostridium sticklandii

<220>
<221>  CDS
<222>  (1)..(1245)

<400>  46
atg agt tta aag gat aag ttt ttt tca cat gta agc caa gaa gat tgg       48
Met Ser Leu Lys Asp Lys Phe Phe Ser His Val Ser Gln Glu Asp Trp
1               5                   10                  15

aat gat tgg aaa tgg caa gta aga aat cgt ata aag act gtt gaa gaa       96
Asn Asp Trp Lys Trp Gln Val Arg Asn Arg Ile Lys Thr Val Glu Glu
            20                  25                  30

ctt aaa aaa tat att cca ctt act cca gaa gaa gaa gaa ggg gta aaa      144
Leu Lys Lys Tyr Ile Pro Leu Thr Pro Glu Glu Glu Glu Gly Val Lys
        35                  40                  45

cgc cgt ctt gat aca tta cgt atg gct att act cca tac tat cta tcg 192
Arg Arg Leu Asp Thr Leu Arg Met Ala Ile Thr Pro Tyr Tyr Leu Ser
    50                  55                  60

cta att gat gta gaa aat cca aat gac cct gta aga aag caa gct gta 240
Leu Ile Asp Val Glu Asn Pro Asn Asp Pro Val Arg Lys Gln Ala Val
65                  70                  75                  80

cct ctt tct tta gag ctg cat cgc gca gcg tct gat atg gaa gac cca 288
Pro Leu Ser Leu Glu Leu His Arg Ala Ala Ser Asp Met Glu Asp Pro
                85                  90                  95

ctt cat gaa gat gga gat tct cca gtt cca gga ctt aca cat cgc tat 336
Leu His Glu Asp Gly Asp Ser Pro Val Pro Gly Leu Thr His Arg Tyr
            100                 105                 110

cct gat cgt gtt ctt ctt tta atg act gat caa tgt tca gta tac tgc 384
Pro Asp Arg Val Leu Leu Leu Met Thr Asp Gln Cys Ser Val Tyr Cys
        115                 120                 125

cgc cac tgt act cgt aga cgc ttc gct ggt caa aca gat tct gct gtt 432
Arg His Cys Thr Arg Arg Arg Phe Ala Gly Gln Thr Asp Ser Ala Val
    130                 135                 140

gat acg aag caa ata gat gct gcg att gaa tat atc aaa aat act cca 480
Asp Thr Lys Gln Ile Asp Ala Ala Ile Glu Tyr Ile Lys Asn Thr Pro
145                 150                 155                 160

caa gta aga gac gtt cta ctt tca gga gga gat gct cta tta atc tca 528
Gln Val Arg Asp Val Leu Leu Ser Gly Gly Asp Ala Leu Leu Ile Ser
                165                 170                 175

gat gaa aag ctt gag tac aca atc aaa aga ctt cgt gaa ata cca cac 576
Asp Glu Lys Leu Glu Tyr Thr Ile Lys Arg Leu Arg Glu Ile Pro His
            180                 185                 190

gtt gag gtt att cgt att gga tca cgt gta cca gtt gta atg cca caa 624
Val Glu Val Ile Arg Ile Gly Ser Arg Val Pro Val Val Met Pro Gln
        195                 200                 205

cgt att aca cca gaa cta gtt tct atg ctt aaa aag tat cat cca gta 672
Arg Ile Thr Pro Glu Leu Val Ser Met Leu Lys Lys Tyr His Pro Val
    210                 215                 220

tgg tta aat aca cac ttc aac cat cct aat gaa att act gaa gag tct 720
Trp Leu Asn Thr His Phe Asn His Pro Asn Glu Ile Thr Glu Glu Ser
225                 230                 235                 240

aaa cgt gca tgt gag tta ctt gct gat gca ggt att cct ctt gga aat 768
Lys Arg Ala Cys Glu Leu Leu Ala Asp Ala Gly Ile Pro Leu Gly Asn
                245                 250                 255

caa agt gtg ctt ctt gca ggt gta aat gat tgc atg cac gtt atg aaa 816
Gln Ser Val Leu Leu Ala Gly Val Asn Asp Cys Met His Val Met Lys
            260                 265                 270

aaa cta gta aat gat tta gtt aaa ata cgc gta cgt cct tac tat att 864
Lys Leu Val Asn Asp Leu Val Lys Ile Arg Val Arg Pro Tyr Tyr Ile
        275                 280                 285

tat caa tgt gac ctt tca gtt gga att gag cac ttt cgc act cca gtt      912
Tyr Gln Cys Asp Leu Ser Val Gly Ile Glu His Phe Arg Thr Pro Val
    290                 295                 300

gca aag gga ata gaa ata att gaa ggc tta aga gga cat act tca gga      960
Ala Lys Gly Ile Glu Ile Ile Glu Gly Leu Arg Gly His Thr Ser Gly
305                 310                 315                 320

tac tgc gtt cct aca ttt gtt gtg cat gca cct ggt ggt gga gga aaa     1008
Tyr Cys Val Pro Thr Phe Val Val His Ala Pro Gly Gly Gly Gly Lys
                325                 330                 335

act cca gtt atg cca aac tat gtt att tca caa aat cac aat aaa gtt     1056
Thr Pro Val Met Pro Asn Tyr Val Ile Ser Gln Asn His Asn Lys Val
            340                 345                 350

att tta cgt aac ttt gaa ggt gta att aca act tac gat gag cct gat     1104
Ile Leu Arg Asn Phe Glu Gly Val Ile Thr Thr Tyr Asp Glu Pro Asp
        355                 360                 365

cat tat act ttc cac tgt gac tgt gat gta tgc act gga aaa aca aat     1152
His Tyr Thr Phe His Cys Asp Cys Asp Val Cys Thr Gly Lys Thr Asn
    370                 375                 380

gtt cat aag gtt gga gta gct gga ctt cta aat gga gag aca gcg aca     1200
Val His Lys Val Gly Val Ala Gly Leu Leu Asn Gly Glu Thr Ala Thr
385                 390                 395                 400

ctt gaa cca gag ggt ttg gaa aga aaa caa aga gga cat cac taa         1245
Leu Glu Pro Glu Gly Leu Glu Arg Lys Gln Arg Gly His His
                405                 410


<210>  47
<211>  414
<212>  PRT
<213>  Clostridium sticklandii

<400>  47

Met Ser Leu Lys Asp Lys Phe Phe Ser His Val Ser Gln Glu Asp Trp
1               5                   10                  15


Asn Asp Trp Lys Trp Gln Val Arg Asn Arg Ile Lys Thr Val Glu Glu
            20                  25                  30


Leu Lys Lys Tyr Ile Pro Leu Thr Pro Glu Glu Glu Glu Gly Val Lys
        35                  40                  45


Arg Arg Leu Asp Thr Leu Arg Met Ala Ile Thr Pro Tyr Tyr Leu Ser
    50                  55                  60


Leu Ile Asp Val Glu Asn Pro Asn Asp Pro Val Arg Lys Gln Ala Val
65                  70                  75                  80


Pro Leu Ser Leu Glu Leu His Arg Ala Ala Ser Asp Met Glu Asp Pro

                85                  90                  95

Leu His Glu Asp Gly Asp Ser Pro Val Pro Gly Leu Thr His Arg Tyr
            100                 105                 110

Pro Asp Arg Val Leu Leu Leu Met Thr Asp Gln Cys Ser Val Tyr Cys
        115                 120                 125

Arg His Cys Thr Arg Arg Arg Phe Ala Gly Gln Thr Asp Ser Ala Val
    130                 135                 140

Asp Thr Lys Gln Ile Asp Ala Ala Ile Glu Tyr Ile Lys Asn Thr Pro
145                 150                 155                 160

Gln Val Arg Asp Val Leu Leu Ser Gly Gly Asp Ala Leu Leu Ile Ser
                165                 170                 175

Asp Glu Lys Leu Glu Tyr Thr Ile Lys Arg Leu Arg Glu Ile Pro His
            180                 185                 190

Val Glu Val Ile Arg Ile Gly Ser Arg Val Pro Val Val Met Pro Gln
        195                 200                 205

Arg Ile Thr Pro Glu Leu Val Ser Met Leu Lys Lys Tyr His Pro Val
    210                 215                 220

Trp Leu Asn Thr His Phe Asn His Pro Asn Glu Ile Thr Glu Glu Ser
225                 230                 235                 240

Lys Arg Ala Cys Glu Leu Leu Ala Asp Ala Gly Ile Pro Leu Gly Asn
                245                 250                 255

Gln Ser Val Leu Leu Ala Gly Val Asn Asp Cys Met His Val Met Lys
            260                 265                 270

Lys Leu Val Asn Asp Leu Val Lys Ile Arg Val Arg Pro Tyr Tyr Ile
        275                 280                 285

Tyr Gln Cys Asp Leu Ser Val Gly Ile Glu His Phe Arg Thr Pro Val
    290                 295                 300

Ala Lys Gly Ile Glu Ile Ile Glu Gly Leu Arg Gly His Thr Ser Gly
305                 310                 315                 320

Tyr Cys Val Pro Thr Phe Val Val His Ala Pro Gly Gly Gly Gly Lys
                325                 330                 335

Thr Pro Val Met Pro Asn Tyr Val Ile Ser Gln Asn His Asn Lys Val
            340                 345                 350

Ile Leu Arg Asn Phe Glu Gly Val Ile Thr Thr Tyr Asp Glu Pro Asp
        355                 360                 365

His Tyr Thr Phe His Cys Asp Cys Asp Val Cys Thr Gly Lys Thr Asn
    370                 375                 380

Val His Lys Val Gly Val Ala Gly Leu Leu Asn Gly Glu Thr Ala Thr
385                 390                 395                 400

Leu Glu Pro Glu Gly Leu Glu Arg Lys Gln Arg Gly His His
                405                 410

<210>  48
<211>  1251
<212>  DNA
<213>  Porphyromonas gingivalis

<220>
<221>  CDS
<222>  (1)..(1251)

<400>  48
atg gca gaa agt cgt aga aag tat tat ttc cct gat gtc acc gat gag       48
Met Ala Glu Ser Arg Arg Lys Tyr Tyr Phe Pro Asp Val Thr Asp Glu
1               5                   10                  15

caa tgg tac gac tgg cat tgg cag gtc ctc aat cga att aag acg ctc       96
Gln Trp Tyr Asp Trp His Trp Gln Val Leu Asn Arg Ile Lys Thr Leu
            20                  25                  30

gac cag ctg aaa aag tac gtt aca ctc acc gct gaa gaa gaa gag gga      144
Asp Gln Leu Lys Lys Tyr Val Thr Leu Thr Ala Glu Glu Glu Glu Gly
        35                  40                  45

gta aaa gaa tcg ccc aaa gta ctc cga atg gct atc aca cct tat tat      192
Val Lys Glu Ser Pro Lys Val Leu Arg Met Ala Ile Thr Pro Tyr Tyr
    50                  55                  60

ttg agt ttg ata gac ccc gag aat cct aat tgt ccg att cgt aaa caa      240
Leu Ser Leu Ile Asp Pro Glu Asn Pro Asn Cys Pro Ile Arg Lys Gln
65                  70                  75                  80

gcc att cct act caa cag gaa ctg gta cgt gct cct gaa gat cag gta      288
Ala Ile Pro Thr Gln Gln Glu Leu Val Arg Ala Pro Glu Asp Gln Val
                85                  90                  95

gac cca ctt agt gaa gat gaa gat tcg ccc gta ccc gga ctg act cat      336
Asp Pro Leu Ser Glu Asp Glu Asp Ser Pro Val Pro Gly Leu Thr His
            100                 105                 110

cgt tat ccg gat cgt gta ttg ttc ctt atc acg gac aaa tgt tcg atg      384
Arg Tyr Pro Asp Arg Val Leu Phe Leu Ile Thr Asp Lys Cys Ser Met
        115                 120                 125

tac tgt cgt cat tgt act cgc cgt cgc ttc gca gga cag aaa gat gct      432
Tyr Cys Arg His Cys Thr Arg Arg Arg Phe Ala Gly Gln Lys Asp Ala
    130                 135                 140

tct tct cct tct gag cgc atc gat cga tgc att gac tat ata gcc aat      480
Ser Ser Pro Ser Glu Arg Ile Asp Arg Cys Ile Asp Tyr Ile Ala Asn
145                 150                 155                 160

aca ccg aca gtc cgc gat gtt ttg cta tcg gga ggc gat gcc ctc ctt      528
Thr Pro Thr Val Arg Asp Val Leu Leu Ser Gly Gly Asp Ala Leu Leu
                165                 170                 175

gtc agc gac gaa cgc ttg gaa tac ata ttg aag cgt ctg cgc gaa gta      576
Val Ser Asp Glu Arg Leu Glu Tyr Ile Leu Lys Arg Leu Arg Glu Val
            180                 185                 190

cct cat gtg gag att gtt cgt ata gga agc cgt acg ccg gta gtc ctc      624
Pro His Val Glu Ile Val Arg Ile Gly Ser Arg Thr Pro Val Val Leu
        195                 200                 205

cct cag cgt ata acg cct caa ttg gtg gat atg ctc aaa aaa tat cat      672
Pro Gln Arg Ile Thr Pro Gln Leu Val Asp Met Leu Lys Lys Tyr His
    210                 215                 220

ccg gtg tgg ctg aac act cac ttc aac cac ccg aat gaa gtt acc gaa      720
Pro Val Trp Leu Asn Thr His Phe Asn His Pro Asn Glu Val Thr Glu
225                 230                 235                 240

gaa gca gtg gag gct tgt gaa aga atg gcc aat gcc ggt att ccg ttg      768
Glu Ala Val Glu Ala Cys Glu Arg Met Ala Asn Ala Gly Ile Pro Leu
                245                 250                 255

ggt aac caa acg gtt tta ttg cgt gga atc aat gat tgt aca cat gtg      816
Gly Asn Gln Thr Val Leu Leu Arg Gly Ile Asn Asp Cys Thr His Val
            260                 265                 270

atg aag aga ttg gta cat ttg ctg gta aag atg cgt gtg cgt cct tac      864
Met Lys Arg Leu Val His Leu Leu Val Lys Met Arg Val Arg Pro Tyr
        275                 280                 285

tat ata tat gta tgc gat ctt tcg ctt gga ata ggt cat ttc cgc acg      912
Tyr Ile Tyr Val Cys Asp Leu Ser Leu Gly Ile Gly His Phe Arg Thr
    290                 295                 300

ccg gta tct aaa gga atc gaa att atc gaa aat ttg cgc gga cac acc      960
Pro Val Ser Lys Gly Ile Glu Ile Ile Glu Asn Leu Arg Gly His Thr
305                 310                 315                 320

tcg ggc tat gca gtt cct acc ttt gtg gta ggt gct ccg ggg ggt ggt     1008
Ser Gly Tyr Ala Val Pro Thr Phe Val Val Gly Ala Pro Gly Gly Gly
                325                 330                 335

ggt aag ata cct gta acg ccg aac tat gtt gta tct cag tcc cca cga     1056
Gly Lys Ile Pro Val Thr Pro Asn Tyr Val Val Ser Gln Ser Pro Arg
            340                 345                 350

cat gtg gtt ctt cgc aat tat gaa ggt gtt atc aca acc tat acg gag     1104

His Val Val Leu Arg Asn Tyr Glu Gly Val Ile Thr Thr Tyr Thr Glu
        355                 360                 365

ccg gag aat tat cat gag gag tgc gat tgt gag gac tgt cga gcc ggt      1152
Pro Glu Asn Tyr His Glu Glu Cys Asp Cys Glu Asp Cys Arg Ala Gly
    370                 375                 380

aag cat aaa gag ggt gta gct gca ctt tcc gga ggt cag cag ttg gct      1200
Lys His Lys Glu Gly Val Ala Ala Leu Ser Gly Gly Gln Gln Leu Ala
385                 390                 395                 400

atc gag cct tcc gac tta gct cgc aaa aaa cgc aag ttt gat aag aac      1248
Ile Glu Pro Ser Asp Leu Ala Arg Lys Lys Arg Lys Phe Asp Lys Asn
                405                 410                 415

tga                                                                  1251


<210>  49
<211>  416
<212>  PRT
<213>  Porphyromonas gingivalis

<400>  49

Met Ala Glu Ser Arg Arg Lys Tyr Tyr Phe Pro Asp Val Thr Asp Glu
1               5                   10                  15


Gln Trp Tyr Asp Trp His Trp Gln Val Leu Asn Arg Ile Lys Thr Leu
            20                  25                  30


Asp Gln Leu Lys Lys Tyr Val Thr Leu Thr Ala Glu Glu Glu Glu Gly
        35                  40                  45


Val Lys Glu Ser Pro Lys Val Leu Arg Met Ala Ile Thr Pro Tyr Tyr
    50                  55                  60


Leu Ser Leu Ile Asp Pro Glu Asn Pro Asn Cys Pro Ile Arg Lys Gln
65                  70                  75                  80


Ala Ile Pro Thr Gln Gln Glu Leu Val Arg Ala Pro Glu Asp Gln Val
                85                  90                  95


Asp Pro Leu Ser Glu Asp Glu Asp Ser Pro Val Pro Gly Leu Thr His
            100                 105                 110


Arg Tyr Pro Asp Arg Val Leu Phe Leu Ile Thr Asp Lys Cys Ser Met
        115                 120                 125


Tyr Cys Arg His Cys Thr Arg Arg Arg Phe Ala Gly Gln Lys Asp Ala
    130                 135                 140

Ser Ser Pro Ser Glu Arg Ile Asp Arg Cys Ile Asp Tyr Ile Ala Asn
145                 150                 155                 160

Thr Pro Thr Val Arg Asp Val Leu Leu Ser Gly Gly Asp Ala Leu Leu
                165                 170                 175

Val Ser Asp Glu Arg Leu Glu Tyr Ile Leu Lys Arg Leu Arg Glu Val
            180                 185                 190

Pro His Val Glu Ile Val Arg Ile Gly Ser Arg Thr Pro Val Val Leu
        195                 200                 205

Pro Gln Arg Ile Thr Pro Gln Leu Val Asp Met Leu Lys Lys Tyr His
    210                 215                 220

Pro Val Trp Leu Asn Thr His Phe Asn His Pro Asn Glu Val Thr Glu
225                 230                 235                 240

Glu Ala Val Glu Ala Cys Glu Arg Met Ala Asn Ala Gly Ile Pro Leu
                245                 250                 255

Gly Asn Gln Thr Val Leu Leu Arg Gly Ile Asn Asp Cys Thr His Val
            260                 265                 270

Met Lys Arg Leu Val His Leu Leu Val Lys Met Arg Val Arg Pro Tyr
        275                 280                 285

Tyr Ile Tyr Val Cys Asp Leu Ser Leu Gly Ile Gly His Phe Arg Thr
    290                 295                 300

Pro Val Ser Lys Gly Ile Glu Ile Ile Glu Asn Leu Arg Gly His Thr
305                 310                 315                 320

Ser Gly Tyr Ala Val Pro Thr Phe Val Val Gly Ala Pro Gly Gly Gly
                325                 330                 335

Gly Lys Ile Pro Val Thr Pro Asn Tyr Val Val Ser Gln Ser Pro Arg
            340                 345                 350

His Val Val Leu Arg Asn Tyr Glu Gly Val Ile Thr Thr Tyr Thr Glu
        355                 360                 365

Pro Glu Asn Tyr His Glu Glu Cys Asp Cys Glu Asp Cys Arg Ala Gly
    370                 375                 380

Lys His Lys Glu Gly Val Ala Ala Leu Ser Gly Gly Gln Gln Leu Ala

385                 390                 395                 400

Ile Glu Pro Ser Asp Leu Ala Arg Lys Lys Arg Lys Phe Asp Lys Asn
                405                 410                 415

<210>  50
<211>  1251
<212>  DNA
<213>  Porphyromonas gingivalis

<220>
<221>  CDS
<222>  (1)..(1251)

<400>  50
atg gca gaa agt cgt aga aag tat tat ttc cct gat gtc acc gat gag       48
Met Ala Glu Ser Arg Arg Lys Tyr Tyr Phe Pro Asp Val Thr Asp Glu
1               5                   10                  15

caa tgg tac gac tgg cat tgg cag gtc atc aat cga att aag acg ctc       96
Gln Trp Tyr Asp Trp His Trp Gln Val Ile Asn Arg Ile Lys Thr Leu
            20                  25                  30

gac cag ctg aaa aag tac gtt aca ctc acc gct gaa gaa gaa gag gga      144
Asp Gln Leu Lys Lys Tyr Val Thr Leu Thr Ala Glu Glu Glu Glu Gly
        35                  40                  45

gta aaa gaa tcg ccc aaa gta ctc cga atg gct atc aca cct tat tat      192
Val Lys Glu Ser Pro Lys Val Leu Arg Met Ala Ile Thr Pro Tyr Tyr
    50                  55                  60

ttg agt ttg ata gac ccc gag aat cct aat tgt ccg att cgt aaa caa      240
Leu Ser Leu Ile Asp Pro Glu Asn Pro Asn Cys Pro Ile Arg Lys Gln
65                  70                  75                  80

gcc att cct act caa cag gaa ctg gta cgt gct cct gaa gat cag gta      288
Ala Ile Pro Thr Gln Gln Glu Leu Val Arg Ala Pro Glu Asp Gln Val
                85                  90                  95

gac cca ctt agt gaa gat gaa gat tcg ccc gta ccc gga ctg act cat      336
Asp Pro Leu Ser Glu Asp Glu Asp Ser Pro Val Pro Gly Leu Thr His
            100                 105                 110

cgt tat ccg gat cgt gta ttg ttc ctt atc acg gac aaa tgt tcg atg      384
Arg Tyr Pro Asp Arg Val Leu Phe Leu Ile Thr Asp Lys Cys Ser Met
        115                 120                 125

tac tgt cgt cat tgt act cgc cgt cgc ttc gca gga cag aaa gat gct      432
Tyr Cys Arg His Cys Thr Arg Arg Arg Phe Ala Gly Gln Lys Asp Ala
    130                 135                 140

tct tct cct tct gag cgc atc gat cga tgc att gac tat ata gcc aat      480
Ser Ser Pro Ser Glu Arg Ile Asp Arg Cys Ile Asp Tyr Ile Ala Asn
145                 150                 155                 160

aca ccg aca gtc cgc gat gtt ttg cta tcg gga ggc gat gcc ctc ctt      528
Thr Pro Thr Val Arg Asp Val Leu Leu Ser Gly Gly Asp Ala Leu Leu
                165                 170                 175

gtc agc gac gaa cgc ttg gaa tac ata ttg aag cgt ctg cgc gaa gta      576
Val Ser Asp Glu Arg Leu Glu Tyr Ile Leu Lys Arg Leu Arg Glu Val
            180                 185                 190

cct cat gtg gag att gtt cgt ata gga agc cgt acg ccg gta gtc ctc      624
Pro His Val Glu Ile Val Arg Ile Gly Ser Arg Thr Pro Val Val Leu
        195                 200                 205

cct cag cgt ata acg cct caa ttg gtg gat atg ctc aaa aaa tat cat      672
Pro Gln Arg Ile Thr Pro Gln Leu Val Asp Met Leu Lys Lys Tyr His
    210                 215                 220

ccg gtg tgg ctg aac act cac ttc aac cac ccg aat gaa gtt acc gaa      720
Pro Val Trp Leu Asn Thr His Phe Asn His Pro Asn Glu Val Thr Glu
225                 230                 235                 240

gaa gca gtg gag gct tgt gaa aga atg gcc aat gcc ggt att ccg ttg      768
Glu Ala Val Glu Ala Cys Glu Arg Met Ala Asn Ala Gly Ile Pro Leu
                245                 250                 255

ggt aac caa acg gtt tta ttg cgt gga atc aat gat tgt aca cat gtg      816
Gly Asn Gln Thr Val Leu Leu Arg Gly Ile Asn Asp Cys Thr His Val
            260                 265                 270

atg aag aga ttg gta cat ttg ctg gta aag atg cgt gtg cgt cct tac      864
Met Lys Arg Leu Val His Leu Leu Val Lys Met Arg Val Arg Pro Tyr
        275                 280                 285

tat ata tat gta tgc gat ctt tcg ctt gga ata ggt cat ttc cgc acg      912
Tyr Ile Tyr Val Cys Asp Leu Ser Leu Gly Ile Gly His Phe Arg Thr
    290                 295                 300

ccg gta tct aaa gga atc gaa att atc gaa aat ttg cgc gga cac acc      960
Pro Val Ser Lys Gly Ile Glu Ile Ile Glu Asn Leu Arg Gly His Thr
305                 310                 315                 320

tcg ggc tat gca gtt cct acc ttt gtg gta ggt gct ccg ggg ggt ggt     1008
Ser Gly Tyr Ala Val Pro Thr Phe Val Val Gly Ala Pro Gly Gly Gly
                325                 330                 335

ggt aag ata cct gta acg ccg aac tat gtt gta tct cag tcc cca cga     1056
Gly Lys Ile Pro Val Thr Pro Asn Tyr Val Val Ser Gln Ser Pro Arg
            340                 345                 350

cat gtg gtt ctt cgc aat tat gaa ggt gtt atc aca acc tat acg gag     1104
His Val Val Leu Arg Asn Tyr Glu Gly Val Ile Thr Thr Tyr Thr Glu
        355                 360                 365

ccg gag aat tat cat gag gag tgc gat tgt gag gac tgt cga gcc ggt     1152
Pro Glu Asn Tyr His Glu Glu Cys Asp Cys Glu Asp Cys Arg Ala Gly
    370                 375                 380

aag cat aaa gag ggt gta gct gca ctt tcc gga ggt cag cag ttg gct     1200
Lys His Lys Glu Gly Val Ala Ala Leu Ser Gly Gly Gln Gln Leu Ala
385                 390                 395                 400

atc gag cct tcc gac tta gct cgc aaa aaa cgc aag ttt gat aag aac     1248
Ile Glu Pro Ser Asp Leu Ala Arg Lys Lys Arg Lys Phe Asp Lys Asn
                405                 410                 415

tga                                                                  1251

<210>  51
<211>  416
<212>  PRT
<213>  Porphyromonas gingivalis

<400>  51

Met Ala Glu Ser Arg Arg Lys Tyr Tyr Phe Pro Asp Val Thr Asp Glu
1               5                   10                  15

Gln Trp Tyr Asp Trp His Trp Gln Val Ile Asn Arg Ile Lys Thr Leu
            20                  25                  30

Asp Gln Leu Lys Lys Tyr Val Thr Leu Thr Ala Glu Glu Glu Glu Gly
        35                  40                  45

Val Lys Glu Ser Pro Lys Val Leu Arg Met Ala Ile Thr Pro Tyr Tyr
    50                  55                  60

Leu Ser Leu Ile Asp Pro Glu Asn Pro Asn Cys Pro Ile Arg Lys Gln
65                  70                  75                  80

Ala Ile Pro Thr Gln Gln Glu Leu Val Arg Ala Pro Glu Asp Gln Val
                85                  90                  95

Asp Pro Leu Ser Glu Asp Glu Asp Ser Pro Val Pro Gly Leu Thr His
            100                 105                 110

Arg Tyr Pro Asp Arg Val Leu Phe Leu Ile Thr Asp Lys Cys Ser Met
        115                 120                 125

Tyr Cys Arg His Cys Thr Arg Arg Arg Phe Ala Gly Gln Lys Asp Ala
    130                 135                 140

Ser Ser Pro Ser Glu Arg Ile Asp Arg Cys Ile Asp Tyr Ile Ala Asn
145                 150                 155                 160

Thr Pro Thr Val Arg Asp Val Leu Leu Ser Gly Gly Asp Ala Leu Leu
                165                 170                 175

Val Ser Asp Glu Arg Leu Glu Tyr Ile Leu Lys Arg Leu Arg Glu Val
            180                 185                 190

Pro His Val Glu Ile Val Arg Ile Gly Ser Arg Thr Pro Val Val Leu
        195                 200                 205

Pro Gln Arg Ile Thr Pro Gln Leu Val Asp Met Leu Lys Lys Tyr His
    210                 215                 220

Pro Val Trp Leu Asn Thr His Phe Asn His Pro Asn Glu Val Thr Glu
225                 230                 235                 240

Glu Ala Val Glu Ala Cys Glu Arg Met Ala Asn Ala Gly Ile Pro Leu
                245                 250                 255

Gly Asn Gln Thr Val Leu Leu Arg Gly Ile Asn Asp Cys Thr His Val
            260                 265                 270

Met Lys Arg Leu Val His Leu Leu Val Lys Met Arg Val Arg Pro Tyr
        275                 280                 285

Tyr Ile Tyr Val Cys Asp Leu Ser Leu Gly Ile Gly His Phe Arg Thr
    290                 295                 300

Pro Val Ser Lys Gly Ile Glu Ile Ile Glu Asn Leu Arg Gly His Thr
305                 310                 315                 320

Ser Gly Tyr Ala Val Pro Thr Phe Val Val Gly Ala Pro Gly Gly Gly
                325                 330                 335

Gly Lys Ile Pro Val Thr Pro Asn Tyr Val Val Ser Gln Ser Pro Arg
            340                 345                 350

His Val Val Leu Arg Asn Tyr Glu Gly Val Ile Thr Thr Tyr Thr Glu
        355                 360                 365

Pro Glu Asn Tyr His Glu Glu Cys Asp Cys Glu Asp Cys Arg Ala Gly
    370                 375                 380

Lys His Lys Glu Gly Val Ala Ala Leu Ser Gly Gly Gln Gln Leu Ala
385                 390                 395                 400

Ile Glu Pro Ser Asp Leu Ala Arg Lys Lys Arg Lys Phe Asp Lys Asn
                405                 410                 415

<210>  52
<211>  416
<212>  PRT
<213>  Porphyromonas gingivalis

<400>  52

Met Ala Glu Ser Arg Arg Lys Tyr Tyr Phe Pro Asp Val Thr Asp Glu

1               5                   10                  15

Gln Trp Asn Asp Trp His Trp Gln Val Leu Asn Arg Ile Glu Thr Leu
            20                  25                  30

Asp Gln Leu Lys Lys Tyr Val Thr Leu Thr Ala Glu Glu Glu Glu Gly
        35                  40                  45

Val Lys Glu Ser Leu Lys Val Leu Arg Met Ala Ile Thr Pro Tyr Tyr
    50                  55                  60

Leu Ser Leu Ile Asp Pro Glu Asn Pro Asn Cys Pro Ile Arg Lys Gln
65                  70                  75                  80

Ala Ile Pro Thr His Gln Glu Leu Val Arg Ala Pro Glu Asp Gln Val
                85                  90                  95

Asp Pro Leu Ser Glu Asp Glu Asp Ser Pro Val Pro Gly Leu Thr His
            100                 105                 110

Arg Tyr Pro Asp Arg Val Leu Phe Leu Ile Thr Asp Lys Cys Ser Met
        115                 120                 125

Tyr Cys Arg His Cys Thr Arg Arg Arg Phe Ala Gly Gln Lys Asp Ala
    130                 135                 140

Ser Ser Pro Ser Glu Arg Ile Asp Arg Cys Ile Asp Tyr Ile Ala Asn
145                 150                 155                 160

Thr Pro Thr Val Arg Asp Val Leu Leu Ser Gly Gly Asp Ala Leu Leu
                165                 170                 175

Val Ser Asp Glu Arg Leu Glu Tyr Ile Leu Lys Arg Leu Arg Glu Ile
            180                 185                 190

Pro His Val Glu Ile Val Arg Ile Gly Ser Arg Thr Pro Val Val Leu
        195                 200                 205

Pro Gln Arg Ile Thr Pro Gln Leu Val Asp Met Leu Lys Lys Tyr His
    210                 215                 220

Pro Val Trp Leu Asn Thr His Phe Asn His Pro Asn Glu Val Thr Glu
225                 230                 235                 240

Glu Ala Val Glu Ala Cys Glu Arg Met Ala Asn Ala Gly Ile Pro Leu
                245                 250                 255

Gly Asn Gln Thr Val Leu Leu Arg Gly Ile Asn Asp Cys Thr His Val
            260                 265                 270

Met Lys Arg Leu Val His Leu Leu Val Lys Met Arg Val Arg Pro Tyr
        275                 280                 285

Tyr Ile Tyr Val Cys Asp Leu Ser Leu Gly Ile Gly His Phe Arg Thr
    290                 295                 300

Pro Val Ser Lys Gly Ile Glu Ile Ile Glu Asn Leu Arg Gly His Thr
305                 310                 315                 320

Ser Gly Tyr Ala Val Pro Thr Phe Val Val Asp Ala Pro Gly Gly Gly
                325                 330                 335

Gly Lys Ile Pro Val Met Pro Asn Tyr Val Val Ser Gln Ser Pro Arg
            340                 345                 350

His Val Val Leu Arg Asn Tyr Glu Gly Val Ile Thr Thr Tyr Thr Glu
        355                 360                 365

Pro Glu Asn Tyr His Glu Glu Cys Asp Cys Glu Asp Cys Arg Ala Gly
    370                 375                 380

Lys His Lys Glu Gly Val Ala Ala Leu Ser Gly Gly Gln Gln Leu Ala
385                 390                 395                 400

Ile Glu Pro Ser Asp Leu Ala Arg Lys Lys Arg Lys Phe Asp Lys Asn
                405                 410                 415


<210>  53
<211>  60
<212>  DNA
<213>  Artificial

<220>
<223>  primer

<400>  53
tatcaattcg ttacaggcga tacatggcac gcttcggcgc gtgtaggctg gagctgcttc      60


<210>  54
<211>  60
<212>  DNA
<213>  Artificial

<220>
<223>  primer

<400>  54
gatgtcgcgg ctggtgagta accagccgca gggataacaa catatgaata tcctccttag     60


<210>  55
<211>  20
<212>  DNA
<213>  Artificial

<220>
<223>  primer

<400>  55
ttaccgagca gcgttcagag                                                  20


<210>  56
<211>  20
<212>  DNA
<213>  Artificial

<220>
<223>  primer

<400>  56
cacctggcgg tgacaaccat                                                  20


<210>  57
<211>  60
<212>  DNA
<213>  Artificial

<220>
<223>  primer

<400>  57
gcggcgtgaa gtttcccaac ccgttctgcc tctcttcttc gtgtaggctg gagctgcttc     60


<210>  58
<211>  60
<212>  DNA
<213>  Artificial

<220>
<223>  primer

<400>  58
ttacaacgtt accgggtgtt ctttctcgcc tttcttaaac catatgaata tcctccttag     60


<210>  59
<211>  471
<212>  PRT
<213>  Bacillus subtilis

<400>  59

Met Lys Asn Lys Trp Tyr Lys Pro Lys Arg His Trp Lys Glu Ile Glu

1               5                   10                  15

Leu Trp Lys Asp Val Pro Glu Glu Lys Trp Asn Asp Trp Leu Trp Gln
            20                  25                  30

Leu Thr His Thr Val Arg Thr Leu Asp Asp Leu Lys Lys Val Ile Asn
        35                  40                  45

Leu Thr Glu Asp Glu Glu Glu Gly Val Arg Ile Ser Thr Lys Thr Ile
    50                  55                  60

Pro Leu Asn Ile Thr Pro Tyr Tyr Ala Ser Leu Met Asp Pro Asp Asn
65                  70                  75                  80

Pro Arg Cys Pro Val Arg Met Gln Ser Val Pro Leu Ser Glu Glu Met
                85                  90                  95

His Lys Thr Lys Tyr Asp Leu Glu Asp Pro Leu His Glu Asp Glu Asp
            100                 105                 110

Ser Pro Val Pro Gly Leu Thr His Arg Tyr Pro Asp Arg Val Leu Phe
        115                 120                 125

Leu Val Thr Asn Gln Cys Ser Met Tyr Cys Arg Tyr Cys Thr Arg Arg
    130                 135                 140

Arg Phe Ser Gly Gln Ile Gly Met Gly Val Pro Lys Lys Gln Leu Asp
145                 150                 155                 160

Ala Ala Ile Ala Tyr Ile Arg Glu Thr Pro Glu Ile Arg Asp Cys Leu
                165                 170                 175

Ile Ser Gly Gly Asp Gly Leu Leu Ile Asn Asp Gln Ile Leu Glu Tyr
            180                 185                 190

Ile Leu Lys Glu Leu Arg Ser Ile Pro His Leu Glu Val Ile Arg Ile
        195                 200                 205

Gly Thr Arg Ala Pro Val Val Phe Pro Gln Arg Ile Thr Asp His Leu
    210                 215                 220

Cys Glu Ile Leu Lys Lys Tyr His Pro Val Trp Leu Asn Thr His Phe
225                 230                 235                 240

Asn Thr Ser Ile Glu Met Thr Glu Glu Ser Val Glu Ala Cys Glu Lys
                245                 250                 255

Leu Val Asn Ala Gly Val Pro Val Gly Asn Gln Ala Val Val Leu Ala
            260                 265                 270

Gly Ile Asn Asp Ser Val Pro Ile Met Lys Lys Leu Met His Asp Leu
        275                 280                 285

Val Lys Ile Arg Val Arg Pro Tyr Tyr Ile Tyr Gln Cys Asp Leu Ser
    290                 295                 300

Glu Gly Ile Gly His Phe Arg Ala Pro Val Ser Lys Gly Leu Glu Ile
305                 310                 315                 320

Ile Glu Gly Leu Arg Gly His Thr Ser Gly Tyr Ala Val Pro Thr Phe
                325                 330                 335

Val Val Asp Ala Pro Gly Gly Gly Gly Lys Ile Ala Leu Gln Pro Asn
            340                 345                 350

Tyr Val Leu Ser Gln Ser Pro Asp Lys Val Ile Leu Arg Asn Phe Glu
        355                 360                 365

Gly Val Ile Thr Ser Tyr Pro Glu Pro Glu Asn Tyr Ile Pro Asn Gln
    370                 375                 380

Ala Asp Ala Tyr Phe Glu Ser Val Phe Pro Glu Thr Ala Asp Lys Lys
385                 390                 395                 400

Glu Pro Ile Gly Leu Ser Ala Ile Phe Ala Asp Lys Glu Val Ser Phe
                405                 410                 415

Thr Pro Glu Asn Val Asp Arg Ile Lys Arg Arg Glu Ala Tyr Ile Ala
            420                 425                 430

Asn Pro Glu His Glu Thr Leu Lys Asp Arg Arg Glu Lys Arg Asp Gln
        435                 440                 445

Leu Lys Glu Lys Lys Phe Leu Ala Gln Gln Lys Lys Gln Lys Glu Thr
    450                 455                 460

Glu Cys Gly Gly Asp Ser Ser
465                 470

## Description

### FIELD

This disclosure relates to alanine 2,3-aminomutase nucleic acid and amino acid sequences, cells having alanine 2,3-aminomutase activity which can convert alpha-alanine to beta-alanine, and methods using these cells to make beta-alanine, pantothenic acid, 3-hydroxypropionic acid, and other organic compounds

### BACKGROUND

Organic chemicals such as organic acids, esters, and polyols can be used to synthesize plastic materials and other products. To meet the increasing demand for organic chemicals, more efficient and cost-effective production methods are being developed which utilize raw materials based on carbohydrates rather than hydrocarbons For example, certain bacteria have been used to produce large quantities of lactic acid used in the production of polylactic acid.

3-hydroxypropionic acid (3-HP) is an organic acid Several chemical synthesis routes have been described to produce 3-HP, and biocatalytic routes have also been disclosed (WO 01/16346 to Suthers et al) 3-HP has utility for specialty synthesis and can be converted to commercially important intermediates by known art in the chemical industry, such as acrylic acid by dehydration, malonic acid by oxidation, esters by esterification reactions with alcohols, and 1,3-propanediol by reduction

### SUMMARY

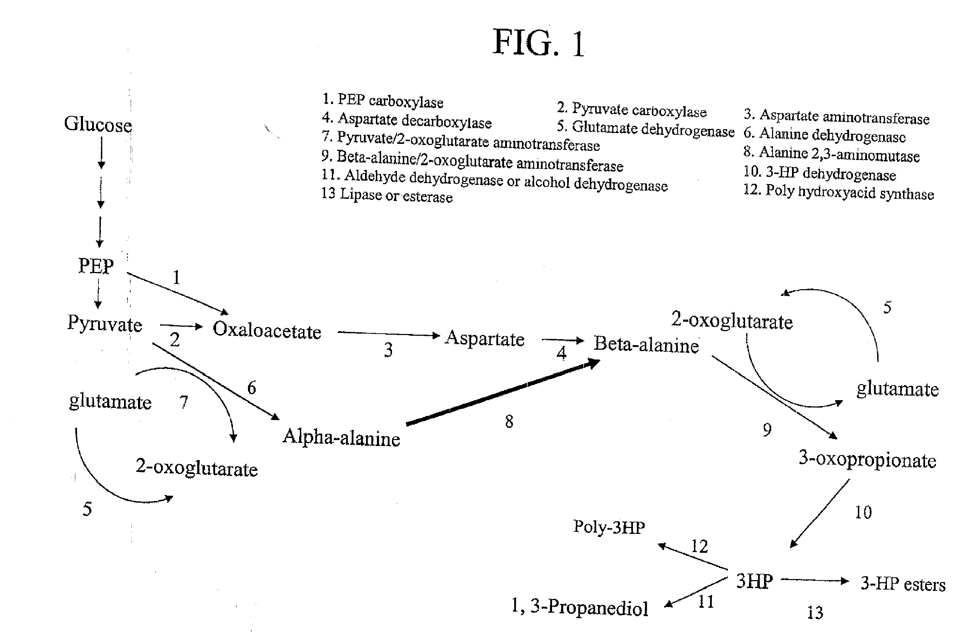

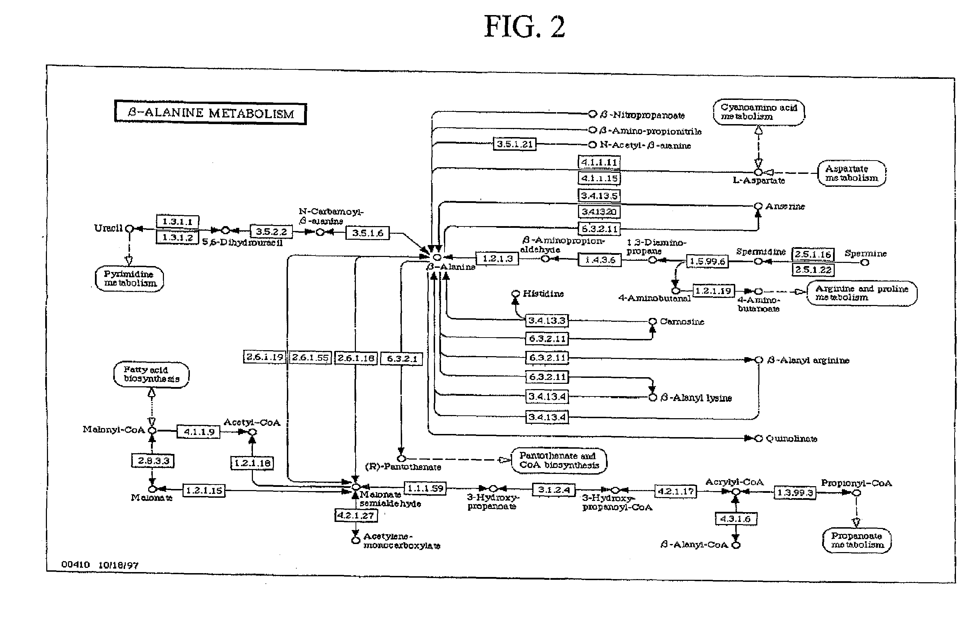

The compound 3-hydroxypropionic acid (3-HP) can be produced biocatalytically from PEP or pyruvate, through a key beta-alanine intermediate (FIG 1). Beta-alanine can be synthesized in cells from carnosine, beta-alanyl arginine, beta-alanyl lysine, uracil via 5,6-dihydrouracil and N-carbamoyl-beta-alanine, N-acetyl-beta-alanine, anserine, or aspartate (FIGS 1 and 2) However, these routes are relatively inefficient because they require rare precursors or starting compounds that are more valuable than 3-HP Therefore, production of 3-HP using biocatalytic routes would be more efficient if alpha-alanine could be converted to beta-alanine directly (FIG. 1).

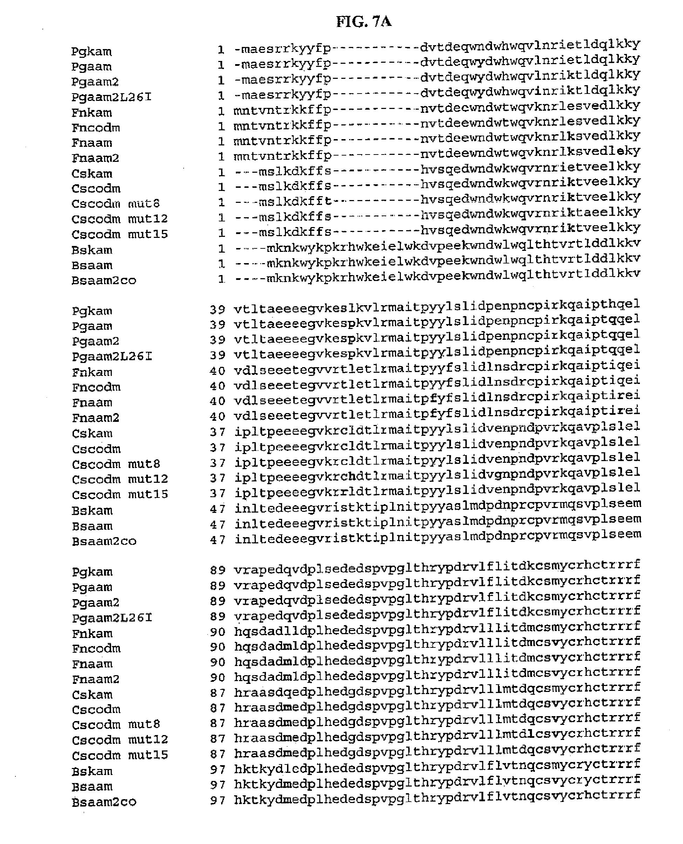

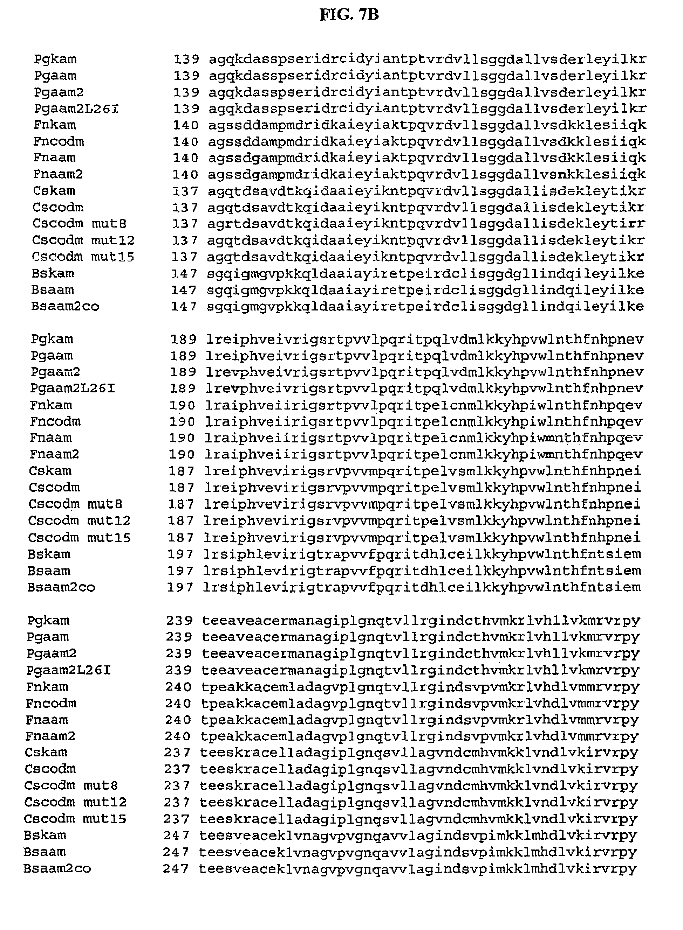

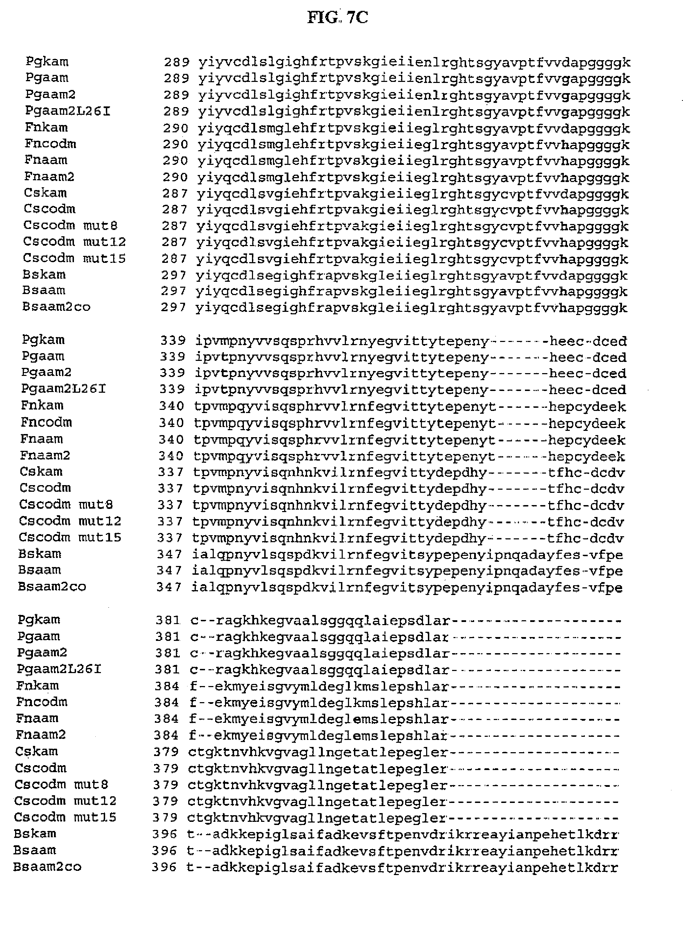

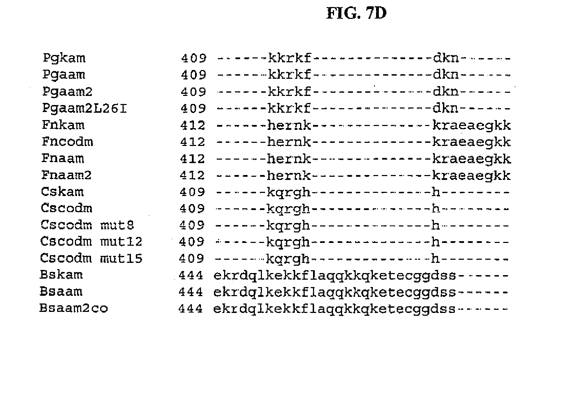

Disclosed herein are novel mutated lysine 2,3 aminomutase nucleic acid and protein sequences that have alanine 2,3-aminomutase biological activity. In one example, a mutated lysine 2,3 aminomutase includes one or more of the following substitutions: P/S11T; N19Y; L/K/R/T26I; E/R30K; L/V32A; K36E; S/I/C52R; L/T53P/H; Y63F; E/N/D71G; H/I/S85Q;Q/L/E86R; Q/L95M; K/M/Q125L, M128V; Y132H; Q/S141R; A/D/S/M144G; D179N; K/Q187R; I192V; L228M; D331G/H; M/Q342T; or K/Q/1398E, where the letter(s) before the number represents the one letter amino acid code for the amino acid found in a lysine 2,3 aminomutase, the number represents the amino acid position (based on the numbering for *Porphyromonas gingivalis* lysine 2,3 aminomutase (SEQ ID NO: 52), see FIG. 7), and the letter(s) after the number represents the one letter amino acid code for the amino acid found in the alanine 2,3 aminomutase. One skilled in the art will understand that the actual first amino acid and amino acid number may vary depending on the lysine 2,3 aminomutase sequence to be mutated, and understand that the position in the homologous sequence can be determined by aligning the sequences For example, as shown in FIG. 7, position 1 of *Porphyromonas gingivalis* lysine 2,3 aminomutase corresponds to position 12 *of Fusobacterium nucleatum* lysine 2,3 aminomutase (SEQ ID NO: 10), position 9 *of Clostridium sticklandii* lysine 2,3 aminomutase (SEQ ID NO: 33), and position 8 *of Bacillus subtilis* lysine 2,3 aminomutase (SEQ ID NO: 59). A similar analysis can be made using methods known in the art for each of the remaining positions based on the information provided in FIG. 7 and other publicly available lysine 2,3 aminomutase sequences (examples include, but are not limited to, GenBank Accession Nos YP_028406 for *Bacillus anthracis* str. Sterne; BAC95867 for *Vibrio vulnificus* YJ016; ZP_00330962 for *Moorella thermoacetica;* ZP_00322274 for *Haemophilus influenzae;* ZP_00298043 for *Methanosarcina barkeri* str Fusaro; ZP_0314982 for *Microbulbifer degradans* and NP_781545 for *Clostridium tetani* E88).

Any lysine 2,3 aminomutase can be mutagenized using standard molecular biology methods to generate an alanine 2,3-aminomutase For example, lysine 2,3 aminomutases from a prokaryote such as *Bacillus, Clostridium, Escherichia, Fusobacterium, Haemophilus, Methanosarcina, Microbulbifer, Moorella, Porphyromonas, Thermoanaerobacter* or *Vibrio* can be mutated to include one or more of the following substitutions, P/S11 T; N19Y; L/K/R/126I; E/R30K; L/V32A; K36E; S/T/C52R; L/T53P/H; Y63F; E/N/D71G; HII/S85Q; Q/L/E86R; Q/L95M; K/M/Q125L; M128V; Y132H; Q/S141R; A/A/S/M144G; D179N; K/Q187R; I192V; L228M; D331G/H; M/Q342I; or K/Q/I398E, such as at least 2, at least .3, at least 4, at least 5, at least 6, at least 8, at least 9, at least 10, at least 11, at least 12, at least 13, at least 14, at least 15, at least 16, at least 17, at least 18, at least 19, at least 20, at least 21, at least 22, at least 2.3, at least 24, or no more than 3, no more than 4, no more than 5, no more than 6, no more than 8, no more than 9, no more than 10, no more than 11, no more than 12, no more than 13, no more than 14, no more than 15, no more than 16, no more than 17, no more than 18, no more than 19, no more than 20, no more than 21, no more than 22, or no more than 23 of such substitutions Particular combinations of such substitutions include, but are not limited to: (1) N19Y, L/I53P/H, H/I/S85Q, D331G/H, and M/Q342I; (2) N19Y, E/R30K, L/I53P/H, H/I/S85Q, I192V, D331G/H, and M/Q342T; (3) N19Y, L/K/R/T26I; E/R30K, L/T53P/H, H/I/S8SQ, I192V, D331G/H, and M/Q342T; (4) E/R30K, Y63F, Q/L/E86R, Q/L95M, M128V, A/D/S/M144G, L228M, D331G/H, and K/Q/T398E; (5) E/R30K, K36E, Y63F, Q/L/E86R, Q/L95M, M128V, A/D/S/M144G, D179N, L228M, D331G/H, and K/Q/T398E; (6) E/R30K, Q/L95M, M128V, and D331G/H; (7) P/S11T, E/R30K, Q/L95M, M128V, Q/S141R, K/Q187R, and D331G/H; (8) E/R30K, L/V32A, L/T53P/H, E/N/D71G, Q/L95M; K/M/Q125L, M128V, and D331G/H; (9) E/R30K, C52R, Q/L95M; M128V, and D331G/H; (10) Q/L95M, M128V, and D331G/H; (11) Q/L95M, M128V; Y132H, and D331G/H; and (12) Q/L95M, M128V, and D331G/H.

Particular examples of alanine 2,3-aminomutase molecules include the nucleic acid sequences shown in SEQ ID NOS: 18, 20, 42, 44, 46, 48, and 50, and their corresponding amino acid sequences shown in SEQ ID NOS: 19, 21, 43, 45, 47, 49, and 51, as well as variants, fragments, fusions, and polymorphisms of these sequences that retain the ability to interconvert alpha-alanine to beta-alanine. The disclosed alanine 2,3-aminomutase sequences can be used to transform cells, such that the transformed cells have alanine 2,3-aminomutase activity, which allows the cells to produce beta-alanine from alpha-alanine Binding agents that specifically bind to an alanine 2,3-aminomutase are encompassed by this disclosure.

Cells having alanine 2,3-aminomutase activity, which allow the cell to convert alpha-alanine to beta-alanine, are disclosed. Such cells can be eukaryotic or prokaryotic cells, such as yeast cells, plant cells, *Lactobacillus, Lactococcus, Bacillus,* or *Escherichia* cells. In one example, the cell is transformed with a mutated lysine 2,3-aminomutase that confers to the transformed cells alanine 2,3-aminomutase activity In another example, cells are transformed with SEQ ID NO: 18, 20, 42, 44, 46, 48, or 50 (or fragments, fusions, or variants thereof that retain alanine 2,3-aminomutase activity. The disclosed cells can be used to produce nucleic acid molecules, peptides, and organic compounds. The peptides can be used to catalyze the formation of organic compounds or can be used as antigens to create specific binding agents.

A production cell having at least one exogenous nucleic acid, such as a nucleic acid encoding for an alanine 2,3-aminomutase, is disclosed In one example, the nucleic acid sequence includes SEQ ID NO: 8, 20, 42, 44, 46,48, or 50 (or fragments, variants, or fusions thereof that retain alanine 2,3-aminomutase activity): In another example, the nucleic acid sequence encodes an amino acid sequence shown in SEQ ID NO: 19, 21, 43, 45, 47, 49, or 51 (or fragments, variants or fusion proteins that of that retain alanine 2,3-aminomutase activity) Production cells can be used to express polypeptides that have an enzymatic activity such as pyruvate/2-oxoglutarate aminotransferase, beta-alanine/2-oxoglutarate aminotransferase, and 3-hydroxypropionate dehydrogenase capable of producing 3-hydroxypropionate from 3-oxopropionate Methods of producing polypeptides encoded by the nucleic acid sequences described above are disclosed

Methods of identifying a cell having alanine 2,3-aminomutase activity are disclosed In one example, the method includes culturing a cell functionally deleted for *panD* in media which does not include beta-alanine nor pantothenate For example, the cell can produce alpha-alanine from media sources of carbon, oxygen, hydrogen, and nitrogen, but does not include beta-alanine Cells capable of growing in the media are identified, wherein cell growth indicates that the cell is producing beta-alanine from alpha-alanine, which indicates the cell has alanine 2,3-aminomutase activity, In contrast, absence of cell growth indicates that the cell is not producing beta-alanine from alpha-alanine, which indicates the cell does not have alanine 2,3-aminomutase activity. In one example, prior to culturing the cell for selection, cells are transformed with one or more mutated lysine 2,3. aminomutases

A method of producing a peptide having alanine 2,3-aminomutase activity is disclosed In one example, the method includes culturing cells having at least one exogenous nucleic acid molecule that encodes an alanine 2,3-aminomutase (such as SEQ ID NO: 8, 20, 42, 44, 46, 48, or 50, or fragments, variants, or fusions thereof that retain alanine 2,3-aminomutase activity) which is capable of producing beta-alanine from alpha-alanine

A method of producing 3-HP from beta-alanine using the disclosed cells having alanine 2,3-aminomutase activity are disclosed In one example, the cell is transfected with one or more enzymes necessary to convert 3-HP from beta-alanine. In another example, the method includes purifying beta-alanine from the cell, then contacting the beta-alanine with polypeptides necessary to convert 3-HP from beta-alanine.

The cells, alanine 2,3-aminomutase nucleic and amino acid sequences (such as SEQ ID NO: 8, 20, 42, 44, 46, 48, or 50, or fragment, variants, or fusions thereof that retain alanine 2,3-aminomutase activity), and methods disclosed herein, can be used to produce pantothenate, 3-HP, and derivatives thereof such as coenzyme A (CoA), and other organic compounds such as 1,3-propanediol, acrylic acid, polymerized acrylate, esters of acrylate, polymerized 3-HP, co-polymers of' 3-HP and other compounds such as butyrates, valerates and other compounds, esters of 3-HP, and malonic acid and its esters. 3-HP is both biologically and commercially important For example, the nutritional industry can use 3-HP as a food, feed additive or preservative, while the derivatives mentioned above can be produced from 3-HP.

Nucleic acid molecules encoding for an alanine 2,3-aminomutase (such as SEQ ID NO: 8, 20, 42, 44, 46, 48, or 50, or fragments, variants, or fusions thereof that retain alanine 2,3-aminomutase activity) can be used to engineer host cells with the ability to produce 3-HP as well as other organic compounds such as those listed above Alanine 2,3-aminomutase peptides (such as SEQ ID NOS: 19, 21, 43, 45, 47, 49, and 51, as well as variants, fragments, or fusions of these sequences that retain the ability to interconvert alpha-alanine to beta-alanine) can be used in cell-free systems to make 3-HP as well as other organic compounds such as those listed above The cells described herein can be used in culture systems to produce large quantities of 3-HP as well as other organic compounds such as those listed above.

One aspect of the disclosure provides cells, which in addition to alanine 2,3-aminomutase activity, include other enzyme activities, such as pyruvate/2 -oxoglutarate aminotransferase activity, beta-alanine/2-oxoglutarate aminotransferase activity and 3-hydroxypropionate dehydrogenase activity Additionally, the cell can include poly hydroxyacid synthase activity, or lipase or esterase activity.

In another example, a cell including alanine 2,3-aminomutase activity; pyruvate/2-oxoglutarate aminotransferase activity, beta-alanine/2-oxoglutarate aminotransferase activity, and 3-hydroxypropionate dehydrogenase activity, produces a product, for example, 3 -HP or an ester of 3-IIP, such as methyl 3-hydroxypropionate, ethyl 3-hydroxypropionate, propyl 3-hydroxypropionate, or butyl 3-hydroxypropionate Accordingly, the disclosure also provides methods of producing one or more of these products, In some examples the method includes culturing the cell that includes alanine 2,3-aminomutase activity, pyruvate/2-oxoglutarate aminotransferase activity, beta-alanine/2-oxoglutarate aminotransferase activity, and 3-hydroxypropionate dehydrogenase activity under conditions that allow the product to be produced. These cells also can include lipase or esterase activity.

Another aspect of the disclosure provides cells, which in addition to alanine 2,3-aminomutase activity, have pyruvate/2-oxoglutarate aminotransferase activity, beta-alanine/2-oxoglutarate aminotransferase activity, 3-hydroxypropionate dehydrogenase activity, and poly hydroxyacid synthase activity. This cell can be used, for example, to produce products such as polymerized 3-HP and co-polymers of 3-HP and other compounds such as butyrates, valerates and other compounds

Cells which produce 1,3-propanediol and methods of their use are disclosed 1,3-propanediol can be generated from 3-HP via the use of polypeptides having enzymatic activity. When making 1,3-propanediol from 3-HP, a combination of a polypeptide having aldehyde dehydrogenase activity (such as an enzyme from the 1.21- class) and a polypeptide having alcohol dehydrogenase activity (such as an enzyme from the 1.1.1. - class) can be used, such as aldehyde dehydrogenase (NAD(P)+) (EC 1 2 1.-) and alcohol dehydrogenase (EC 1 1.1.1),

In some examples, products are produced *in vitro* (outside of a cell). In other examples, products are produced using a combination of *in vitro and in vivo* (within a cell) methods In yet other examples, products are produced *in vivo.* For methods involving *in vivo* steps, the cells can be isolated cultured cells or whole organisms such as transgenic plants, non-human mammals, or single-celled organisms such as yeast and bacteria (such as *Lactobacillus, Lactococcus, Bacillus,* and *Escherichia* cells) Hereinafter such cells are referred to as production cells Products produced by these production cells can be organic products such as beta-alanine, 3-HP, pantothenate, and derivatives thereof such as organic acids, polyols (such as 1,3-propanediol), coenzyme A (CoA), as well as an alanine 2,3-aminomutase described herein.

Pantothenate, a vitamin essential to many animals for growth and health, is involved in fatty acid synthesis and degradation. Deficiency of the vitamin results in generalized malaise clinically Therefore, pantothenate produced using the methods disclosed herein can be administered to a subject having a pantothenic deficiency, at a therapeutically effective dose. Cells that produce pantothenate, and methods of producing pantothenate from beta-alanine using the disclosed cells, are disclosed. Production cells used to produce pantothenate and/or CoA, can be used to express alpha-ketopantoate hydroxymethyltransferase (E.C.2.1.2.11), alpha-ketopantoate reductase (E.C. 1.1 1.169), and pantothenate synthase (E.C.6.3.2.1), to produce pantothenate, or in addition pantothenate kinase (E C. 2 7 1 33), 4'-phosphopantethenoyl-1 -cysteine synthetase (EC6325),4'-phosphopantothenoylcysteine decarboxylase (E.C. 4 1 1 36), ATP:4'-phosphopantetheine adenyltransferase (E.C. 2 7.7.3), and dephospho-CoA kinase (E.C 2 7.124), to produce coenzyme A.

### BRIEF DESCRIPTION OF THE FIGURES

**FIG. 1** is a diagram of a pathway for generating 3-HP and derivatives thereof via a beta. alanine intermediate, and for making beta-alanine from alpha-alanine,
**FIG. 2** is a diagram of known metabolic routes by which beta-alanine is produced and consumed.

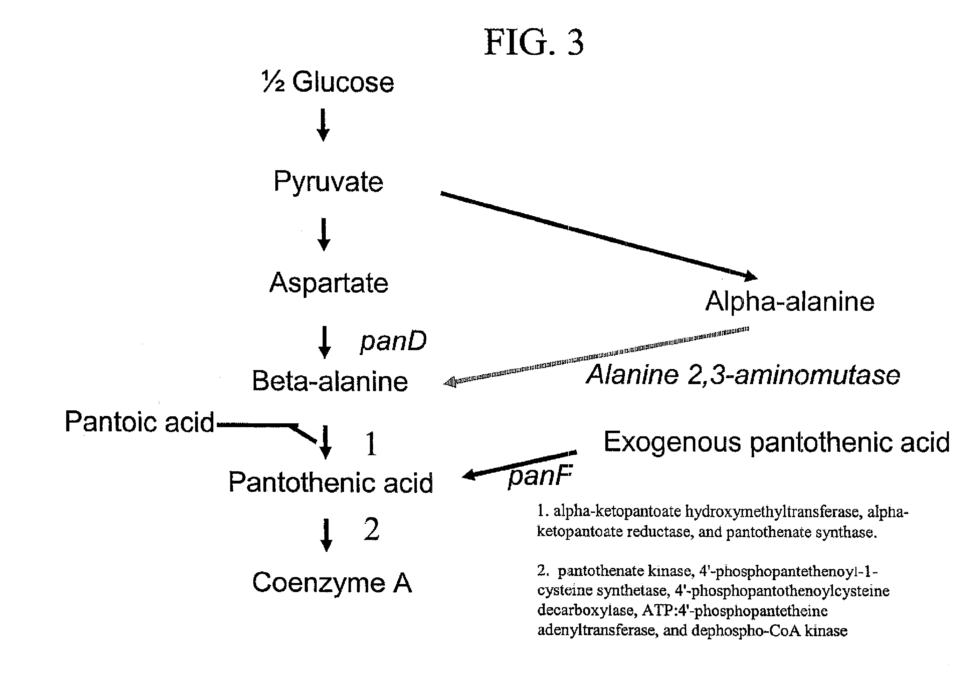

**FIG. 3** is a diagram showing alternative pathways for generating coenzyme A and pantothenate from beta-alanine via aspartate decarboxylase (panD) or via alanine 2,3-aminomutase

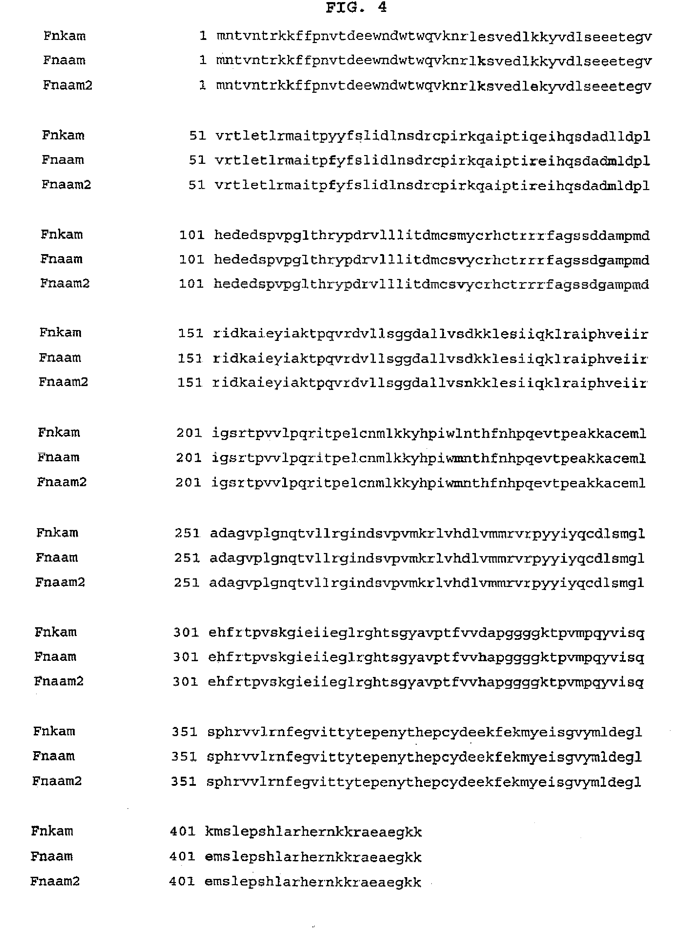

**FIG. 4** is an alignment showing a lysine 2,3-aminomutase protein sequence from *F*. *nucleatum* (Fnkam; SEQ ID NO: 10) and two variant sequences (Fnaam, SEQ ID NO: 19; and Fnaam2, SEQ ID NO: 21) that result in a protein having alanine 2,3 aminomutase activity. Substitutions in the lysine 2,3-aminomutase protein sequence are noted in bold.

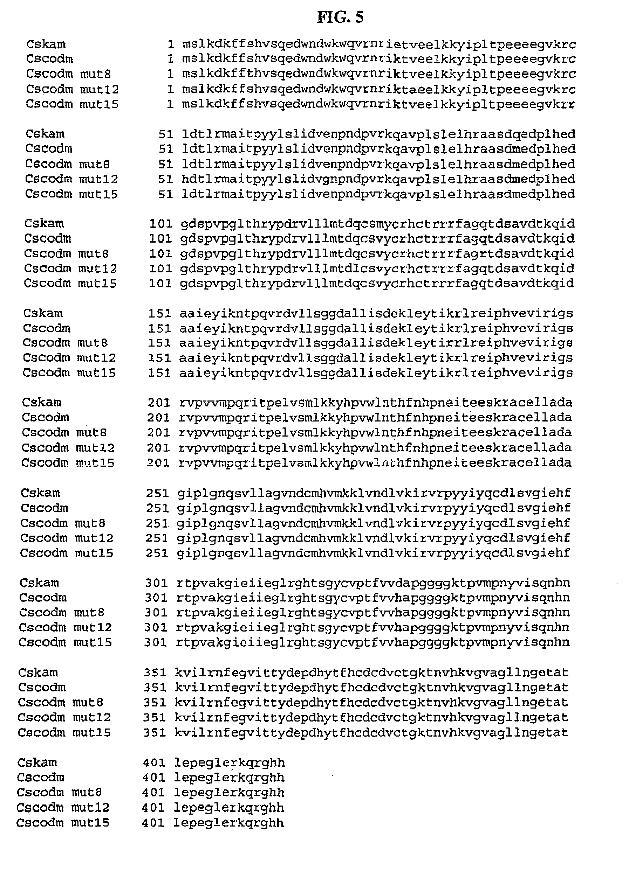

**FIG. 5** is an alignment showing a lysine 2,3-aminomutase protein sequence from C. *sticklandii* (Cskam; SEQ ID NO: 33), a lysine 2,3-aminomutase protein sequence from *C sticklandii* having E28K, I.93M, M126V, and D329H substitutions (Cscodm; SEQ ID NO: 41), and three variant sequences (Cscodm mut8, SEQ ID NO: 43; Cscodm mut12, SEQ ID NO: 45; and Cscodm mut15, SEQ ID NO: 47) that result in a protein having alanine 2,3 aminomutase activity. Substitutions in the lysine 2,3-aminomutase protein sequence are noted in bold

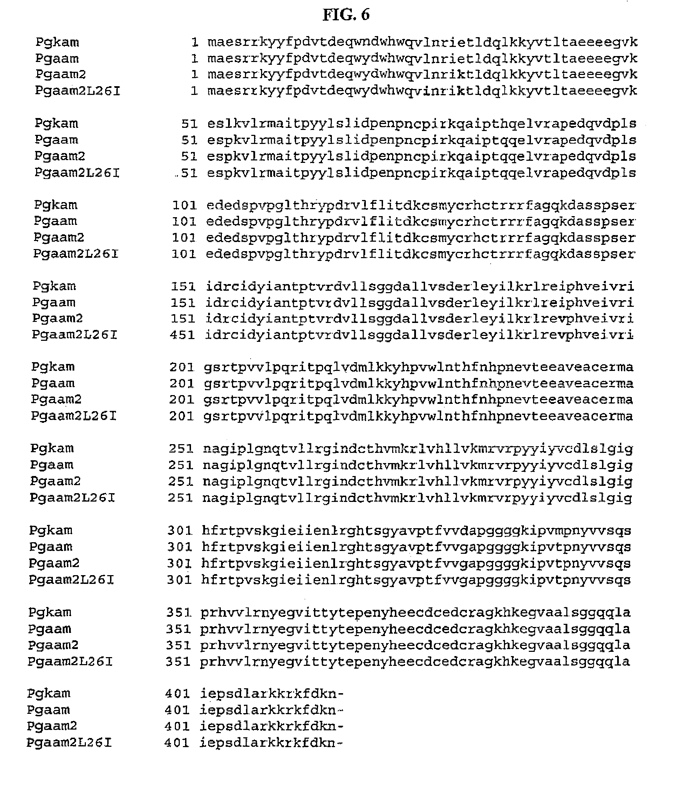

**FIG. 6** is an alignment showing a lysine 2,3-aminomutase protein sequence from *P gingivalis* (Pgkam; SEQ ID NO: 52), and three variant sequences (Pgaam SEQ ID NO: 6; Pgaam2, SEQ ID NO: 49; and Pgaam2 L26I, SEQ ID NO: 51) that result in a protein having alanine 2,3 aminomutase activity Substitutions in the lysine 2,3-aminomutase protein sequence are noted in bold.
**FIGS. 7A-D** is an alignment of lysine 2,3-aminomutases (from *P. gingivalis* (Pgkam, SEQ ID NO: 52); *F nucleatum* (Fnkam; SEQ ID NO: 10); C. *sticklandii* (Cskam; SEQ ID NO: 33) and *B. subtilis* (Bskam; SEQ ID NO: 59)) and alanine 2,3-aminomutases (from *P. gingivalis* (Pgaam SEQ ID NO: 6; Pgaam2, SEQ ID NO: 49; and Pgaam2 L26I, SEQ ID NO: 51); *F nucleatum* (Fnaam, SEQ ID NO: 19; and Fnaam2, SEQ ID NO: 21); *C sticklandii* (Cscodm mut8, SEQ ID NO: 43; Cscodm mut12, SEQ ID NO: 45; and Cscodm mut15, SEQ ID NO: 47) *and B subtilis* (Bsaam, SEQ ID NO: 2 and Bsaam2co, SEQ ID NO: 4)) as well as intermediate sequences *from F nucleatum* (Fncodm, SEQ ID NO: 14) and *C sticklandii* (Cscodm, SEQ ID NO: 41) Residues in bold denote mutations in variants of lysine 2,3-aminomutase that possess alanine 2,3-aminomutase activity

### SEQUENCE LISTING

The nucleic and amino acid sequences listed in the accompanying sequence listing are shown using standard letter abbreviations for nucleotide bases, and three letter code for amino acids, Only one strand of each nucleic acid sequence is shown, but the complementary strand is understood as included by any reference to the displayed strand.
SEQ ID NO: 1 is an alanine 2,3 aminomutase nucleic acid sequence *from B subtilis.*
SEQ ID NO: 2 is the protein sequence encoded by SEQ ID NO: 1.
SEQ ID NO: 3 is an alanine 2,3 aminomutase nucleic acid sequence from B *subtilis.*
SEQ ID NO: 4 is the protein sequence encoded by SEQ ID NO: 3
SEQ ID NO: 5 is an alanine 2,3 aminomutase nucleic acid sequence from *P. gingivalis.*
SEQ ID NO: 6 is the protein sequence encoded by SEQ ID NO: 5
SEQ ID NOS: 7 and 8 are nucleic acid PCR primers used to clone an *F*. *nucleatum* lysine 2,3-aminomutase
SEQ ID NO: 9 is a lysine 2,3-aminomutase nucleic acid sequence from *F*. *nucleatum,*
SEQ ID NO: 10 is the protein sequence encoded by SEQ ID NO: 9.
SEQ ID NO: 11 is a partially codon-optimized lysine 2,3-aminomutase nucleic acid sequence from *F nucleatum.*
SEQ ID NO: 12 is the protein sequence encoded by SEQ ID NO: 11
SEQ ID NO: 13 is a mutated partially codon-optimized lysine 2,3-aminomutase nucleic acid sequence from *F*. *nucleatum.*
SEQ ID NO: 14 is the protein sequence encoded by SEQ ID NO: 13.
SEQ ID NOS: 15-17 are nucleic acid primers used to mutate a codon-optimized lysine 2,3-aminomutase nucleic acid sequence *from F nucleatum.*
SEQ ID NO: 18 is an alanine 2,3 aminomutase nucleic acid sequence from *F. nucleatum*
SEQ ID NO: 13 is the protein sequence encoded by SEQ ID NO: 18.
SEQ ID NO: 20 is an alanine 2,3 aminomutase nucleic acid sequence *from F. nucleatum.*
SEQ ID NO: 21 is the protein sequence encoded by SEQ ID NO: 20.
SEQ ID NOS: 22-27 are nucleic acid PCR primers used to clone a lysine 2,3-aminomutase nucleic acid sequence from *C sticklandii.*
SEQ ID NOS: 28-31 are nucleic acid primers used for genome walking to clone a lysine 2,3-aminomutase nucleic acid sequence from *C sticklandii,*
SEQ ID NO: 32 is a lysine 2,3-aminomutase nucleic acid sequence from *C sticklandii*
SEQ ID NO: 33 is the protein sequence encoded by SEQ ID NO: 32
SEQ ID NO: 34 is a partially codon-optimized lysine 2,3-aminomutase nucleic acid sequence from *C sticklandii*
SEQ ID NO: 35 is the protein sequence encoded by SEQ ID NO: 34
SEQ ID NOS: 36-39 are nucleic acid primers used to mutate a partially codon-optimized lysine 2,3-aminomutase nucleic acid sequence from *C sticklandii.*
SEQ ID NO: 40 is a mutated codon-optimized lysine 2,3-aminomutase nucleic acid sequence from *C sticklandii*
SEQ ID NO: 41 is the protein sequence encoded by SEQ ID NO: 40.
SEQ ID NO: 42 is an alanine 2,3 aminomutase nucleic acid sequence from *C sticklandii,*
SEQ ID NO: 43 is the protein sequence encoded by SEQ ID NO: 42
SEQ ID NO: 44 is an alanine 2,3 aminomutase nucleic acid sequence from *C sticklandii*
SEQ ID NO: 45 is the protein sequence encoded by SEQ ID NO: 44
SEQ ID NO: 46 is an alanine 2,3 aminomutase nucleic acid sequence from *C sticklandii.*
SEQ ID NO: 47 is the protein sequence encoded by SEQ ID NO: 46.
SEQ ID NO: 48 is an alanine 2,3 aminomutase nucleic acid sequence from *P gingivalis.*
SEQ ID NO: 49 is the protein sequence encoded by SEQ ID NO: 48.
SEQ ID NO: 50 is an alanine 2,3 aminomutase nucleic acid sequence from *P gingivalis*
SEQ ID NO: 51 is the protein sequence encoded by SEQ ID NO: 50
SEQ ID NO: 52 is a lysine 2,3-aminomutase protein sequence *from P gingivalis*
SEQ ID NOS: 53 and 54 are PCR primers used to amplify a CAT gene of pKD3
SEQ ID NOS: 55 and 56 are PCR primers used to confirm correct insertion of the CAT gene into the *panD* locus
SEQ ID NDS: 57 and 58 are nucleic acid sequences of primers used to amplify the *CAT* gene of pKD3
SEQ ID NO: 59 is a lysine 2,3-aminomutase protein sequence from *B. subtilis*

### DETAILED DESCRIPTION OF SEVERAL EMBODIMENTS

The following explanations of terms and methods are provided to better describe the present disclosure and to guide those of ordinary skill in the art in the practice of the present disclosure The singular forms "a," "an," and "the" refer to one or more than one, unless the context clearly dictates otherwise.. For example, the term "comprising a nucleic acid molecule" includes single or plural nucleic acid molecules and is considered equivalent to the phrase "comprising at least one nucleic acid molecule." The term "or" refers to a single element of stated alternative elements or a combination of two or more elements, unless the context clearly indicates otherwise. As used herein. "comprises" means "includes." Ihus, "comprising A or B," means "including A, B, or A and B," without excluding additional elements.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below, The materials, methods, and examples are illustrative only and not intended to be limiting. Other features and advantages of the disclosure are apparent from the following detailed description and the claims.

**Alanine 2,3-aminomutase:** An enzyme which can convert alpha-alanine to beta-alanine and vice versa, for example in a cell. Includes any alanine 2,3-aminomutase gene, cDNA, RNA, or protein from any organism, such as a prokaryote In one example, an alanine 2,3-aminomutase is a mutated lysine 2,3-aminomutase which has alanine 2,3-aminomutase activity Lysine 2,3-aminomutases (or genes annotated in genetic databases as lysine 2,3 aminomutase) can be obtained from any organism, such as a prokaryote, for example *Bacillus subtilis, Porphyromonas gingivalis, Fusobacterium nucleatum, Clostridium sticklandii, or Escherichia coli,* and mutated using any method known in the art.

In particular examples, an alanine 2,3-aminomutase nucleic acid sequence includes a sequence shown in SEQ ID NO: 1, 3, 5, 18, 20, 42, 44, 46, 8, or 50, as well as fragments, variants, or fusions thereof that retain the ability to encode a peptide or protein having alanine 2,3-aminomutase activity In another example, an alanine 2,3-aminomutase protein includes an amino acid sequence shown in SEQ ID NO: 2, 4, 6, 19, 21, 43, 45, 47, 49, or 51, as well as fragments, fusions, or variants thereof that retain alanine 2,3-aminomutase activity

In another example, an alanine 2,3-aminomutase sequence includes a full-length wild-type sequence, such as SEQ ID NO: 2, 4, 6, 19, 21, 43, 45, 47, 49, or 51, as well as shorter sequences which retain the ability to convert alpha-alanine to beta-alanine, such as at least 9 contiguous amino acids (for example at least 10, at least 11, at least 12, at least 13, at least 14, at least 15, at least 20, at least 25, at least 30, at least 35, at least 40, at least 45, at least 50, at least 55, at least 60, at least 70, at least 80, at least 90, at least 100, at least 150, at least 200, at least 250, at least 300, at least 350, at least 400, at least 410, or at least 450 contiguous amino acids of SEQ ID NO: 2, 4, 6, 19, 21, 43, 45, 4 7, 49, or 51 Particular fragments of an alanine 2,3-aminomutase sequence include, but are not limited to, amino acids 50-390 of SEQ ID NO: 2 or 4, amino acids 101-339 of SEQ ID NO: 2 or 4, amino acids 15-390 of SEQ ID NO: 2 or 4, and amino acids 15-340 of SEQ ID NO: 2 or 4 (the corresponding fragments in SEQ ID NOS: 6, 19, 21, 43, 45, 47, 49, and 51 (such as amino acids 42-342 of SEQ ID NO: 6, 49 or 51) are encompassed by this disclosure, and can be determined by using FIG. 7) Such fragments (or full-length peptides) can be fused to other peptide sequences, as long as the resulting peptide has alanine 2,3-aminomutase activity. This description includes alanine 2,3-aminomutase allelic variants, as well as any variant, fragment, or fusion sequence which retains the ability to convert alpha-alanine to beta-alanine.

Alanine 2,3-aminomutase activity: The ability of an alanine 2,3-aminomutase to convert alpha-alanine to beta-alanine and vice versa In one example, such activity occurs in a cell In another example, such activity occurs *in vitro.* Such activity can be measured using any assay known in the art, for example the screening assays and enzyme assays described in the Examples below. In addition, an enzyme with alanine 2,3-aminomutase activity can be identified by incubating the enzyme with either alpha-alanine or beta-alanine and determining the reaction products by high-performance liquid chromatography (for example using the method of, Abe et al. J Chromatography B, 712:43-9, 1998). In one example, it is the ability of'an alanine 2,3-aminomutase to convert alpha-alanine to beta-alanine in an *E. coli* mutant functionally deleted for the *panD* gene.

In one example, a mutated lysine 2,3 aminomutase has at least 10%, at least 20%, at least 50%, at least 60%, at least 70%, at least 80%, at least 90%, or even at least 100% of the alanine 2,3 aminomutase activity as the peptide sequence shown in SEQ ID NO: 51.

Antibody: A molecule including an antigen binding site which specifically binds (immunoreacts with) an antigen. Examples include polyclonal antibodies, monoclonal antibodies, humanized monoclonal antibodies, or immunologically effective portions thereof.

Includes immunoglobulin molecules and immunologically active portions thereof: Naturally occurring antibodies (such as IgG) include four polypeptide chains, two heavy (H) chains and two light (L) chains inter-connected by disulfide bonds However, the antigen-binding function of an antibody can be performed by fragments of a naturally occurring antibody Immunologically effective portions of monoclonal antibodies include, but are not limited to: Fab, Fab', F(ab')₂, Fabc and Fv portions (for a review, see Better and Horowitz, Methods. Enzymol 1989, 178:476-96). Other examples of antigen-binding fragments include, but are not limited to: (i) an Fab fragment consisting of the VL, VH, CL and CHI domains; (ii) an Fd fragment consisting of the VH and CH1 domains; (iii) an Fv fragment consisting of the VL and VH domains of a single arm of an antibody, (iv) a dAb fragment which consists of a VH domain; (v) an isolated complimentarity determining region (CDR); and (vi) an F(ab')₂ fragment, a bivalent fragment comprising two Fab fragments linked by a disulfide bridge at the hinge region Furthermore, although the two domains of the Fv fragment are coded for by separate genes, a synthetic linker can be made that enables them to be made as a single protein chain (known as single chain Fv (scFv) by recombinant methods. Such single chain antibodies are also included.

"Specifically binds" refers to the ability of particular agent (a "specific binding agent") to specifically react with a particular analyte, for example to specifically immunoreact with an antibody, or to specifically bind to a particular peptide sequence. The binding is a non-random binding reaction, for example between an antibody molecule and an antigenic determinant. Binding specificity of an antibody is typically determined from the reference point of the ability of the antibody to differentially bind the specific antigen and an unrelated antigen, and therefore distinguish between two different antigens, particularly where the two antigens have unique epitopes An antibody that specifically binds to a particular epitope is referred to as a "specific antibody",

Monoclonal or polyclonal antibodies that can be produced to an alanine 2,3-aminomutase polypeptide (such as SEQ ID NO: 19, 21, 43, 45, 47, 49, or 51), fragments of an alanine 2,3-aminomutase polypeptide (such as amino acids 50-390 of SEQ ID NO: 2 or 4, for example amino acids 101-339 of SEQ ID NO: 2 or 4, or amino acids 15-390 of SEQ ID NO: 2 or 4, for example amino acids 15-331 of SEQ ID NO: 2 or 4; or the corresponding fragments in SEQ ID NOS: 6, 19, 21, 43, 45, 47, 49, and 51 (such as amino acids 42-342 of SEQ ID NO: 6, 49 or 51) that can be determined by using FIG. 7), or variants, or fusions thereof are encompassed by this disclosure. Optimally, antibodies raised against one or more epitopes on a polypeptide antigen will specifically detect that polypeptide. That is, antibodies raised against one particular polypeptide would recognize and bind that particular polypeptide, and would not substantially recognize or bind to other polypeptides The determination that an antibody specifically binds to a particular polypeptide is made by any one of number of standard immunoassay methods; for instance, Western blotting (for example see Sambrook et al (ed), Molecular Cloning: A Laboratory Manual, 2nd ed., vol. 1-3, Cold Spring Harbor Laboratory Press, Cold Spring Harbor, N.Y, 1989)

To determine that an antibody preparation (such as a preparation produced in a mouse against an alanine 2,3-aminomutase polypeptide, for example SEQ ID NO: 19*,* 21, 43, 45, 47, 49, or 51) specifically detects the appropriate polypeptide (such as an alanine 2,3.-aminomutase polypeptide) by Western blotting, total cellular protein can be extracted from cells and separated by SDS-polyacrylamide gel electrophoresis The separated total cellular protein can then be transferred to a membrane (such as nitrocellulose), and the antibody preparation incubated with the membrane After washing the membrane to remove non-specifically bound antibodies, the presence of specifically bound antibodies can be detected using an appropriate secondary antibody (such as an anti-mouse antibody) conjugated to an enzyme such as alkaline phosphatase since application of 5-bromo-4-chloro-3-indolyl phosphate/nitro blue tetrazolium results in the production of a densely blue-colored compound by immuno-localized alkaline phosphatase

Substantially pure polypeptides suitable for use as an immunogen can be obtained from transected cells, transformed cells, or wild-type cells. Polypeptide concentrations in the final preparation can be adjusted, for example, by concentration on an Amicon filter device, to the level of a few micrograms per milliliter In addition, polypeptides ranging in size from full-length polypeptides to polypeptides having as few as nine amino acid residues can be utilized as immunogens Such polypeptides can be produced in cell culture, can be chemically synthesized using standard methods, or can be obtained by cleaving large polypeptides into smaller polypeptides that can be purified Polypeptides having as few as nine amino acid residues in length can be immunogenic when presented to an immune system in the context of a Major Histocompatibility Complex (MHC) molecule such as an MHC class I or MHC class II molecule Accordingly, peptides having at least 9, 10, 11, 12, 13, 14, 15, 20, 25, 30, 35, 40, 45, 50, 55, 60,'70, 80, 90, 100, 150, 200, 250, 300, 350, 400, 410, 450, 500, or more consecutive amino acid residues of an alanine 2,3-aminomutase polypeptide can be used as immunogens for producing antibodies.

Monoclonal antibodies to any of the polypeptides disclosed herein can be prepared from murine hybridomas according to the classic method of Kohler & Milstein (Nature 256:495, 1975) or a derivative method thereof.

Polyclonal antiserum containing antibodies to the heterogeneous epitopes of any polypeptide disclosed herein can be prepared by immunizing suitable animals with the polypeptide (or fragment, fusion, or variant thereof), which can be unmodified or modified to enhance immunogenicity An effective immunization protocol for rabbits can be found in Vaitukaitis et al. (J Clin. Endocrinol Metab. 33:988-91,1971)

Antibody fragments can be used in place of whole antibodies and can be readily expressed in prokaryotic host cells Methods of making and using immunologically effective portions of monoclonal antibodies, also referred to as "antibody fragments," are well known and include those described in Better & Horowitz (Methods Enzymol, 178:476-96, 1989), Glockshuber et al. (Biochemistry 29:1362-,7,1990), U S Patent No. 5,648,231 ("Expression of Functional Antibody Fragments"), U.S. Patent No. 4,946,778 ("Single Polypeptide Chain Binding Molecules"), U.S. Patent No 5,455,030 ("Immunotherapy Using Single Chain Polypeptide Binding Molecules"), and references cited therein

Antigen: A compound, composition, or substance that can stimulate the production of antibodies or a I-cell response in an animal, including compositions that are administered, such as injected or absorbed, to an animal An antigen reacts with the products of Specific humoral or cellular immunity, including those induced by heterologous immunogens. The term "antigen" includes all related antigenic epitopes

**cDNA** (complementary **DNA):** A piece of DNA lacking internal, non-coding segments (introns) and regulatory sequences which determine transcription. cDNA can be synthesized in the laboratory by reverse transcription from messenger RNA extracted from cells.

**Conservative substitution:** One or more amino acid substitutions (for example 1, 2, 5 or 10 residues) for amino acid residues having similar biochemical properties Typically, conservative substitutions have little to no impact on the activity of a resulting polypeptide For example, a conservative substitution is an amino acid substitution in an alanine 2,3-aminomutase peptide that does not substantially affect the ability of the peptide to convert alpha-alanine to beta-alanine.

In a particular example, a conservative substitution is an amino acid substitution in an alanine 2,3-amuiomutase peptide, such as a conservative substitution in SEQ ID NO: 19, 21, 43, 45, 47, 49, or 51, that does not significantly alter the ability of the protein to convert alpha-alanine to beta-alanine Methods that can be used to determine alanine 2,3-aminomutase activity are disclosed in the Examples below An alanine scan can be used to identify which amino acid residues in an alanine 2,3-aminomutase peptide can tolerate an amino acid substitution In one example, alanine 2,3-aminomutase activity is not reduced by more than 25%, for example not more than 20%, for example not more than 10%, when an alanine, conservative or amino acid (such as those listed below), or a non-conservative amino acid, is substituted for one or more native amino acids

In one example, one conservative substitution is included in the peptide, such as a conservative substitution in any of SEQ ID NOS: 2, 4, 6, 19, 21, 43, 45, 47, 49, or 51. In another example, 10 or less conservative substitutions are included in the peptide, such as five or less. A polypeptide can be produced to contain one or more conservative substitutions by manipulating the nucleotide sequence that encodes that polypeptide using, for example, standard procedures such as site-directed mutagenesis or PCR. Alternatively, a polypeptide can be produced to contain one or more conservative substitutions by using standard peptide synthesis methods

Substitutional variants are those in which at least one residue in the amino acid sequence has been removed and a different residue inserted in its place Examples of amino acids which may be substituted for an original amino acid in a protein and which are regarded as conservative substitutions include: Ser for Ala; Lys for Arg; Gln or His for Asn; Glu for Asp; Ser for Cys; Asn for Gln; Asp for Glu; Pro for Gly; Asn or Gln for His; Leu or Val for Ile; Ile or Val for Leu; Arg or Gln for Lys; Leu or Ile for Met; Met, Leu or Iyr for Phe; Thr for Ser; Ser for Thr; Tyr for Irp; Trp or Phe for Tyr; and Ile or Leu for Val.

Further information about conservative substitutions can be found in, among other locations in, Ben-Bassat et al , (J Bacteriol 169:751-7, 198'7), O'Regan et al., (Gene 77:237-51, 1989), Sahin-Toth et al, (Protein Sci 3:240-7,1994), Hochuli et al., (Bio/Technology 6:1321-5, 1988), WO 00/67796 (Curd et al.) and in standard textbooks of genetics and molecular biology.

Deletion: The removal of a sequence of nucleic acid, for example DNA, the regions on either side being joined together.

**Detectable:** Capable or having an existence or presence ascertained. For example, production of beta-alanine from alpha-alanine is detectable if the signal generated from the beta-alanine is strong enough to be measurable

**DNA:** Deoxyribonucleic acid DNA is a long chain polymer which comprises the genetic 5 material of most living organisms (some viruses have genes comprising ribonucleic acid, RNA). The repeating units in DNA polymers are four different nucleotides, each of which comprises one of the four bases, adenine, guanine, cytosine and thymine bound to a deoxyribose sugar to which a phosphate group is attached. Triplets of nucleotides, referred to as codons, in DNA molecules code for amino acid in a polypeptide The term codon is also used for the corresponding (and complementary) sequences of three nucleotides in the mRNA into which the DNA sequence is transcribed

**Exogenous:** The term "exogenous" as used herein with reference to nucleic acid molecule and a particular cell refers to any nucleic acid molecule that does not originate from that particular cell as found in nature. Thus, a non-naturally-occurring nucleic acid molecule is considered to be exogenous to a cell once introduced into the cell. A nucleic acid molecule that is naturally-occurring also can be exogenous to a particular cell For example, an entire chromosome isolated from a cell of person X is an exogenous nucleic acid molecule with respect to a cell of person Y once that chromosome is introduced into Y's cell.

**Functional deletion.** A mutation, partial or complete deletion, insertion, or other variation made to a gene sequence which reduces or inhibits production of the gene product, or renders the gene product non-functional. For example, functional deletion of *panD* in *E coli* prevents the production of β-alanine from aspartate by aspartate decarboxylase, which is encoded by the *panD* gene. This functional deletion of *panD* in *E. coli* inactivates aspartate decarboxylase which results in growth inhibition of the *E. coli* in the absence of beta-alanine or pantothenate in the growth medium.

**Functionally Equivalent:** Having an equivalent function In the context of a alanine 2,3-aminomutase molecule, functionally equivalent molecules include different molecules that retain the function of alanine 2,3-aminomutase. For example, functional equivalents can be provided by sequence alterations in an alanine 2,3-aminomutase, wherein the peptide with one or more sequence alterations retains a function of the unaltered peptide, such that it retains its ability to convert alpha-alanine to beta-alanine,

Examples of sequence alterations include, but are not limited to, substitutions (conservative and non conservative), deletions, mutations, frameshifts, and insertions In one example, a given polypeptide binds an antibody, and a functional equivalent is a polypeptide that binds the same antibody Thus a functional equivalent includes peptides that have the same binding specificity as a polypeptide, and that can be used as a reagent in place of the polypeptide (such as in the production of pantothenic acid and 3-HP). In one example a functional equivalent includes a polypeptide wherein the binding sequence is discontinuous, wherein the antibody binds a linear epitope Thus, if the peptide sequence is MNIVNIRKKF (amino acids 1-10 of SEQ ID NO: 19) a functional equivalent includes discontinuous epitopes, that can appear as follows (**=any number of intervening amino acids): NH₂ -**-M**N**I**V**N**I**R**K**K**F-COOH. In this example, the polypeptide is functionally equivalent to amino acids 1-10 of SEQ ID NO: 19 if the three dimensional structure of the polypeptide is such that it can bind a monoclonal antibody that binds amino acids 1-10 of SEQ ID NO: 19.

5 **Hybridization:** The ability of complementary single-stranded DNA or RNA to form a duplex molecule In some examples, hybridization is used to determine the complementarity between two or more nucleotide sequences Nucleic acid hybridization techniques can be used to obtain an isolated nucleic acid within the scope of the disclosure. Briefly, any nucleic acid molecule having some homology to an alanine 2,3-aminomutase (such as homology to SEQ ID NO: 1, 3, 5, 18, 20, 42, 44, 46, 48, or 50, or variants or fragments thereof) can be used as a probe to identify a similar nucleic acid molecule by hybridization under conditions of moderate to high stringency Once identified, the nucleic acid then can be purified, sequenced, and analyzed to determine if it is an alanine 2,3-aminomutase having alanine 2,3-aminomutase activity.

Hybridization can be done by Southern or Northern analysis to identify a DNA or RNA sequence, respectively, that hybridizes to a probe The probe can be labeled, for example with a biotin, a fluorophore, digoxygenin, an enzyme, or a radioisotope such as ³²P The DNA or RNA to be analyzed can be electrophoretically separated on an agarose or polyacrylamide gel, transferred to nitrocellulose, nylon, or other suitable membrane, and hybridized with the probe using standard techniques well known in the art such as those described in sections 7.39-7-52 of Sambrook *et al.,* (1989) Molecular Cloning, second edition, Cold Spring Harbor Laboratory, Plainview, NY. Typically, a probe is at least about 20 nucleotides in length For example, a probe including 20 contiguous nucleotides of an alanine 2,3-aminomutase (such as 20 contiguous nucleotides of SEQ ID NO: 1, 3, 5, 18, 20, 42, 44, 46, 48, or 50) can be used to identify an identical or similar nucleic acid In addition, probes longer or shorter than 20 nucleotides can be used

The disclosure also provides isolated nucleic acid sequences that are at least about 12 nucleotides in length (such as at least about 13, 14, 15, 16, 17, 18, 19, 20, 25, 30, 40, 50, 60, 100, 250, 500, 750, 1000, 1400, 2000, 3000, 4000, or 5000 nucleotides in length) and hybridize, under hybridization conditions, to the sense or antisense strand of an alanine 2,3-aminomutase nucleic acid sequence, for example SEQ ID NO: 1, 3, 5, 18, 20, 42, 44, 46, 48, or 50). The hybridization conditions can be moderately or highly stringent hybridization conditions.

Moderately stringent hybridization conditions are when the hybridization is performed at about 42°C in a hybridization solution containing 25 mM KPO₄ (pH 74), 5X SSC, 5X Denhart's solution, 50 µg/mL denatured, sonicated salmon sperm DNA, 50% formamide, 10% Dextran sulfate, and 1-15 ng/mL probe (about 5x10⁷ cpm/µg), while the washes are performed at about 50°C with a wash solution containing 2X SSC and 0.1% sodium dodecyl sulfate.

Highly stringent hybridization conditions are when the hybridization is performed at about 42°C in a hybridization solution containing 25 mM KPO₄ (pH 7.4), 5X SSC, 5X Denhart's solution, 50 µg/mL denatured, sonicated salmon sperm DNA, 50% formamide, 10% Dextran sulfate, and 1-15 ng/mL probe (about 5x10⁷ cpm/µg), while the washes are performed at about 65°C with a wash solution containing O.2X SSC and 0.1% sodium dodecyl sulfate

**Isolated:** An "isolated" biological component (such as a nucleic acid molecule or protein) has been substantially separated or purified away from other biological components in the cell of the 5 organism in which the component naturally occurs (such as other chromosomal and extrachromosomal DNA and RNA, and proteins). Nucleic acid molecules and proteins that have been "isolated" include nucleic acid molecules and proteins purified by standard purification methods, The term also embraces nucleic acid molecules and proteins prepared by recombinant expression in a host cell as well as chemically synthesized nucleic acids, proteins and peptides

In one example, isolated refers to a natuzally-accurring nucleic acid molecule that is not immediately contiguous with both of the sequences with which it is immediately contiguous (one on the 5' end and one on the 3' end) in the naturally-occurring genome of the organism from which it is derived For example, an isolated nucleic acid molecule can be, without limitation, a recombinant DNA molecule of any length, provided one of the nucleic acid sequences normally found immediately flanking that recombinant DNA molecule in a naturally-occurring genome is removed or absent Thus, an isolated nucleic acid molecule includes, without limitation, a recombinant DNA that exists as a separate molecule (such as a cDNA or a genomic DNA, fragment produced by PCR or restriction endonuclease treatment) independent of other sequences as well as recombinant DNA that is incorporated into a vector, an autonomously replicating plasmid, a virus (such as a retrovirus, adenovirus, or herpes virus), or into the genomic DNA of a prokaryote or eukaryote In addition, an isolated nucleic acid can include a recombinant DNA molecule that is part of a hybrid or fusion nucleic acid sequence

In one example, the term "isolated" as used with reference to a nucleic acid molecule also includes any non-naturally occurring nucleic acid sequence since non-naturally-occurring nucleic acid sequences are not found in nature and do not have immediately contiguous sequences in a naturally-occurring genome For example, non-naturally-occurring nucleic acid molecule such as an engineered nucleic acid molecule is considered to be isolated nucleic acid. Engineered nucleic acid molecules can be made using common molecular cloning or chemical nucleic acid synthesis techniques. Isolated non-naturally-accurring nucleic acid molecules can be independent of other sequences, or incorporated into a vector, an autonomously replicating plasmid, a virus (such as a retrovirus, adenovirus, or herpes virus), or the genomic DNA of a prokaryote or eukaryote In addition, a non-naturally-occurring nucleic acid molecules can include a nucleic acid molecule that is part of a hybrid or fusion nucleic acid sequence

**Lysine 2,3-aminomutase:** An enzyme which can convert alpha-lysine to beta-lysine Includes any lysine 2,3-aminomutase gene, cDNA, RNA, or protein from any organism, such as a prokaryote, for example *Bacillus subtilis, Clostridium subterminale, Porphyromonas gingivalis, Fusobacterium nucleatum, Clostridium sticklandii,* or *Escherichia coli.* This description includes lysine 2,3-aminomutase allelic variants, as well as any variant, fragment, or fusion sequence which retains the ability to convert alpha-lysine to beta-lysine. In one example, includes peptides encoded by genes annotated as lysine 2,3-aminomutase in public sequence databases, such as GenBank and EMBL Particular examples of lysine 2,3-aminomutases proteins (and the corresponding nucleic acid sequences) include the following publicly available sequences: GenBank Accession Nos YP 028406 *for Bacillus anthracis* str Sterne; BAC95867 for *Vibrio vulnificus* YJ016; ZP_00330962 for *Moorel*/*a thermoacetica;* ZP_00322274 for *Haemophilus influenzae;* ZP_00298043 for *Methanasarcina barkeri* str Fusaro; ZP_00314982 for *Microbulbifer degradans;* and NP_781545 for *Clostridium tetani* E88

**Mutated lysine 2,3 aminomutase:** A lysine 2,3 aminomutase molecule containing one or more amino acid substitutions that result in a peptide having alanine 2,3, aminomutase activity. Examples of such mutations include, but are not limited to, one or more of the following : P/S11I; N19Y; L/K/R/I26I; E/R30K; L/V32A; K36E; S/T/C52R; L/I53P/H; Y63F; E/N/D71G; IM/S85Q;Q/L7E86R; Q/L95M; K/M/Q125L; M128V; Y132H; Q/S141R; A/D/S/M144G; D179N; K/Q187R; I192V; I228M; D331G/H; M/Q342I; or K/Q/T398E, such as at least 2, at least 3, at least 4, at least 5, at least 6, at least 8, at least 9, at least 10, at least 11, at least 12, at least 13, at least 14, at least 15, at least 16, at least 17, at least 18, at least 19, at least 20, at least 21, at least 22, at least 23, at least 24, or no more than 3, no more than 4, no more than 5, no more than 6, no more than 8, no more than 9, no more than 10, no more than 11, no more than 12. no more than 13, no more than 14, no more than 15, no more than 16, no more than 17, no more than 18, no more than 19, no more than 20, no more than 21, no more than 22, or no more than 23 of such substitutions. Particular combinations of such substitutions include, but are not limited to: ((1) N19Y, L/T53P/H, H/I/S85Q, D331G/H, and M/Q342T; (2) N19Y, E/R30K, L/T53P/H, H/I/S85Q, I192V, D331G/H, and M/Q342I; (3) N19Y, L/K/R/T26I; E/R30K, L/I53P/H, H/I/S8SQ, I192V, D331G/H, andM/Q342T; (4) E/R30K, Y63F, Q/L/E86R, Q/L95M, M128V, A/D/S/M144G, L228M, D331G/H, and K/Q/T398E, (5) E/R30K, K36E, Y63F, Q/L/E86R, Q/L95M, M128V, A/D/S/M144G, D179N, L228M, D331G/H, and K/Q/T398E; (6) E/R30K, Q/L95M, M128V, and D331G/H; (7) P/S11I, E/R30K, Q/L95M, M128V, Q/S141R, K/Q187R, and D331G/H; (8) E/R30K, L/V32A, L/I53P/H, E/N/D71G, Q/L95M; K/M/Q125L, M128V, and D331G/H; (9) E/R30K, C52R, Q/L95M; M128V, andD331G/11; (10) Q/L95M, M128V, and D331G/H; (11) Q/L95M, M128V; Y132H, and D331G/H; and (12) Q/L95M, M128V, and D331G/H

**Nucleic acid:** Encompasses both RNA and DNA including, without limitation, cDNA, genomic DNA, and synthetic (such as chemically synthesized) DNA The nucleic acid can be double-stranded or single-stranded Where single-stranded, the nucleic acid can be the sense strand or the antisense strand In addition, nucleic acid can be circular or linear.

**Oligonucleotide: A** linear polynucleotide (such as DNA or RNA) sequence of at least 9 nucleotide, for example at least 12, at least 15, at least 18, at least 20, at least 24, at least 25, at least 27, at least 30, at least 50, at least 100 or even at least 200 nucleotides long In particular examples, an oligonucleotide includes at least 9 contiguous nucleotides of SEQ ID NO: 1, 3, 5, 18, 20, 42, 44, 46, 48, or 50 (or the complementary strand thereof), such as at least 12, at least 15, at least 18, at least 20, at least 24, at least 25, at least 2 7, at least 30, at least 50, at least 100 or even at least 200 contiguous nucleotides of SEQ ID NO: 1, 3, 5, 18, 20, 42, 44, 46, 48, or 50 (or the complementary strand thereof

**Operable linked:** A first nucleic acid sequence is operably linked with a second nucleic acid sequence when the first nucleic acid sequence is placed in a functional relationship with the second nucleic acid sequence For instance, a promoter is operably linked to a coding sequence if the promoter affects the transcription or expression of the coding sequence Generally, operably linked DNA sequences are contiguous and, where necessary to join two protein coding regions, in the same reading frame.

**ORF (open reading frame):** A series of nucleotide triplets (codons) coding for amino acids without any termination codons These sequences are usually translatable into a peptide

**Pantothenate or Pantothenic Acid:** A commercially significant vitamin which is used in cosmetics, medicine, and nourishment. The terms pantothenic acid and pantothenate are used interchangeably herein, and refer not only to the free acid but also to the salts of D-pantvthenic acid, such as the calcium salt, sodium salt, ammonium salt or potassium salt Pantothenate can be produced by chemical synthesis or biotechnologically from beta-alanine using the cells and methods disclosed herein Methods for measuring the amount of pantothenate are known (for example see U.S. Patent No 6,184,006 to Rieping et al and U.S. Patent No. 6,177,264 to Eggeling et al ). For example, a quantitative determination of D-parztothenate can be made by using the *Lactobacillus plantarum* pantothenate assay (test strain: *Lactobacillus plantarum* AICC 8014, Cat No 3211-30-3; culture medium: Bacto pantothenate assay medium (DIFCO Laboratories, Michigan, USA), cat No 0604-15-3). This indicator strain can grow only in the presence of pantothenate in the indicated culture medium and displays a photometrically measurable, linear dependency of the growth on the concentration of pantothenate in the medium The hemicalcium salt of pantothenate can be used for calibration (Sigma Catalog Number P 2250). The optical density can be determined at a wavelength of 580 nm

**Peptide Modifications:** The present disclosure includes alanine 2,3-aminomutase peptides, as well as synthetic embodiments In addition, analogues (non-peptide organic molecules), derivatives (chemically functionalized peptide molecules obtained starting with the disclosed peptide sequences) and variants (homologs) having alanine 2,3-aminomutase activity can be utilized in the methods described herein. The peptides disclosed herein include a sequence of amino acids that can be either L- or D- amino acids, naturally occurring and otherwise

Peptides can be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6- membered ring. Amino groups of the peptide, whether amino-terminal or side chain, may be in the form of a pharmaceutically-acceptable acid addition salt, such as the HCl, HBI, acetic, benzoic, toluene sulfonic, maleic, tartaric and other organic salts, or may be modified to C₁-C₁₆ alkyl or dialkyl amino or further converted to an amide.

Hydroxyl groups of the peptide side chains may be converted to C₁-C₁₆ alkoxy or to a C₁-C₁₆ ester using well-recognized techniques. Phenyl and phenolic rings of the peptide side chains may be substituted with one or more halogen atoms, such as F, Cl, Br or I, or with C₁-C₁₆ alkyl, C₁-C₁₆ alkoxy, carboxylic acids and esters thereof, or amides of such carboxylic acids Methylene groups of the peptide side chains can be extended to homologous C₂-C₄ alkylenes Ihiols can be protected with any one of a number of well-recognized protecting groups, such as acetamide groups Those skilled in the art will also recognize methods for introducing cyclic structures into the peptides of this disclosure to select and provide conformational constraints to the structure that result in enhanced stability For example, a C- or N-terminal cysteine can be added to the peptide, so that when oxidized the peptide will contain a disulfide bond, generating a cyclic peptide. Other peptide cyclizing methods include the formation of thioethers and carboxyl- and amino-terminal amides and esters.

Peptidomimetic and organomimetic embodiments are also within the scope of the present disclosure, whereby the three-dimensional arrangement of the chemical constituents of such peptido-and organomimetics mimic the three-dimensional arrangement of the peptide backbone and component amino acid side chains, resulting in such peptido- and organomimetics of the proteins of this invention having detectable alanine 2,3-aminomutase activity. For computer modeling applications, a phatmacophore is an idealized, three-dimensional definition of the structural requirements for biological activity. Peptido- and organomimetics can be designed to fit each pharmacophore with current computer modeling software (using computer assisted drug design or CADD). See Walters, "Computer-Assisted Modeling of Drugs", in Klegerman & Groves, eds, 1993, Pharmaceutical Biotechnology, Interpharm Press: Buffalo Grove, IL, pp. 165-174 and Principles of Pharmacology Munson (ed) 1995, Ch 102, for descriptions of techniques used in CADD Also included within the scope of'the disclosure are mimetics prepared using such techniques, In one example, a mimetic mimics the alanine 2,3-aminomutase activity generated by an alanine 2,3-aminomutase or a variant, fragment, or fusion thereof:

**Polynucleotide:** A linear nucleic acid sequence of any length Therefore, a polynucleotide includes molecules which are at least 15, at least 25, at least 50, at least 75, at least 100, at least 200 at least 300, at least 400, at least 500, at least 600, at least 700, at least 800, at least 900, at least 1000, at least 1100, or even at least 1200 nucleotides long In particular examples, a polynucleotide includes at least 15 contiguous nucleotides of SEQ ID NO: 1, 3, 5, 18, 20, 42, 44, 46, 48, or 50 (or the complementary strand thereof), such as at least 25, at least 50, at least 75, at least 100, at least 200 at least 300, at least 400, at least 500, at least 600, at least 700, at least 800, at least 900, at least 1000, at least 1100, or even at least 1200 contiguous nucleotides of SEQ ID NO: 1, 3, 5, 18, 20, 42, 44, 46, 48, or 50 (or the complementary strand thereof)

**Probes and primers:** A "probe" includes an isolated nucleic acid molecule containing a detectable label or reporter molecule Exemplary labels include radioactive isotopes, ligands, chermiluminescent agents, fluorophores, and enzymes Methods for labeling and guidance in the choice of labels appropriate for various purposes are discussed in, for example, Sambrook et al. (ed), Molecular Cloning: A Laboratory Manual 2nd ed., vol 1-3, Cold Spring Harbor Laboratory Press, Cold Spring Harbor, N.Y., 1989, and Ausubel et al (ed.) Current Protocols in Molecular Biology, Greene Publishing and Wiley-Interscience, New York (with periodic updates), 198 7..

"Primers" are typically nucleic acid molecules having ten or more nucleotide (such as nucleic acid molecules having between about 10 nucleotides and about 100 nucleotides) A primer can be annealed to a complementary target nucleic acid strand by nucleic acid hybridization to form a hybrid between the primer and the target nucleic acid strand, and then extended along the target nucleic acid strand by, for example, a DNA polymerase enzyme. Primer pairs can be used for amplification of a nucleic acid sequence, for example, by the polymerase chain reaction (PCR) or other nucleic-acid amplification methods.

Methods for preparing and using probes and primers are described, for example, in references such as Sambrook et al. (ed), Molecular Cloning: A Laboratory Manual, 2nd ed , vol 1-3, Cold Spring Harbor Laboratory Press, Cold Spring Harbor, N.Y., 1989; Ausubel et al. (ed.), Current Protocols in Molecular Biology, Green Publishing and Wiley-Interscience, New York (with periodic updates), 1987; and Innis et al., PCR Protocols: A Guide to Methods and Applications, Academic Press: San Diego, 1990. PCR primer pairs can be derived from a known sequence, for example, by using computer programs intended for that purpose such as Primer (Version 05, © 1991, Whitehead Institute for Biomedical Research, Cambridge, Mass.). One of skill in the art will appreciate that the specificity of a particular probe or primer increases with the length, but that a probe or primer can range in size from a full-length sequence to sequences as short as five consecutive nucleotides. Thus, for example, a primer of 20 consecutive nucleotides can anneal to a target with a higher specificity than a corresponding primer of only 15 nucleotides. Thus, in order to obtain greater specificity, probes and primers can be selected that include, for example, 25, 30, 35, 40, 50, 60, 70, 80, 90, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, or more consecutive nucleotides.

**Promoter:** An array of nucleic acid control sequences which direct transcription of a nucleic acid A promoter includes necessary nucleic acid sequences near the start site of transcription, such as, in the case of a polymerase II type promoter, a TATA element A promoter also optionally includes distal enhancer or repressor elements which can be located as much as several thousand base pairs from the start site of transcription

**Purified:** The term purified does not require absolute purity; rather, it is intended as a relative term Thus, for example, a purified peptide preparation is one in which the peptide or protein is more enriched than the peptide or protein is in its environment within a cell, such that the peptide is substantially separated from cellular components (nucleic acids, lipids, carbohydrates, and other polypeptides) that may accompany it. In another example, a purified peptide preparation is one in which the peptide is substantially-free from contaminants, such as those that might be present following chemical synthesis of the peptide

In one example, an alanine 2,3-aminomutase peptide is purified when at least 50% by weight of a sample is composed of the peptide, for example when at least 60%, 70%, 80%, 85%, 90%, 92%, 95%, 98%, or 99% or more of a sample is composed of the peptide Examples of methods that can be used to purify a peptide, include, but are not limited to the methods disclosed in Sambrook et al (Molecular Cloning A Laboratory Manual, Cold Spring Harbor, New York, 1989, Ch. 17). Protein purity can be determined by, for example, polyacrylamide gel electrophoresis of a protein sample, followed by visualization of a single polypeptide band upon staining the polyacrylamide gel; highpressure liquid chromatography; sequencing; or other conventional methods.

**Recombinant:** A recombinant nucleic acid molecule is one that has a sequence that is not naturally occurring or has a sequence that is made by an artificial combination of two otherwise separated segments of sequence This artificial combination can be accomplished using methods known in the art, such as chemical synthesis and the artificial manipulation of isolated segments of nucleic acid molecules, such as by genetic engineering techniques Recombinant is also used to describe nucleic acid molecules that have been artificially manipulated, but contain the same regulatory sequences and coding regions that are found in the organism from which the nucleic acid was isolated.

**Sequence identity/similarity:** The identity/similarity between two or more nucleic acid sequences, or two or more amino acid sequences, is expressed in terms of the identity or similarity between the sequences Sequence identity can be measured in terms of percentage identity; the higher the percentage, the more identical the sequences are Sequence similarity can be measured in terms of percentage similarity (which takes into account conservative amino acid substitutions); the higher the percentage, the more similar the sequences are Homologs or orthologs of nucleic acid or amino acid sequences possess a relatively high degree of sequence identity/similarity when aligned using standard methods This homology is more significant when the orthologous proteins or cDNAs are derived from species which are more closely related (such as human and mouse sequences), compared to species more distantly related (such as human and *C*. *elegans* sequences).

Methods of alignment of sequences for comparison are well known in the art Various programs and alignment algorithms are described in: Smith & Waterman, Adv. Appl Math 2:482, 1981; Needleman & Wunsch, J Mol Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc acids Res 16:10881-90, 1988; Huang et al Computer Appls in the Biosciences 8, 155-65, 1992; and Pearson et al., Meth. Mol. Bio. 24:307-31, 1994. Altschul et al., J. Mol Biol 215:403-10, 1990, presents a detailed consideration of sequence alignment methods and homology calculations.

The NCBI Basic Local Alignment Search Tool (BLAST) (Altschul et al., J. Mol Biol 215:40.3-10, 1990) is available from several sources, including the National Center for Biological Information (NCBI, National Library of Medicine, Building 38A, Room 8N805, Bethesda, MD 20894) and on the Internet, for use in connection with the sequence analysis programs blastp, blastn, blastx, tblastn and tblastx Additional information can be found at the NCBI web site

BLASTN is used to compare nucleic acid sequences, while BLASIP is used to compare amino acid sequences To compare two nucleic acid sequences, the options can be set as follows: -i is set to a file containing the first nucleic acid sequence to be compared (such as C:\seq1 txt); -j is set to a file containing the second nucleic acid sequence to be compared (such as C:\seq2 txt); -p is set to blastn; -o is set to any desired file name (such as C:\output txt); -q is set to -1; -r is set to 2; and all other options are left at their default setting. For example, the following command can be used to generate an output file containing a comparison between two sequences: C:\B12seq -i c:\seq1 txt-j c:\seq2 txt -p blastn -o c:\output txt -q -1 -r 2

To compare two amino acid sequences, the options of B12seq can be set as follows: -i is set to a file containing the first amino acid sequence to be compared (such as C:\seq1.txt); -j is set to a file containing the second amino acid sequence to be compared (such as C:\seq2.txt); -p is set to blastp; -o is set to any desired file name (such as C:\output.txt); and all other options are left at their default setting For example, the following command can be used to generate an output file containing a comparison between two amino acid sequences: C:\B12seq -i c:\seq1.txt-j c:\seq2.txt-p blastp -o c:\output txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences.. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. For example, a nucleic acid sequence that has 1166 matches when aligned with a test sequence having 1554 nucleotides is 75.0 percent identical to the test sequence (1166÷1554*100=75.0). The percent sequence identity value is rounded to the nearest tent For example, 75.11, 75.12, 75.13, and 75.14 are rounded down to 75.1, while 75.15, 75.16, 75.17, 75.18, and 75.19 are rounded up to 75.2 The length value will always be an integer In another example, a target sequence containing a 20-nucleotide region that aligns with 20 consecutive nucleotides from an identified sequence as follows contains a region that shares 75 percent sequence identity to that identified sequence (i.e., 15÷20*100=75).

                    1                  20
Target Sequence:    AGGTCGTGTACTGTCAGTCA
                    | || ||| |||| |||| |
Identified Sequence: ACGTGGTGAACTGCCAGTGA

For comparisons of amino acid sequences of greater than about 30 amino acids, the Blast 2 sequences function is employed using the default BLOSUM62 matrix set to default parameters, (gap existence cost of 11, and a per residue gap cost of 1). Homologs are typically characterized by possession of at least 70% sequence identity counted over the full-length alignment with an amino acid sequence using the NCBI Basic Blast 2.0, gapped blastp with databases such as the nr or swissprot database. Queries searched with the blastn program are filtered with DUST (Hancock and Armstrong, 1994, Comput Appl Biosci 10:67-70) Other programs use SEG. In addition, a manual alignment can be performed Proteins with even greater similarity will show increasing percentage identities when assessed by this method, such as at least 75%, 80%, 85%, 90%, 95%, or 99% sequence identity

When aligning short peptides (fewer than around 30 amino acids), the alignment should be performed using the Blast 2 sequences function, employing the PAM30 matrix set to default parameters (open gap 9, extension gap 1 penalties) Proteins with even greater similarity to the reference sequence will show increasing percentage identities when assessed by this method, such as at least 60%, 70%, 75%, 80%, 85%, 90%, 95%, 98%, 99% sequence identity When less than the entire sequence is being compared for sequence identity, homologs will typically possess at least 75% sequence identity over short windows of 10-20 amino acids, and can possess sequence identities of at least 85%, 90%, 95% or 98% depending on their identity to the reference sequence Methods for determining sequence identity over such short windows are described at the NCBI web site

One indication that two nucleic acid molecules are closely related is that the two molecules hybridize to each other under stringent conditions Stringent conditions are sequence-dependent and are different under different environmental parameters Nucleic acid molecules that hybridize under stringent conditions to an alanine 2,3-aminomutase gene sequence typically hybridize to a probe based on either an entire alanine 2,3-aminomutase gene or selected portions of the gene, respectively, under conditions described above.

Nucleic acid sequences that do not show a high degree of'identity may nevertheless encode identical or similar (conserved) amino acid sequences, due to the degeneracy of the genetic code. Changes in a nucleic acid sequence can be made using this degeneracy to produce multiple nucleic acid molecules that all encode substantially the same protein. Such homologous nucleic acid sequences can, for example, possess at least 60%, 70%, 80%, 90%, 95%, 98%, or 99% sequence identity determined by this method.

One of skill in the art will appreciate that these sequence identity ranges are provided for guidance only; it is possible that strongly significant homo logs could be obtained that fall outside the ranges provided.

An alternative (and not necessarily cumulative) indication that two nucleic acid sequences are substantially identical is that the polypeptide which the first nucleic acid encodes is immunologically cross reactive with the polypeptide encoded by the second nucleic acid.

Specific binding agent: An agent that binds substantially only to a defined target, such as a peptide target. For example, an alanine 2,3-aminomutase binding agent includes anti-alanine 2,3-aminomutase antibodies and other agents (such as a peptide or drug) that bind substantially to only an alanine 2,3-aminomutase Antibodies to an alanine 2,3-aminomutase protein (or fragments thereof) can be used to purify or identify such a protein.

**Transformed:** A transformed cells is one into which a nucleic acid molecule has been introduced, for example by molecular biology techniques Transformation encompasses all techniques by which a nucleic acid molecule can be introduced into such a cell, including, but not limited to transfection with viral vectors, conjugation, transformation with plasmid vectors, and introduction of naked DNA by electroporation, lipofection, and particle gun acceleration.

**Variants, fragments or fusions:** The disclosed alanine 2,3 aminomutase sequences include variants, fragments, and fusions thereof that retain alanine 2,3 aminomutase activity. DNA sequences which encode for an alanine 2,3 aminomutase protein (for example SEQ ID NO: 1, 3, 5, 18, 20, 42, 44, 46, 48, or 50), fusion alanine 2,3 aminomutase protein, or a fragment or variant of an alanine 2,3 aminomutase protein, can be engineered to allow the protein to be expressed in eukaryotic cells, bacteria, insects, or plants. To obtain expression, the DNA sequence can be altered and operably linked to other regulatory sequences. The final product, which contains the regulatory sequences and the protein, is referred to as a vector. This vector can be introduced into eukaryotic, bacteria, insect, or plant cells. Once inside the cell the vector allows the protein to be produced.

A fusion protein includes an alanine 2,3-aminomutase (or variant or fragment thereof), for example SEQ ID NO: 19, 21, 43, 45, 47, 49, or 51, linked to other amino acid sequences that do not significantly decrease alanine 2,3-aminomutase activity, for example the ability to convert alpha-alanine to beta-alanine. In one example, the other amino acid sequences are no more than about 10, 12, 15, 20, 25, 30, or 50 amino acids in length. In addition, spacer sequences can be placed between the alanine 2,3-aminomutase sequence and the additional amino acid sequence. Such spacers can be at least 4, at least 6, or at least10 amino acids

One of ordinary skill in the art will appreciate that a DNA sequence can be altered in numerous ways without affecting the biological activity of the encoded protein For example, PCR can be used to produce variations in the DNA sequence which encodes an alanine 2,3-aminomutase Such variants can be variants optimized for codon preference in a host cell used to express the protein, or other sequence changes that facilitate expression.

**Vector:** A nucleic acid molecule as introduced into a cell, thereby producing a transformed cell A vector may include nucleic acid sequences that permit it to replicate in the cell, such as an origin of replication. A vector may also include one or more selectable marker genes and other genetic elements known in the art

### Alanine 2,3-Aminomutase Nucleic Acids and Polypeptides

Polypeptides having alanine 2,3-aminomutase activity are disclosed herein In one example, the polypeptide is a mutated lysine 2,3-aminomutase sequence. In one example, a mutated lysine 2,3 aminomutase includes one or more of the following substitutions: P/S11T; N19Y; L/K/R/T26I; E/R30K; L/V32A; K36E; S/T/C52R; L/T53P/H; Y63F; E/N/D71G; H/I/S85Q;Q/L/E86R; Q/L95M; K/M/QI25L; M128V; Y132H; Q/S141R; A/D/S/M144G; D179N; K/Q187R; I192V; L228M; D331 G/H; M/Q342T; or K/Q/T398E, where the letter(s) before the number represents the one letter amino acid code for the amino acid found in a lysine 2,3 aminomutase, the number represents the amino acid position based on the numbering for *Porphyromonas gingivalis* lysine 2,3 aminomutase (SEQ ID NO: 52) (see FIG. 7), and the letter(s) after the number represents the one letter amino acid code for the amino acid found in the alanine 2,3 aminomutase The actual first amino acid and amino acid number can vary depending on the lysine 2,3 aminomutase sequence to be mutated. However, one skilled in the art is able to determine the position in the homologous sequence by aligning the sequences. For example, as shown in FIG. 7, position 128 of *Porphyromonas gingivalis* lysine 2,3 aminomutase corresponds to position 129 of *Fusobacterium nucleatum* lysine 2,3 aminomutase (SEQ ID NO: 10), position 126 of *Clostridium sticklandii* lysine 2,3 aminomutase (SEQ ID NO: 33), and position 136 of *Bacillus subtilis* lysine 2,3 aminomutase (SEQ ID NO: 59). A similar analysis can be made using methods known in the art for each of the remaining 24 positions based on the information provided in FIG. 7 and other publicly available lysine 2,3 aminomutase sequences.

Any lysine 2,3 aminomutase can be mutagenized using standard molecular biology methods to generate an alanine 2,3-aminomutase. For example, lysine 2,3 aminomutases from a prokaryote such as *Bacillus, Clostridium, Escherichia, Fusobacterium, Haemophilus, Methanosarcina, Microbulbifer; Moorella, Porphyromonas, Thermoanaerobacter* or *Vibrio* can be mutated to include one or more of the following substitutions, P/S11P; N19Y; L/K/R/T26I; E/R30K; L/V32A; K36E; S/T/C52R; L/T53P/H; Y63F; E/N/D71G; H/I/S85Q; Q/L/E86R; Q/L95M, K/M/Q125L; M128V; Y132H; Q/S141R; A/D/S/M144G; D179N; K/Q187R; I192V; L228M; D331G/H; M/Q342T; or K/Q/T398E, such as at least 2, at least 3, at least 4, at least 5, at least 6, at least 8, at least 9, at least 10, at least 11, at least 12, at least 13, at least 14, at least 15, at least 16, at least 17, at least 18, at least 19, at least 20, at least 21, at least 22, at least 23, at least 24, or no more than 3, no more than 4, no more than 5, no more than 6, no more than 8, no more than 9, no more than 10, no more than 11, no more than 12, no more than 13, no more than 14, no more than 15, no more than 16, no more than 17, no more than 18, no more than 19, no more than 20, no more than 21, no more than 22, or no more than 23 of such substitutions. Particular combinations of such substitutions include, but are not limited to: (1) N19Y, L/T53P/H, H/I/S85Q, D331G/H, and M/Q342T; (2) N19Y, E/R30K, L/T53P/H, H/I/S85Q, I192V, D331G/H, and M/Q342T; (3) N19Y, L/K/R/T261; E/R30K, L/T53P/H, H/I/S85Q, I192V, D331G/H, and M/Q342I; (4) E/R30K, Y63F, Q/L/E86R, Q/L95M, M128V, A/D/S/M144G, L228M, D331G/H, and K/Q/T398E; (5) E/R30K, K36E, Y63F, Q/L/E86R, Q/L95M, M128V, A/D/S/M144G, D179N, L228M, D331G/H, and K/Q/I398E; (6) E/R30K, Q/L95M, M128V, and D331G/H; (7) P/S11I, E/R30K, Q/L95M, M128V, Q/S141R, K/Q187R, and D331G/H; (8) E/R30K, L/V32A, L/T53P/H, E/N/D71G, Q/L95M; K/M/Q125L, M128V, and D331G/H; (9) E/R30K, C52R, Q/L95M; M128V, and D331G/H; (10) Q/L95M, M128V, and D331G/H; (11) Q/L95M, M128V; Y132H, and D331G/H; and (12) Q/L95M, M128V, and D331G/H

Specific examples of peptides having alanine 2,3-aminomutase activity are shown in SEQ ID NOS: 19, 21, 43, 45, 47, 49, and 51. However, the disclosure also encompasses variants, fusions, and fragments of SEQ ID NOS: 19, 21, 43, 45, 47, 49, and 51 which retain alanine 2,3-aminomutase activity. In particular examples, variants, fusions, and fragments of SEQ ID NOS: 19, 21, 43, 45, 47, 49, and 51 have at least 50% of the alanine 2,3-aminomutase activity as SEQ ID NO: 41, such as at least 60%, at least 70%, at least 80%, at least 90%, at least 95%, at least 98% or even at least 100% of the alanine 2,3-aminomutase activity of SEQ ID NO: 41. In other examples, variants, fusions, and fragments of SEQ ID NOS: 19, 21, 43, 45, 47, 49, and 51 have at least 50% of the alanine 2,3-aminomutases activity as SEQ ID NO: 51, such as at least 60%, at least 70%, at least 80%, at least 90%, at least 95%, at least 98% or even at least 100% of the alanine 2,3-aminomutase activity of SEQ ID NO: 51

Examples of fragments which can be used include, but are not limited to: amino acids 1-390, 15-390, 50-390, 50-350, 60-350, 15-340, 75-340,19-331, or 100-339 of SEQ ID NO: 2 or 4 (the corresponding fragments in SEQ ID NOS: 6, 19, 21, 43, 45, 47, 49, and 51 (such as amino acids 42-342 of SEQ ID NO: 6,49 or 51) are encompassed by this disclosure, and can be determined by using FIG 7). The disclosure also provides alanine 2,3-aminomutase peptides that contain at least 15 contiguous amino acids of SEQ ID NO: 19, 21, 43, 45, 4*7,* 49, or 51, which retain alanine 2,3-aminomutase activity Alanine 2,3-aminomutase peptides can also include at least 16, at least 17, at least 18, at least 19, at least 20, at least 21, at least 22, at least 23, at least 24, at least 25, at least 26, at least 27, at least 28, at least 29, at least 30, at least 50, at least 75, at least 100, at least 150, at least 200, at least 250, at least 300 or more contiguous amino acid residues of SEQ ID NO: 19, 21, 43, 45, 47, 49, or 5

Variants include substitution of one or more amino acids, such as one or more conservative amino acid substitutions, one or more non-conservative amino acid substitutions, or combinations thereof Variants also include deletion or insertion of one or more amino acids (or combinations thereof, such as a single deletion together with multiple insertions), such as addition or deletion of no more than 50 amino acids, no more than 20 amino acids, no more than 10 amino acids, no more than 5 amino acids, or no more than 2 amino acids, such as an addition or deletion of 1-5 amino acids, 1-10 amino acids, or 2-20 amino acids In one example, a variant alanine 2,3 aminomutase has no more than 5, no more than 10, no more than 20, no more than 30, no more than 40, or no more than 50 conservative substitutions. Non-conservative substitutions are those wherein the amino acids have more substantial difference, such as their effect on maintaining: (a) the structure of'the polypeptide backbone in the area of the substitution, for example, as a sheet or helical conformation; (b) the charge or hydrophobicity of the polypeptide at the target site; or (c) the bulk of the side chain. The substitutions that in general are expected to produce the greatest changes in polypeptide function are those in which: (a) a hydrophilic residue, such as serine or threonine, is substituted for (or by) a hydrophobic residue, such as leucine, isoleucine, phenylalanine, valine or alanine; (b) a cysteine or proline is substituted for (or by) any other residue; (c) a residue having an electropositive side chain, such as lysine, arginine, or histidine, is substituted for (or by) an electronegative residue, such as glutamic acid or aspartic acid; or (d) a residue having a bulky side chain, such as phenylalanine, is substituted for (or by) one not having a side chain, such as glycine The effects of these amino acid substitutions (or other deletions or additions) can be assessed for peptides having alanine 2,3-aminomutase activity by analyzing the ability of the peptide to catalyze the conversion of alpha-alanine to beta-alanine using the assays disclosed herein.

Variant alanine 2,3-aminomutase peptide sequences can be produced by manipulating the nucleotide sequence encoding an alanine 2,3-aminomutase peptide using standard procedures such as site-directed mutagenesis or PCR.

Examples of substitutions which can be made, while still retaining alanine 2,3-aminomutase activity, include, but are not limited to: V108L, I240S, D295E, Y290F, or combinations thereof, in SEQ ID NO: 21 (the corresponding substitutions in the other disclosed sequences, such as Y289W for SEQ ID NO: 6, 49 or 51; Y290W for SEQ ID NO: 19, Y287W for SEQ ID NO: 41, 43, 45, or 47; and Y297W for SEQ ID NO: 2 or 4, are encompassed by this disclosure, and can be determined by using FIG 7), as well as combinations thereof. Variant alanine 2,3-aminomutase peptides share at least 60, at least 65, at least 70, at least 75, at least 80, at least 85, at least 90, at least 95, at least 97, at least 98, or at least 99% sequence identity with any of SEQ ID NOS: 2, 4, 6, 19, 21, 43, 45, 47, 49, or 51, as long as the peptide encoded by the amino acid sequence retains alanine 2,3-aminomutase activity

Fusion proteins (and the corresponding nucleic acid sequences) can be generated using the disclosed alanine 2,3-aminomutase sequences. Fusion sequences can include a full-length alanine 2,3-aminomutase protein sequence (such as SEQ ID NO: 2, 4, 6, 19, 21, 43, 45, 47, 49, or 51), or a variant or fragment thereof that has alanine 2,3-aminomutase activity, linked to a second amino acid sequence In some examples, the second amino acid sequence includes at least 5 amino acids, such as at least 10 amino acids, at least 20 amino acids, at least 30 amino acids, at least 50 amino acids, at least 75 amino acids, at least 100 amino acids, at least 150 amino acids, or even at least 200 amino acids. In particular examples, the alanine 2,3-aminomutase sequence and the second amino acid sequence are linked via a spacer sequence. Particular examples of spacers include on or more alanine or glycine residues, or other nonpolar amino acids or neutral polar amino acids In some examples, spacers are no more than 50 amino acids, such as no more than 20 amino acids, no more than 10 amino acids, no more than 5 amino acids, for example 5-50 amino acids.

Also disclosed are isolated nucleic acid molecules that encode polypeptides having alanine 2,3-aminomutase activity, for example a sequence which includes SEQ ID NO: 18, 20, 42, 44, 46, 48, or 50. However, the disclosure also encompasses variants, fusions, and fragments of SEQ ID NOS: 18, 20, 42, 44, 46, 48, and 50 which retain the ability to encode a protein having alanine 2,3-aminomutase activity. In one example an isolated nucleic acid molecule encoding a peptide having alanine 2,3--aminomutase activity is operably linked to a promoter sequence, and can be part of a vector. The nucleic acid can be a recombinant nucleic acid that can be used to transform cells and make transformed cells or transgenic non-human mammals (such as mice, rats, and rabbits).

Transformed cells including at least one exogenous nucleic acid molecule which encodes a peptide having alanine 2,3-aminomutase activity (such as SEQ ID NO: 18, 20, 42, 44, 46, 48, or 50 or fragments, fusions, or variants thereof that retain alanine 2,3-aminomutase activity), are disclosed. In one example, such a transformed cell produces beta-alanine from alpha-alanine. In another example, the cell produces 3-HP, pantothenate, CoA, or organic compounds such as 1,3-propanediol. Transformed cells can be eukaryotic or prokaryotic, such as bacterial cells, plant cells, or yeast cells

Nucleic acid sequences encoding an alanine 2,3-aminomutase can contain an entire nucleic acid sequence encoding the enzyme, as well as a portions thereof that retain the desired enzyme activity. For example, an alanine 2,3-aminomutase nucleic acid molecule can contain at least 15 contiguous nucleotides of an alanine 2,3-aminomutase nucleic acid sequence (such as SEQ ID NO: 18, 20, 42, 44, 46, 48, or 50). It will be appreciated that the disclosure also provides isolated nucleic acid molecules that contain at least 16, at least 17, at least 18, at least 19, at least 20, at least 21, at least 22, at least 23, at least 24, at least 25, at least 26, at least 27, at least 28, at least 29, at least 30, at least 40, at least 50, at least 75, at least 100, at least 200, at least 500, at least 1000, at least 1200 or more nucleotides, such as at least this many contiguous nucleotides of SEQ ID NO: 18, 20, 42, 44, 46, 48, or 50. In some examples, fragments of SEQ ID NO: 18, 20, 42, 44, 46, 48, or 50 (or the complementary strand) do not encode a protein having alanine 2,3-aminomutase but are shorter fragments which can be used as probes or primers.

Variant alanine 2,3-aminomutase nucleic acid sequences are disclosed herein Variants can contain a single insertion, a single deletion, a single substitution, multiple insertions, multiple deletions, multiple substitutions, or any combination thereof (such as a single deletion together with multiple insertions) as long as the peptide encoded thereby retains alanine 2,3-aminomutase activity (or can function as a probe or primer). Such isolated nucleic acid molecules can share at least 60%, at least 70, at least 75, at least 80, at least 85, at least 90, at least 95, at least 97, at least 98, or at least 99% sequence identity with an alanine 2,3-aminomutase sequence (such as SEQ ID NO: 18, 20, 42, 44,46,48, or 50), as long as the peptide encoded by the nucleic acid retains the desired enzyme activity, such as alanine 2,3-aminomutase activity.

For example, the following variations can be made to the alanine 2,3-aminomutase nucleic acid sequence: for SEQ ID NO: 13, 18, or 20, the "t" at position 96 or 384 can be substituted with a "c" "a" or "g"; the "a" at position 438 or 480 can be substituted with a "c" "t" or "g"; the "t" at position 1056 can be substituted with an "g" "a" or "c;" for SEQ ID NO: 42, 44, or 46, the "a" at position 144 can be substituted with a "g"; the "a" at position 672 can be substituted with a "c" "t" or "g"; and the "t" at position 1107; can be substituted with a "c;" for SEQ ID NO: 48 and 50, the "a" at position 576 can be substituted with a "g" "t" or "c; the "c" at 864 can be substituted with a "t"; and the "t" at position 1200; can be substituted with a "g" "a" or "c." Similar substitutions can be made to the other alanine 2,3 amino acid sequences disclosed herein using the sequence listing

The coding region of an alanine 2,3-aminomutase sequence can be altered by taking advantage of the degeneracy of the genetic code to alter the coding sequence in such a way that, while the nucleic acid sequence is substantially altered, it nevertheless encodes a peptide having an amino acid sequence identical or substantially similar to the native amino acid sequence For example, codon preferences and codon usage tables for a particular species can be used to engineer isolated nucleic acid molecules that take advantage of the codon usage preferences of that particular species. Because of the degeneracy of the genetic code, alanine is encoded by the four nucleotide codon triplets: GCT, GCA, GCC, and GCG. Thus, the nucleic acid sequence of'the open reading frame can be changed at an alanine position to any of these codons without affecting the amino acid sequence of the encoded polypeptide or the characteristics of the polypeptide Based upon the degeneracy of the genetic code, nucleic acid variants can be derived from a nucleic acid sequence using standard DNA mutagenesis techniques as described herein, or by synthesis of nucleic acid sequences Thus, this disclosure also encompasses nucleic acid molecules that encode the same polypeptide but vary in nucleic acid sequence by virtue of the degeneracy of the genetic code. Therefore, the alanine 2,3-aminomutases disclosed herein can be designed to have codons that are preferentially used by a particular organism of interest (for example as described in the Examples below).

Nucleic acids encoding variants, fusions, and fragments of an alanine 2,3-aminomutase (such as those disclosed above), are encompassed by this disclosure. The disclosure also provides isolated nucleic acid sequences that encode for an alanine 2,3-aminomutase, wherein the sequence is at least 12 nucleotides in length (such as at least 13, at least 14, at least 15, at least 16, at least 17, at least 18, at least 19, at least 20, at least 25, at least 30, at least 40, at least 50, at least 60, at least 100, at least 250, at least 500, at least 750, at least 1000, at least 1200, or at least 1500 nucleotides in length) and hybridizes, under hybridization conditions, to the sense or antisense strand of a nucleic acid molecule encoding the enzyme The hybridization conditions can be moderately or highly stringent hybridization conditions,

Alanine 2,3 aminomutase peptides and nucleic acid molecules encoding such peptides can be produced by standard DNA mutagenesis techniques, for example, M13 primer mutagenesis. Details of these techniques are provided in Sambrook et al. (ed.), Molecular Cloning: A Laboratory Manual 2nd ed , vol. 1-3, Cold Spring Harbor Laboratory Press, Cold Spring, Harbor, N.Y., 1989, Ch 15 Nucleic acid molecules can contain changes of a coding region to fit the codon usage bias of the particular organism into which the molecule is to be introduced

### Cells with Alanine 2,3-Aminomutase Activity

Cells having alanine 2,3-aminomutase activity are disclosed. Such cells can produce beta-alanine from alpha-alanine. In one example, such cells have alanine 2,3-aminomutase activity due to a naturally occurring mutation or a mutation induced in the chromosome(s) of the cell, for example by exposing the cell to chemical or UV mutagenesis. Cells including alanine 2,3-aminomutase activity can be eukaryotic or prokaryotic. Examples of such cells include, but are not limited to *Lactobacillus, Lactococcus, Bacillus, Escherichia, Geobacillus, Corynebacterium, Clostridium,* fungal, plant, and yeast cells In one example, a plant cell is part of a plant, such as a transgenic plant

In one example, cells having alanine 2,3-aminomutase activity are transformed cells. Such cells can include at least one exogenous nucleic acid molecule that encodes an alanine 2,3-aminomutase, for example a sequence that includes SEQ ID NO: 18, 20, 42, 44, 46, 48, or 50, or variants, fragments, or fusions thereof that retain the ability to encode a protein having alanine 2,3-aminomutase activity. In one example, the exogenous nucleic acid molecule is a mutated lysine 2,3-aminomutase, such as a mutated prokaryotic lysine 2,3-aminomutase. In specific examples, the mutated prokaryotic lysine 2,3-aminomutase is a mutated *Bacillus subtilis, Porphyromonas gingivalis, Fusobacterium nucleatum or Clostridium sticklandii* lysine 2,3-aminomutase Other lysine 2,3-aminomutases can be identified by using methods known in the art, for example by searching for similar sequences on BLAST or by using hybridization methods In a specific example, the mutated lysine 2,3-aminomutase is a *mutated B subtilis, F. nucleatum P gingivalis, or C sticklandii* lysine 2,3-aminomutase In a particular example, the mutated lysine 2,3-aminomutase is a mutated *F*. *nucleatum* lysine 2,3-aminomutase having a substitution at position E30K; K36E; Y63F; Q86R; L95M; M128V; D144G; D179N; L228M; D331H; or K398E. In yet another example, the mutated lysine 2,3-aminomutase is a mutated C *sticklandii* lysine 2,3-aminomutase having a substitution at position P/S11T; E30K; V32A; C52R; L53P/H; E71G; Q95M; Q125L; M128V; Q141R; K87R; orD331G/H. In yet another example, the mutated lysine 2,3-aminomutase is a mutated *P. gingivalis* lysine 2,3-aminomutase having a substitution at position N19Y; L26I; E30K; L53P/H; H85Q; I192V; D331G/H; or M342T In all these examples, substitutions in a particular lysine 2,3-aminomutase can be present singly, or any combination thereof.

Cells which include alanine 2,3-aminomutase activity as well as other enzyme activities, are disclosed Such cells can be used to produce beta-alanine, 3-HP, pantothenate, CoA, and organic acids, polyols such as 1,3-propanediol, polymerized 3-HP, co-polymers of 3-HP and other compounds such as butyrates, valerates and other compounds, and esters of 3-HP

For example, cells having alanine 2,3-aminomutase activity along with pyruvate/2-oxoglutarate aminotransferase activity, beta-alanine/2-oxoglutarate aminotransferase activity, and 3-hydroxypropionate dehydrogenase activity capable or producing 3-hydroxypropionate from 3-oxopropionate are disclosed. In these examples, the cells can be used to produce 3-HP In another example, such cells further contain lipase or esterase activity. Such cells can be used to produce an ester of 3-HP, such as methyl 3-hydroxypropionate, ethyl 3-hydroxypropionate, propyl 3-hydroxypropionate, butyl 3-hydroxypropionate, or 2-ethylhexyl 3-hydroxypropionate

In another example, cells having alanine 2,3-aminomutase activity also include pyruvate/2-oxoglutarate aminotransferase activity, beta-alanine/2-oxoglutarate aminotransferase activity, and 3-hydroxypropionate dehydrogenase activity; and poly hydroxacid synthase activity. Such cells can be used to produce polymerized 3-HP.

Alternatively, cells having alanine 2,3-aminomutase activity also include pyruvate/2. oxoglutarate aminotransferase activity, beta-alanine/2-oxoglutarate aminotransferase activity, 3-hydroxypropionate dehydrogenase activity and alcohol dehydrogenase activity Such cells can be used to produce 1,3-propanediol.

In one example, cells having alanine 2,3 aminomutase activity also have alpha-ketopantoate hydroxymethyltransferase (E.C. 2.1 2.11), alpha-ketopantoate reductase (E.C. 1.1.1169), and pantothenate synthase (E.C. 63.2 1) activity Such cells can be used to produce pantothenate, Alternatively or in addition, the cells also have pantothenate kinase (EC. 2.7.1.33),4'-phosphopantethenoyl-1-cysteine synthetase (E.C. 6.3.2.5), 4'-phosphopantothenoylcysteine decarboxylase (E.C. 4.1.1 36), ATP:4`-phosphopantetheine adenyltransferase (E.C 2.7.7.3), and dephospho-CoA kinase (E C 27.1 24) activity Such cells can be used to produce coenzyme A (CoA).

The enzyme activities can be provided by expressing nucleic acid molecules that encode enzymes having the desired activity, or by supplying the enzyme directly.

### Methods to Identify Cells Having Alanine 2,3-Aminomutase Activity

A method of identifying a cell having alanine 2,3-aminomutase activity is disclosed The method includes culturing a cell, such as a prokaryotic cell, which is functionally deleted for *panD,* in media which includes alpha-alanine, but not beta-alanine or pantothenate, or in media in which the cell can produce alpha-alanine from media sources of carbon, oxygen, hydrogen, and nitrogen, but which does not include beta-alanine or pantothenate, and identifying cells capable of growing in the beta-alanine or pantothenate deficient-media. In particular examples, the cell is also functionally deleted for *panF* such that the cell is incapable of concentrative uptake of pantothenate from media supplemented with pantothenate. Growth of the cell indicates that the cell is producing beta-alanine from alpha-alanine, which indicates the cell has alanine 2,3-aminomutase activity. In contrast, if a cell does not grow or survive on the beta-alanine or pantothenate deficient-media, this indicates that the cell is not producing beta-alanine from alpha-alanine, which indicates the cell does not have alanine 2,3-aminomutase activity.

In one example, the cell functionally deleted for *panD* is transformed with one or more mutated aminomutases, such as libraries including mutated lysine 2,3-aminomutase. In a particular example, the cell is transformed with a library of mutated lysine 2,3-aminomutases, prior to culturing and screening the cells The enzyme lysine 2,3-aminomutase has been previously described from *Clostridium subterminale* SB4 (Chirpich et al, J. Biol Chem. 245:1778-89, 1970) and *Bacillus subtilis* (Chen et al., Biochem. J 348:539-49, 2000), and has been shown to catalyze the interconversion of lysine and beta-lysine Mutant aminomutase, such as a mutant lysine 2,3-aminomutase, can be screened for their ability to confer alanine 2,3-aminomutase activity. In addition, although a polypeptide having alanine 2,3-aminomutase activity has not been previously described, such an enzyme may exist in nature Ihus, a cell functionally deleted for *panD* can be transformed with a library including a gene encoding for alanine 2,3-aminomutase, and the gene isolated by its ability to confer growth to this cell in media containing alpha-alanine, or carbon, oxygen, hydrogen, and nitrogen sources such that the cell can generate alpha-alanine, but not containing beta-alanine or pantothenate.

In another example, the method further includes identifying a mutation in the mutated aminomutase(s) following identifying a cell which grows in the media, wherein the mutated aminomutase(s) confers alanine 2,3-aminomutase activity to the cell. To identify the mutation, the aminomutase nucleic acid or amino acid can be sequenced and compared to a non-mutated aminomutase sequence, to identify mutations that confer alanine 2,3-aminomutase activity to the cell These mutations can then be transferred to other lysine 2,3-aminomutases, as exemplified in FIG 7, to generate aminomutase variants with alanine 2,3-aminomutase activity.

### Methods of Producing a Peptide having Alanine 2,3-Aminomutase Activity

A method for producing alanine 2,3-aminomutase peptides having alanine 2,3-aminomutase activity, is disclosed The method includes culturing the disclosed cells having alanine 2,3-aminomutase activity under conditions that allow the cell to produce the alanine 2,3-aminomutase peptide In one example, the method includes culturing cells having one or more exogenous nucleic acid molecules which encode for an alanine 2,3-aminomutase (such as a sequence which includes SEQ ID NO: 18, 20, 42, 44, 46, 48, or 50, or variants, fusions, or fragments thereof that retain alanine 2,3-aminomutase activity), such that the alanine 2,3-aminomutase is produced.

A method for making beta-alanine from alpha-alanine is also disclosed. In one example, the method includes culturing the disclosed cells having alanine 2,3-aminomutase activity under conditions that allow the cell to produce beta-alanine from alpha-alanine. In one example, the method includes culturing cells having one or more exogenous nucleic acid molecules which encode for an alanine 2,3-aminomutase, such that the alanine 2,3-aminomutase is capable of producing beta-alanine from alpha-alanine In one example, the exogenous nucleic acid is a sequence that includes SEQ ID NO: 18, 20, 42, 44, 46, 48, or 50 or variants, fusions, or fragments thereof that retain alanine 2,3-aminomutase activity.

In particular examples, the cell is functionally deleted for *panD,* or *panD* and *panF.*

### Pathways for Producing 3-HP, Pantothenate and Derivatives Thereof

Methods and materials related to producing beta-alanine from alpha-alanine, via an alanine 2,3-aminomutase, such as using the disclosed alanine 2,3-aminomutase sequences and the disclosed cells having alanine 2,3-aminomutase activity are disclosed. In addition, methods and materials related to producing pantothenate and 3-HP from beta-alanine, as well as CoA and organic compounds such as 1,3-propanediol, polymerized 3-HP, co-polymers of 3-HP and other compounds such as butyrates, valerates and other compounds, and esters of 3-HP, are disclosed. Specifically, the disclosure provides alanine 2,3-aminomutase nucleic acid molecules (such as SEQ ID NO:18, 20, 42, 44, 46, 48, or 50), peptides (such as SEQ ID NO: 2, 4, 6, 19, 21, 43, 45, 47, 49, or 51), host cells, and methods and materials for producing beta-alanine from alpha-alanine, which can be used to more efficiently make beta-alanine, pantothenate and 3-HP as well as derivatives thereof such as CoA and organic compounds such as 1,3-propanediol, polymerized 3-HP, and esters of 3-HP.

Several metabolic pathways can be used to produce organic compounds from beta-alanine which has been produced from alpha-alanine (FIGS 1 and 3)

### Pathways of 3-HP and it Derivatives

As shown in FIG. 1, 3-HP can be made from beta-alanine by use of a polypeptide having beta-alanine/2-oxoglutarate aminotransferase activity which generates 3-oxopropionate from beta-alanine The 3-oxopropionate can be converted into 3-HP with a polypeptide having 3-HP dehydrogenase activity (EC 1.1.1 59 or 31)

Derivatives of 3-HP can be made from beta-alanine as shown in FIG. 1 The resulting 3-HP can be converted into polymerized 3-HP by a polypeptide having poly hydroxyacid synthase activity (EC 2.3.1.-). Alternatively or in addition, 3-HP can be converted into 1,3-propanediol by polypeptides having oxidoreductase activity or reductase activity.

The resulting 3-HP can be converted into an ester of 3-HP by a polypeptide having lipase or esterase activity (EC 3.1.1-). Alternatively or in addition, 1,3-propanediol can be created from 3-HP, by a combination of a polypeptide having aldehyde dehydrogenase activity and a polypeptide having alcohol dehydrogenase activity

### Pathways of Pantothenate and it Derivatives

As shown in FIG 3, pantothenate can be made from beta-alanine by a peptide having alpha-ketopantoate hydroxymethyltransferase (E.C 2.1.2.11), alpha-ketopantoate reductase (E.C 1.1.1.169), and pantothenate synthase (E.C.6.3.2.1) activities, which converts beta-alanine to pantothenate.

Derivatives of pantothenate can be made from beta-alanine as follows The resulting pantothenate can be converted into CoA by polypeptides having pantothenate kinase (E.C. 2.7.1.33), 4'-phosphopantethenoyl-1-cysteine synthetase (E.C.6.3.2.5), 4'-phosphopantothenoylcysteine decarboxylase (E.C. 4.1.1.36), AIP:4'-phosphopantetheine adenyltransferase (E.C. 2.7.7.3), and dephospho-CoA kinase (E.C. 2.7.1.24) activities.

### Enzymes

Polypeptides having lysine 2,3-aminomutase activity as well as nucleic acid encoding such polypeptides can be obtained from various species including, but not limited to: *C. subterminale, E. coli, B subtilis, P. gingivalis, F. nucleatum, and C. sticklandii* For example, amino acid sequences having lysine 2,3-aminomutase activity are shown in SEQ ID NO: 52 for *P gingivalis,* SEQ ID NO: 33 for *C. sticklandii;* SEQ ID NO: 59 for *B subtilis;* and in SEQ ID NO: 10 for *F. nucleatum.*

Nucleic acid molecules that encode peptides having alanine 2,3-aminomutase activity are disclosed herein. Examples include, but are not limited to, SEQ ID NOS: 48 and 50 *for P gingivalis* (the corresponding amino acid sequences are shown in SEQ ID NOS: 49 and 51), SEQ ID NOS: 18 and 20 for *F. nucleatum* (the corresponding amino acid sequences are shown in SEQ ID NOS: 19 and 21), and SEQ ID NOS: 42, 44, and 46 for C. *sticklandii* (the corresponding amino acid sequences are shown in SEQ ID NOS: 43,45 and 47). In addition, other peptides having alanine 2,3-aminomutase activity as well as nucleic acids encoding such peptides, can be obtained using the methods described herein. For example, alanine 2,3-aminomutase variants can encode a peptide having alanine 2,3-aminomutase activity as described above.

Polypeptides having beta-alanine/2-oxoglutarate aminotransferase activity, 3-hydroxypropionate dehydrogenase activity, as well as nucleic acid encoding such polypeptides can be obtained from various species.

Polypeptides having poly hydroxyacid synthase activity as well as nucleic acid encoding such polypeptides can be obtained from various species including, without limitation, *Rhodobacter sphaeroides, Comamonas acidororans, Ralstonia eutropha,* and *Pseudomonas oleovorans.* For example, nucleic acid that encodes a polypeptide having poly hydroxyacid synthase activity can be obtained *from R sphaeroides* and can have a sequence as set forth in GenBank accession number X97200 Addition information about poly hydroxyacid synthase can be found in Song et al (Biomacromolecules 1:433-9, 2000),

Aldehyde:NAD(+) oxidoreductase activity and alcohol:NAD(+) oxidoreductase activities can be carried out by two different polypeptides as described above, or carried out by a single polypeptide, such as a multi-functional aldehyde-alcohol dehydrogenase (EC 1.2.1.10) from *E coli* (Goodlove et al. Gene 85:209-14, 1989; GenBank Accession No M33504).

Polypeptides having aldehyde dehydrogenase (NAD(P)+) (EC 1.2.1 -) activity as well as nucleic acid encoding such polypeptides can be obtained from various species including, without *limitation, S. cerevisiae.* For example, nucleic acid that encodes a polypeptide having aldehyde dehydrogenase activity can be obtained from *S cerevisiae* and can have a sequence as set forth in GenBank Accession No. 275282 (Tessier et al. FEMS Microbiol Lett 164:29-34, 1998)

Polypeptides having alcohol dehydrogenase activity (EC1.1.1.1) as well as nucleic acid encoding such polypeptides can be obtained from various species including, without limitation, Z. *mobilis.* For example, nucleic acid that encodes a polypeptide having alcohol dehydrogenase activity can be obtained from Z. *mobilis* and can have a sequence as set forth in GenBank accession No M32100

Polypeptides having lipase activity as well as nucleic acid encoding such polypeptides can be obtained from various species including, without limitation, *candida rugosa, Candida tropicalis,* and *Candida albicans.* For example, nucleic acid that encodes a polypeptide having lipase activity can be obtained from C *rugosa* and can have a sequence as set forth in GenBank accession number A81171.

Polypeptides having alpha-ketopantoate hydroxymethyltransferase and pantothenate synthase activity as well as nucleic acid encoding such polypeptides can be obtained from various species including, without limitation, *E. coli.* For example, nucleic acid molecules that encode peptides having alpha-ketopantoate hydroxymethyltransferase and pantothenate synthase activity can be obtained from *E*. *coli* and can have a sequence as set forth in GenBank accession number L17086.

Polypeptides having alpha-ketopantoate reductase, pantothenate kinase, 4'-phosphopantethenoyl-1-cysteine synthetase, 4'-phosphopantothenoylcysteine decarboxylase, ATP:4'-phosphopantetheine adenyltransferase, and dephospho-CoA kinase activity as well as nucleic acid encoding such polypeptides can be obtained from various species including, without limitation, *E. coli.* For example, nucleic acids that encodes polypeptides having alpha-ketopantoate reductase pantothenate kinase, 4'-phosphopantethenoyl-1-cysteine synthetase, 4'-phosphopantothenoylcysteine decarboxylase, AIP:4'-phosphapantetheine adenyltransferase, and dephospho-CoA kinase activity can be obtained from *E. coli* and can have a sequence as set forth in GenBank accession number NC000913.

The term "polypeptide having enzymatic activity" refers to any polypeptide that catalyzes a chemical reaction of other substances without itself being destroyed or altered upon completion of the reaction. Typically, a polypeptide having enzymatic activity catalyzes the formation of one or more products from one or more substrates. Such polypeptides can have any type of enzymatic activity including, without limitation, the enzymatic activity or enzymatic activities associated with enzymes such as alanine 2,3-aminomutase, poly hydroxyacid synthases, beta-alanine/2-oxoglutarate aminotransferases, 3-hydroxypropionate dehydrogenases, lipases, esterases, acetylating aldehyde:NAD(+) oxidoreductases, alcohol:NAD(+) oxidoreductases, aldehyde dehydrogenases, alcohol dehydrogenases, synthase, synthetases, decarboxylases, alpha-ketopantoate hydroxymethyltransferases, alpha-ketopantoate reductases, pantothenate synthases, pantothenate kinases, 4'-phosphopantethenoyl-1-cysteine synthetase, 4'-phosphopantothenoylcysteine decarboxylases, AIP:4'-phosphopantetheine adenyltransferases, dephospho-CoA kinases, acetylating aldehyde:NAD(+) oxidoreductases, alcohol:NAD(+) oxidoreductases, aldehyde dehydrogenases (NAD(P)+), and alcohol dehydrogenases,

### Methods of Making 3-HP, Pantothenate, and Derivatives Thereof

Each step provided in the pathways depicted in FIGS. 1 and 3 can be performed within a cell (*in vivo)* or outside a cell (*in vitro,* such as in a container or column) Additionally, the organic compound products can be generated through a combination of *in vivo* synthesis and *in vitro* synthesis. Moreover, the *in vitro* synthesis step, or steps, can be via chemical reaction or enzymatic reaction.

For example, a cell or microorganism provided herein can be used to perform the steps provided in FIGS. 1 and 3, or an extract containing polypeptides having the indicated enzymatic activities can be used to perform the steps provided in FIGS. 1 and 3 In addition, chemical treatments can be used to perform the conversions provided in FIGS. 1 and 3 For example, 3-oxopropionate can be converted into 3-HP by chemical hydrogenation. Other chemical treatments include, without limitation, trans esterification to convert 3-HP into a 3-HP ester

### Expression of Polypeptides

The peptides described herein, such as the enzymes listed in FIG. 1, can be produced individually in a host cell or in combination in a host cell. Moreover, the peptides having a particular enzymatic activity can be a peptide that is either naturally-occurring or non-naturally-occurring. A naturally-occurring peptide is any peptide having an amino acid sequence as found in nature, including wild-type and polymorphic polypeptides Naturally-occurring peptides can be obtained from any species including, but not limited to, animal (such as mammalian), plant, fungal, and bacterial species A non-naturally-occurring polypeptide is any polypeptide having an amino acid sequence not found in nature. Thus, a non-naturally-occurring polypeptide can be a mutated version of a naturally-occurring polypeptide, or an engineered polypeptide For example, a non-naturally-occurring polypeptide having alanine 2,3-aminomutase activity can be a mutated version of a naturally-occurring polypeptide having lysine 2,3-aminomutase activity that has at least some alanine 2,3-aminomutase activity (such as SEQ ID NO: 19, 21, 43, 45, 47, 49 or 51). A peptide can be mutated by, for example, sequence additions, deletions, substitutions, or combinations thereof using methods known in the art.

Genetically modified cells can be used to perform one or more steps of the steps in the pathways described herein or the genetically modified cells can be used to produce the disclosed polypeptides for subsequent use *in vitro* For example, an individual microorganism can contain exogenous nucleic acid(s) encoding each peptide needed to perform the steps depicted in FIGS 1 and 3. Such cells can contain any number of exogenous nucleic acid molecules For example, a particular cell can contain one, two, three, or four different exogenous nucleic acid molecules with each one encoding the polypeptide(s) necessary to convert pyruvate into 3-HP as shown in FIG. 1, or a particular cell can endogenously produce polypeptides necessary to convert pyruvate into alpha-alanine while containing exogenous nucleic acid that encodes polypeptides necessary to convert alpha-alanine into 3-HP.

In addition, a single exogenous nucleic acid molecule can encode one, or more than one, polypeptide. For example, a single exogenous nucleic acid molecule can contain sequences that encode two, three, on even four different polypeptide Further, the cells described herein can contain a single copy, or multiple copies (such as about 5, 10, 20, 35, 50, 75, 100 or 150 copies), of a particular exogenous nucleic acid molecule, such as a particular enzyme. The cells described herein can contain more than one particular exogenous nucleic acid For example, a particular cell can contain about 50 copies of exogenous nucleic acid molecule X as well as about 75 copies of exogenous nucleic acid molecule Y.

In another example, a cell can contain an exogenous nucleic acid molecule that encodes a polypeptide having alanine 2,3-aminomutase activity, for example SEQ ID NO: 18,20,42,44,46,48, or 50. Such cells can have any detectable level of alanine 2,3-aminomutase activity, including activity detected by the production of metabolites of beta-alanine, such as pantothenate. For example, a cell containing an exogenous nucleic acid molecule that encodes a polypeptide having alanine 2,3-aminomutase activity can have alanine 2,3-aminomutase activity with a specific activity greater than about 1 µg beta-alanine formed per gram dry cell weight per hour (such as greater than about 10, 20, 30, 40, 50, 60, 70, 80, 90, 100, 125, 150, 200, 250, 300, 350, 400, 500, or more µg beta-alanine formed per gram dry cell weight per hour). Alternatively, a cell can have alanine 2,3-aminomutase activity such that a cell extract has a specific activity greater than about 1 ng beta-alanine formed per mg total protein per minute (such as greater than about 10, 20, 30, 40, 50, 60, 70, 80, 90, 100, 125, 150, 200, 250, 300, 350, 400, 500, or more ng beta-alanine formed per mg total protein per minute).

A nucleic acid molecule encoding a polypeptide having enzymatic activity can be identified and obtained using any method such as those described herein. For example, nucleic acid molecules that encode a polypeptide having enzymatic activity can be identified and obtained using common molecular cloning or chemical nucleic acid synthesis procedures and techniques, including PCR. In addition, standard nucleic acid sequencing techniques and software programs that translate nucleic acid sequences into amino acid sequences based on the genetic code can be used to determine whether or not a particular nucleic acid has any sequence homology with known enzymatic polypeptides Sequence alignment software such as MEGALIGN (DNASTAR, Madison, WI, 1997) can be used to compare various sequences.

In addition, nucleic acid molecules encoding known enzymatic polypeptides can be mutated using common molecular cloning techniques (such as site-directed mutagenesis). Possible mutations include, without limitation, deletions, insertions, and substitutions, as well as combinations of deletions, insertions, and nucleotide substitutions Further, nucleic acid and amino acid databases (such as, GenBank or EMBL) can be used to identify a nucleic acid sequence that encodes a polypeptide having enzymatic activity, Briefly, any amino acid sequence having some homology to a polypeptide having enzymatic activity, or any nucleic acid sequence having some homology to a sequence encoding a polypeptide having enzymatic activity can be used as a query to search GenBank. The identified polypeptides then can be analyzed to determine whether or not they exhibit enzymatic activity.

In addition, nucleic acid hybridization techniques can be used to identify and obtain a nucleic acid molecule that encodes a polypeptide having enzymatic activity. Briefly, any nucleic acid molecule that encodes a known enzymatic polypeptide, or fragment thereof, can be used as a probe to identify similar nucleic acid molecules by hybridization under conditions of moderate to high stringency. Such similar nucleic acid molecules then can be isolated, sequenced, and analyzed to determine whether the encoded polypeptide has enzymatic activity.

Expression cloning techniques also can be used to identify and obtain a nucleic acid molecule that encodes a polypeptide having enzymatic activity For example, a substrate known to interact with a particular enzymatic polypeptide can be used to screen a phage display library containing that enzymatic polypeptide. Phage display libraries can be generated as described (Burritt et al., Anal Biochem 238:1-13, 1990), or can be obtained from commercial suppliers such as Novagen (Madison, WI).

**Further**, polypeptide sequencing techniques can be used to identify and obtain a nucleic acid molecule that encodes a polypeptide having enzymatic activity. For example, a purified polypeptide can be separated by gel electrophoresis, and its amino acid sequence determined by, for example, amino acid microsequencing techniques. Once determined, the amino acid sequence can be used to design degenerate oligonucleotide primers Degenerate oligonucleotide primers can be used to obtain the nucleic acid encoding the polypeptide by PCR. Once obtained, the nucleic acid can be sequenced, cloned into an appropriate expression vector, and introduced into a microorganism

Any method can be used to introduce an exogenous nucleic acid molecule into a cell. For example, heat shock, lipofection, electroporation, conjugation, fusion of protoplasts, and biolistic delivery are common methods for introducing nucleic acid into bacteria and yeast cells (for example see Ito et al., J. Bacterol. 153:163-8, 1983; Durrens et al., Curr. Genet 18:7-12, 1990; Sambrook et al., Molecular cloning: A laboratory manual, Cold Spring Harbour Laboratory Press, New York, USA, second edition, 1989; and Becker and Guarente, Methods in Enzymology 194:182-7, 1991). Other methods for expressing an amino acid sequence from an exogenous nucleic acid molecule include, but are not limited to, constructing a nucleic acid such that a regulatory element promotes the expression of a nucleic acid sequence that encodes a polypeptide Typically, regulatory elements are DNA sequences that regulate the expression of other DNA sequences at the level of transcription. Thus, regulatory elements include, without limitation, promoters, enhancers, and the like. Any type of promoter can be used to express an amino acid sequence from an exogenous nucleic acid molecule Examples of promoters include, without limitation, constitutive promoters, tissue-specific promoters, and promoters responsive or unresponsive to a particular stimulus (such as light, oxygen, chemical concentration). Methods for transferring nucleic acids into mammalian cells are also known, such as using viral vectors.

An exogenous nucleic acid molecule contained within a particular cell of the disclosure can be maintained within that cell in any form. For example, exogenous nucleic acid molecules can be integrated into the genome of the cell or maintained in an episomal state. That is, a cell can be a stable or transient transformant. A microorganism can contain single or multiple copies (such as about 5, 10, 20, 35, 50, 75, 100 or 150 copies), of a particular exogenous nucleic acid molecule, such as a nucleic acid encoding an enzyme

### Production of Organic Acids and Related Products via Host Cells

The nucleic acid and amino acid sequences provided herein can be used with cells to produce beta-alanine, pantothenate and 3-HP, as well as derivatives thereof such as CoA, and organic compounds such as 1,3-propanediol, esters of 3-HP, and polymerized 3-HP Such cells can be from any species, such as those listed within the taxonomy web pages at the National Institutes of Health. The cells can be eukaryotic or prokaryotic For example, genetically modified cells can be mammalian cells (such as human, murine, and bovine cells), plant cells (such as corn, wheat, rice, and soybean cells), fungal cells (such as *Aspergillus* and *Rhizopus* cells), yeast cells, or bacterial cells (such as *Lactobacillus, Lactococcus, Bacillus, Escherichia,* and *Clostridium* cells). In one example, a cell is a microorganism. The term "microorganism" refers to any microscopic organism including, but not limited to, bacteria, algae, fungi, and protozoa. Thus, *E*. *coli, B. subtilis, B. licheniformis, S. cerevisiae, Kluvet-omyces lactis, Candida blankii, Candida rugosa,* and *Pichia pastoris* are exemplary microorganisms and can be used as described herein. In another example, the cell is part of a larger organisim, such as a plant, such as a transgenic plant. Examples of plants that can be used to make 3-HP, pantothenate, or other organic compounds from beta-alanine include, but are not limited to, genetically engineered plant crops such as corn, rice, wheat, and soybean.

In one example, a cell is genetically modified such that a particular organic compound is produced. In one embodiment, cells make 3-HP or pantothenate from beta-alanine, such as the pathways shown in FIGS. 1 and 3. In another example, the cells make derivatives of 3-HP or pantothenate, such as CoA, and organic compounds such as 1,3-propanediol, esters of 3-HP, and polymerized 3-HP.

Cells that are genetically modified to synthesize a particular organic compound can include one or more exogenous nucleic acid molecules that encode polypeptides having specific enzymatic activities For example, a microorganism can contain exogenous nucleic acid that encodes a polypeptide having 3-beta-alanine/2-oxoglutarate aminotransferase activity. In this case, beta-alanine can be converted into 2-oxopropionate which can lead to the production of 3-HP A cell can be given an exogenous nucleic acid molecule that encodes a polypeptide having an enzymatic activity that catalyzes the production of a compound not normally produced by that cell. Alternatively, a cell can be given an exogenous nucleic acid molecule that encodes a polypeptide having an enzymatic activity that catalyzes the production of a compound that is normally produced by that cell In this case, the genetically modified cell can produce more of the compound, or can produce the compound more efficiently, than a similar cell not having the genetic modification.

In another example, a cell containing an exogenous nucleic acid molecule that encodes a polypeptide having enzymatic activity that leads to the formation of 3-HP, pantothenate, or derivatives thereof, is disclosed The produced product(s) can be secreted from the cell, eliminating the need to disrupt cell membranes to retrieve the organic compound in one example, the cell produces 3-HP, pantothenate, or derivatives thereof, with the concentration of the product(s) being at least about 100 mg per L (such as at least about 1 g/L, 5 g/L, 10 g/L, 25 g/L, 50 g/L, 75 g/L, 80 g/L, 90 g/L, 100 g/L, or 120 g/L). When determining the yield of a compound such as 3-HP, pantothenate, and/or derivatives thereof for a particular cell, any method can be used (for example see Applied Environmental Microbiology 59(12):4261-5, 1993). A cell within the scope of the disclosure can utilize a variety of carbon sources.

A cell can contain one or more exogenous nucleic acid molecules that encodes a polypeptide(s) having enzymatic activity that leads to the formation of 3-HP, pantothenate, or derivatives thereof, such as CoA, 1,3-propanediol, 3-HP-esters, and polymers and copolymers containing 3-HP. Methods of' identifying cells that contain exogenous nucleic acid molecules are well known Such methods include, without limitation, PCR and nucleic acid hybridization techniques such as Northern and Southern analysis (see hybridization described herein). Immunohisto-chemical and biochemical techniques can also be used to determine if a cell contains particular nucleic acid molecule by detecting the expression of the peptide encoded by that particular nucleic acid molecule For example, an antibody having specificity for a polypeptide can be used to determine whether or not a particular cell contains nucleic acid encoding that polypeptide Further, biochemical techniques can be used to determine if a cell contains a particular nucleic acid molecule encoding a polypeptide having enzymatic activity by detecting an organic product produced as a result of the expression of the polypeptide having enzymatic activity. For example, detection of 3-HP after introduction of exogenous nucleic acid that encodes a polypeptide having 3-hydroxypropioniate dehydrogenase activity into a cell that does not normally express such a polypeptide can indicate that the cell not only contains the introduced exogenous nucleic acid molecule but also expresses the encoded polypeptide from that introduced exogenous nucleic acid molecule. Methods for detecting specific enzymatic activities or the presence of particular organic products are well known, for example, the presence of an organic compound such as 3-HP can be determined as described in Sullivan and Clarke (J. Assoc. Offic. Agr Chemists, 38:514-8, 1955).

### Cells with Reduced Polypeptide Activity

Genetically modified cells having reduced polypeptide activity are disclosed The term "reduced" or "decreased" as used herein with respect to a cell and a particular polypeptide's activity refers to a lower level of activity than that measured in a comparable cell of the same species. For example, a particular microorganism lacking enzymatic activity X has reduced enzymatic activity X if a comparable microorganism has at least some enzymatic activity X

A cell can have the activity of any type of polypeptide reduced including, without limitation, enzymes, transcription factors, transporters, receptors, signal molecules, and the like For example, a cell can contain an exogenous nucleic acid molecule that disrupts a regulatory or coding sequence of a polypeptide having *panD* activity Disrupting *panD* can prevent a cell from making beta-alanine.

Reduced polypeptide activities can be the result of lower polypeptide concentration, lower specific activity of a polypeptide, or combinations thereof. Many different methods can be used to make a cell having reduced polypeptide activity. For example, a cell can be engineered to have a disrupted regulatory sequence or polypeptide-encoding sequence using common mutagenesis or knock-out technology. (Methods in Yeast Genetics (1997 edition), Adams, Gottschling, Kaiser, and Stems, Cold Spring Harbor Press, 1998; Datsenko and Wanner, Proc. Natl Acad Sci USA 97: 6640-5, 2000). Alternatively, antisense technology can be used to reduce the activity of a particular polypeptide For example, a cell can be engineered to contain a cDNA that encodes an antisense molecule that prevents a polypeptide from being translated. The term "antisense molecule" encompasses any nucleic acid molecule or nucleic acid analog (such as peptide nucleic acids) that contains a sequence that corresponds to the coding strand of an endogenous polypeptide, An antisense molecule also can have flanking sequences (such as regulatory sequences). Thus, antisense molecules can be ribozymes or antisense oligonucleotides A ribozyme can have any general structure including, without limitation, hairpin, hammerhead, or axhead structures, provided the molecule cleaves RNA. Further, gene silencing can be used to reduce the activity of a particular polypeptide.

A cell having reduced activity of a polypeptide can be identified using any method For example, enzyme activity assays such as those described herein can be used to identify cells having a reduced enzyme activity.

### Production of Organic Acids and Related Products via In Vitro Techniques

Purified polypeptides having enzymatic activity can be used alone or in combination with cells to produce pantothenate, 3-HP, or derivatives thereof such as CoA, and organic compounds such as 1,3-propanediol, esters of 3-HP, and polymerized 3-HP For example, a preparation including a substantially pure polypeptide having 3-hydroxypropionate dehydrogenase activity can be used to catalyze the formation of 3-HP

Further, cell-free extracts containing a polypeptide having enzymatic activity can be used alone or in combination with purified polypeptides or cells to produce pantothenate, 3-HP, or derivatives thereof For example, a cell-free extract which includes a polypeptide having alanine 2,3-aminomutase activity can be used to form beta-alanine from alpha-alanine, while a microorganism containing polypeptides which have the enzymatic activities necessary to catalyze the reactions needed to form 3-HP from beta-alanine can be used to produce 3-HP In another example, a cell-free extract which includes alpha-ketopantoate hydroxymethyltransferase (E.C. 2.1.2.11), alpha-ketopantoate reductase (E.C. 1.1.1.169), and pantothenate synthase (E.C. 6.3.2.1) can be used to form pantothenate from beta-alanine. Any method can be used to produce a cell-free extract For example, osmotic shock, sonication, or a repeated freeze-thaw cycle followed by filtration and/or centrifugation can be used to produce a cell-free extract from intact cells.

A cell, purified polypeptide, or cell-tree extract can be used to produce 3-HP that is, in turn, treated chemically to produce another compound. For example, a microorganism can be used to produce 3-HP, while a chemical process is used to modify 3-HP into a derivative such as polymerized 3-HP or an ester of 3-HP. Likewise, a chemical process can be used to produce a particular compound that is, in turn, converted into 3-HP or other organic compound (such as 1,3-propanediol, acrylic acid, polymerized acrylate, esters of acrylate, esters of 3-HP, and polymerized 3-HP) using a cell, substantially pure polypeptide or cell free extract described herein For example, a chemical process can be used to produce acrylyl-CoA, while a microorganism can be used convert acrylyl-CoA into 3-HP

Similarly, a cell, purified polypeptide, or cell-free extract can be used to produce pantothenate that is, in turn, treated chemically to produce another compound For example, a microorganism can be used to produce pantothenate, while a chemical process is used to modify pantothenate into a derivative such as CoA. Likewise, a chemical process can be used to produce a particular compound that is, in turn, converted into pantothenate or other compound (such as CoA) using a cell, substantially pure polypeptide, or cell-free extract described herein For example, a chemical process can be used to produce pantothenate, while a microorganism can be used convert pantothenic acid into CoA

### Fermentation of Cells to Produce Organic Acids

A method for producing pantothenate, 3-HP, or derivatives thereof by culturing a production cells, such as a microorganism, in culture medium such that pantothenate, 3-HP, or derivatives thereof, is produced, is disclosed. In general, the culture media or culture conditions can be such that the microorganisms grow to an adequate density and produce the product efficiently. For large-scale production processes, any method can be used such as those described elsewhere (Manual of Industrial Microbiology and Biotechnology, 2nd Edition, Editors: Demain and Davies, ASM Press; and Principles of Fermentation Technology, Stanbury and Whitaker, Pergamon).

Briefly, a large tank (such as a 100 gallon, 200 gallon, 500 gallon, or more tank) containing appropriate culture medium with, for example, a glucose carbon source is inoculated with a particular microorganism After inoculation, the microorganisms are incubated to allow biomass to be produced Once a desired biomass is reached, the broth containing the microorganisms can be transferred to a second tank This second tank can be any size. For example, the second tank can be larger, smaller, or the same size as the first tank Typically, the second tank is larger than the first such that additional culture medium can be added to the broth from the first tank In addition, the culture medium within this second tank can be the same as, or different from, that used in the first tank For example, the first tank can contain medium with xylose, while the second tank contains medium with glucose.

Once transferred, the microorganisms can be incubated to allow for the production of pantothenate, 3-HP, or derivatives thereof. Once produced, any method can be used to isolate the formed product. For example, common separation techniques can be used to remove the biomass from the broth, and common isolation procedures (such as extraction, distillation, and ion-exchange procedures) can be used to obtain the pantothenate, 3-HP, or derivatives thereof from the microorganism-free broth. Alternatively, the product can be isolated while it is being produced, or it can be isolated from the broth after the product production phase has been terminated

### Products Created From the Disclosed Biosynthetic Routes

The compounds produced from any of the steps provided in FIGS 1 and 3 can be chemically converted into other organic compounds For example, 3-HP can be hydrogenated to form 1,3-propanediol, a valuable polyester monomer Hydrogenating an organic acid such as 3-HP can be performed using any method such as those used to hydrogenate succinic acid or lactic acid For example, 3-HP can be hydrogenated using a metal catalyst In another example, 3-HP can be dehydrated to form acrylic acid. Any method can be used to perform a dehydration reaction. For example, 3-HP can be heated in the presence of a catalyst (such as a metal or mineral acid catalyst) to form acrylic acid 1,3-propanediol also can be created using polypeptides having oxidoreductase activity (such as enzymes in the 1.1.1.- class of enzymes) *in vitro* or *in vivo.*

In another example, pantothenate can be used to form coenzyme A. Polypeptides having pantothenate kinase (E C 2.7 1.33), 4'-phosphopantethenoyl-1-cysteine synthetase (EC 6.3.2.5), 4'-phosphopantothenoylcysteine decarboxylase (E.C 4.1.1.36), ATP:4'-phasphopantetheine adenyltransferase (E.C 2.7.7.3), and dephospho-CoA kinase (E.C 2.7 1.24) activities can be used to produce coenzyme A.

### Production of 1,3-propanediol

Methods of producing 1,3-propanediol, and cells for such production, are disclosed. 1,3-propanediol can be generated from either 3-HP-CoA or 3-HP. Cells or microorganisms producing 3-HP-CoA or 3-HP can be engineered to make 1,3-propanediol by cloning genes which encode for enzymes having oxidoreductase/dehydrogenase type activity.

For example, 3-HP-CoA can be converted to 1,3-propanediol in the presence of an enzyme having acetylating aldehyde:NAD(+) oxidoreductase and alcohol:NAD(+) oxidoreductase activities. Such conversion can be performed *in vivo, in vitro,* or a combination thereof. These activities can be carried out by a single polypeptide or by two different polypeptides. Single enzymes include the multi-functional aldehyde-alcohol dehydrogenase (EC 1.2.1.10) from *E. coli* (Goodlove et al Gene 85:209-14, 1989; GenBank Accession No. M33504). Enzymes having a singular activity of acetylating aldehyde:NAD(+) oxidoreductase (EC 12 110) or alcohol:NAD(+) oxidoreductase (EC 1.1.1.1) have been described Genes encoding for acylating aldehyde dehydrogenase from *E. coli* (GenBank Accession No Y09555) and alcohol dehydrogenase from Z *mobilis* (GenBank Accession No M32100) have been isolated and sequenced. The genes encoding for these enzymes can be cloned into a 3-HP-CoA producing organism or cell by well-known molecular biology techniques. Expression of these enzymes in 3-HP-CoA producing organisms or cells will impart it the ability to convert 3-HP-CoA to 1,3-propanediol The substrate specificity of these enzymes for 3-HP-CoA can be changed or improved using well-known techniques such as error prone PCR or mutator *E. coli* strains

Conversion of 3-HP to 1,3-propanediol can be achieved by contacting 3-HP with enzymes having aldehyde dehydrogenase (NAD(P)+) (EC 1 2.1 -) and alcohol dehydrogenase (EC 1.1.1.1) activity Such conversion can be performed *in vivo, in vitro,* or a combination thereof. For example, cloning and expressing these genes in a 3-HP producing microorganism or cell will impart the ability of the cell or organism to convert 3-HP to 1,3-propanediol. The substrate specificity of these enzymes for 3-HP can be changed or improved using well-known techniques as described above

The formation of 1,3-propanediol during fermentation or in an *in vitro* assay can be analyzed using a High Performance Liquid Chromatography (HPLC) The chromatographic separation can be achieved by using a Bio-Rad 87H ion-exchange column. A mobile phase of 0.01N sulfuric acid is passed at a flow rate of 0 6 ml/min and the column maintained at a temperature of 45-65°C. The presence of 1,3-propanediol in the sample can be detected using a refractive index detector (Skraly et al., Appl Environ. Microbiol. 64:98-105, 1998).

### EXAMPLE 1

### Cloning and codon improvement of a Fusobacterium nucleatum lysine 2,3-aminomutase (kam gene)

This example describes methods used to clone a lysine 2,3-aminomutase (E.C. 5.4.3.2) from *F. nucleatum,* and then optimize the codon usage for expression of the gene in *E coli.* One skilled in the art will understand that similar methods can be used to clone a lysine 2,3-aminomutase from any desired organism.

The *F. nucleatum* lysine 2,3-aminomutase has a high specific activity on lysine *(*Barker et al. J Bacteriol. 152(1):201-7, 1982). *F. nucleatum* can utilize lysine as the sole carbon and nitrogen source and thus requires enzymes in the lysine degradation pathway to have high activity. To clone a *F. nucleatum kam* gene encoding lysine 2,3-aminomutase, the following methods were used. *F. nucleatum* subsp *nucleatum* (American Type Culture Collection, Manassas, VA, catalog number ATCC 25586) was propagated in ATCC medium 1053 (Reinforced Clostridial medium) Media was made anaerobic by bubbling anaerobic gas through the media in a Coy anaerobic chamber (85% N2, 5% CO2, 10% H2) The culture was incubated at 37°C in sealed anaerobic tubes Ten mL of culture were harvested and genomic DNA was isolated using the protocol of Mekalanos (Cell 35(1):253-63, 1983)

Primers to amplify the *kam* gene by PCR were based on the complete *F*. *nucleatum* genome sequence (GenBank Accession No: NC 003454), with restriction sites added to allow cloning of the PCR product into plasmids. The PCR primers: CCGGCCCATATGAATACAGTTAATACTAG (SEX 1D NO: 7) and CGCCGCQGATCCTTATTTAAACAATCTCTCCCTGTCG (SEQ ID NO: 8) were used to clone into NdeI and BamHI sites in the vector pET11A (Novagen). The cloned *F*. *nucleatum kam* gene is shown in SEQ ID NO: 9 (with the corresponding amino acid sequence shown in SEQ ID NO: 10).

*The F. nucleatum kam* gene was partially codon optimized for improved expression in *E*. *coli.* Rare arginine codons were replaced by incorporation of primers containing more preferred codons for arginine during amplification reactions Two rounds of primer incorporation resulted in clones with varying codons having been replaced Primers designed to both strands of DNA were 23 to 33 nucleotides in length with one or two codon replacements centered in the primer. The arginine codons AGG, AGA, and CGA were changed to CGT or CGC. To maximize codon optimization, overlapping fragments of the *kam* gene were amplified using template from three different clones and a proofreading DNA polymerase to minimize amplification errors. The fragments were gel purified and used as template in a second round amplification with primers homologous to the beginning and end of the gene.

In a second round of assembly, three overlapping fragments of the most optimized clone were amplified to insure incorporation of the primers used as amplification primers. The fragments were gel purified and used as template in a second round amplification with primers homologous to the beginning and end of the gene. Using restriction sites designed into these primers, the product was cloned into the pET 11A vector (Novagen) and pPRO-Nde. Plasmid pPRO-Nde is a derivative of pPROLar A122 (Clontech Laboratories, Inc., Palo Alto, CA) in which an *Nde*I site was constructed at the intiator ATG codon by oligonucleotide-directed mutagenesis using the QuikChange Site-Directed Mutagenesis kit from Stratagene. The rare arginine codons were reduced from 28 in the wildtype gene to 8 in the assembled clone The partially optimized *F nucleatum kam* gene is shown in SEQ ID NO: 11 (with the corresponding unchanged amino acid sequence shown in SEQ ID NO: 12).

One skilled in the art will appreciate that other codon optimization methods can be used, such as gene assembly using overlapping primers or Multi Site-Directed Mutagenesis (Stratagene)

### EXAMPLE 2

### in vitro mutagenesis of an F. nucleatum kam gene

This example describes methods used to mutagenize the partially optimized *F. nucleatum kam* gene (SEQ ID NO: 11) described in Example 1, to identify mutant lysine 2,3-aminomutase sequences having alanine 2,3-aminomutase activity.

Three mutations obtained previously in a *Bacillus subtilis* aminomutase (see WO 03/062173 and SEQ ID NOS: 1-4) were transferred by directed mutagenesis to their homologous position in the *F. nucleatum* codon-optimized *kam* gene These mutations included L96M, M129V, and D332H substitutions in SEQ ID NO: 12 (which resulted in the sequence shown in SEQ ID NO: 14), where the first amino acid is the wild-type sequence, the number is the amino acid position, and the second amino acid is the residue observed in the alanine 2,3-aminomutase. The mutations were made using a Stratagene QuikChange^{®} Multi Site-Directed Mutagenesis Kit (Stratagene). The primers used to make these mutations were: FnL96M
5'Phos/CATCAATCTGATGCTGATATGTTGGATCCTCTACATGAAG (SEQ ID NO: 15); FnM129V 5'Phos/AACAGACATGTGTTCTGTATACTGTCGCCACTGCACTC (SEQ ID NO: 16); and FnD332H 5'Phos/GTACCAACATTTGTTGTGCATGCACCTGGTGGTG (SEQ ID NO: 17). The sequence for the partially codon-optimized *F. nucleatum kam* gene with the three directed mutations (Fncodm) is shown in SEQ ID NO 13 (and the resulting protein sequence in SEQ ID NO: 14).

The resulting Fncodm clone was tested in a liquid growth test Resuspended colonies were used to inoculate 1.4 mL of M9 minimal media, both with (25 µM) and without pantothenate, in a 2 mL glass tube Media was supplemented with 0.4% glucose, 2 mg/mL L-alanine, 100 µM IPIG, 50 µM Fe(NH₄)₂(SO₄)₂, trace elements, and 25 µg/mL kanamycin. Culture ODs were read when the pantothenate controls reached OD₆₀₀s of approximately 0.7 Performance was measured by comparing growth without pantothenate to growth with pantothenate, and Fncodm was found to have little or no alanine 2,3-aminomutase activity.

To identify mutations that increase alanine 2,3 aminomutase activity, random mutations were introduced into the Fncodm gene (SEQ ID NO: 13) *in vitro* using an error-prone PCR method. Similar methods can be used to introduce mutations into any *kam* gene encoding a lysine 2,3-aminomutase, such as a *kam* gene from *Deinococcus radiodurans* (GenBank Accession No: RDR02336), *Clostridium subterminale* (GenBank Accession No: AF159146), or *Porphyromonas gingivalis* (Incomplete genome, The Institute for Genomic Research).

Mutagenic PCR was conducted based on the protocol of Cadwell and Joyce (PCR Methods Appl. 2:28-33, 1992) This method uses various dilutions of mutagenic buffer containing 212 mM MgCl₂, 2.0 mM MnCl₂, 3.2 mM dTTP, and 3 2 mM dCTP. 6 25 and 9.38 µL of mutagenic buffer were added to separate PCR reactions (each of final volume 100 µL), in addition to 1X Taq PCR buffer with 1.5 mM MgCl₂ 0 25 µM of forward and reverse vector primers, 200 µM of each dNTP, 5 ng of pPRO-Fncodm template DNA, and 10 units of Taq DNA polymerase (Roche). The PCR program consisted of an initial denaturation at 94°C for 2 minutes; 30 cycles of 94°C for 30 seconds, 50°C for 1 minute, and 72°C for 2.25 minutes; and a final extension at 72°C for 7 minutes.

Following PCR, the PCR product was digested with restriction enzymes *NotI* and *NdeI.* Equal amounts of DNA from each treatment were ligated into the vector pPRO-Nde, and transformed into *E. coli* Electromax^{™} DH10B^{™} cells. Plasmid DNA was isolated from single colonies and sequenced to obtain an estimate of the mutation rate (0.57%) Multiple transformations were plated on LB media containing 25 µg/mL kanamycin (LBK25) to obtain approximately 53,000 colonies Colonies were scrapped from plates and plasmid DNA prepared using a Qiagen MiniSpin Plasmid procedure Plasmid DNA was precipitated with ammonium acetate and ethanol to increase its concentration before transformation into selection hosts

### EXAMPLE 3

### Identification of Clones having Alanine 2,3-Aminomutase Activity

This example describes methods used to identify mutated lysine 2,3, aminomutase clones that have alanine 2,3 aminomutase activity

A method of identifying a cell having alanine 2,3-aminomutase activity has been previously disclosed (see WO 03/062173, herein incorporated by reference in its entirety). Briefly, the method includes culturing a cell functionally deleted for *panD* in media that does not include beta-alanine or pantothenate. The *panD* gene encodes for aspartate decarboxylase, which catalyzes the production of beta-alanine from aspartate. This functional deletion of *panD* in *E coli* inactivates aspartate decarboxylase which results in growth inhibition of the *E. coli* due to the requirement for beta-alanine in Coenzyme A production. A cell that is capable of growing in media lacking beta-alanine and pantothenate indicates that it is producing beta-alanine from alpha-alanine, which indicates the cell has alanine 2,3-aminomutase activity

The mutagenized Fncodm library generated above in EXAMPLE 2 was transformed into electrocompetent cells of the *ΔpanD*::CAT strain of *E. coli* (see WO 03/062173 and EXAMPLE 10 herein). Transformants were recovered one hour in SOC media, followed by addition of 100 µM PTG and 25 µg/ml kanamycin and a further three hours of growth The cells were then centrifuged and washed with 0 85% NaCl and resuspended in 500 µL of M9 minimal medium supplemented with 4% glucose, 100 µM IPTG, 50 µM Fe(NH₄)₂(SO₄)₂, 2 mg/mL alpha-L-alanine, trace elements, and 5 µg/mL kanamycin (Sigma, St Louis, MO). Thirty µL of the resuspended cells were used to inoculate 1.33 mL of M9 media in a 2 mL glass tube, After six days, grown culture was used to inoculate liquid LBK25 media. After 5.5 hours of growth, plasmid DNA was isolated and used to transform fresh competent cells of the *ΔpanD*::CAT strain Retransformation of the grown cells reduces mutants that could grow due to an enabling mutation in the host genome, and ensures that growth is due to a plasmid-borne effect.

Transformants were recovered in SOC media for one hour, washed with 0.85% NaCl and suspended in 1 mL of NaCl. Colony counts were obtained from platings on LBK25 media, and remnant resuspension (stored at 4°C) was plated on LBK25 media to obtain approximately 250 colonies per plate. Individual colonies were patched to both LBK25 and M9 minimal media. Colonies that showed superior growth on M9 media were tested in a liquid growth test using 14 mL M9 minimal media (as above) in a 2 mL tube. Resuspended colonies were used to inoculate media both with (25 µM) and without pantothenate Culture ODs were read when the pantothenate controls reached OD₆₀₀s of approximately 0.7. Clones were identified that had a high ratio of growth without pantothenate to growth with pantothenate. The plasmid DNA of superior clones was sequenced using standard molecular biology methods. It was observed that all clones had the same sequence (SEQ ID NO: 18, and the corresponding amino acid sequence in SEQ ID NO: 19)

The *mutated F. nucleatum kam* gene sequence, which encodes for an alanine 2,3-aminomutase (Fnaam), is shown in SEQ ID NO: 18, and the corresponding amino acid sequence shown in SEQ ID NO: 19 In addition to the three directed mutations generated in EXAMPLE 2, six amino acid changes were observed in the mutated sequence, as compared to the *F. nucleatum kam* gene sequence (FIG. 4) These additional substitutions are E31K, Y64F, Q87R, D145G, L229M, and K401E. However, all of these mutations may not be needed for alanine 2,3-aminomutase activity.

A second round mutagenic library was made as described above, using plasmid DNA of the Fnaam mutant as template Since that starting clone now has alanine 2,3-aminomutase activity, selection relied on liquid enrichment techniques rather than plating on solid media. With liquid enrichment, a *ΔpanD*/*ΔpanF* selection host can be utilized. The *panF* gene encodes for a pantothenate permease that allows concentrative uptake of pantothenate. Use of the *panF* deletion mutant prevents possible cross-feeding of inactive clones with pantothenate produced by clones with an active alanine 2,3-aminomutase. The mutagenic library, consisting of DNA from approximately 48,000 clones, was transformed into the *ΔpanD*/*ΔpanF E. coli* BW25113 strain. Half of the recovery was used to inoculate 30 mL of M9 minimal media supplemented with 0.4% glucose, 100 µM IPTG, 20 µM ferric citrate, 2 mg/mL alpha-L-alanine, trace elements, and 25 µg/ml kanamycin. Media was placed in 50 mL tubes and was subjected to occasional mixing Good growth was seen after 8 days and was streaked to M9 minimal media.

A colony was tested in liquid growth tests as described above and performed approximately four times better than the Fnaam mutant in the Δ*panD*/Δ*panF* strain. Plasmid DNA from the new mutant was sequenced and used to retransform the Δ*panD* strain. Liquid growth tests of the retransformants confirmed that the growth advantage was conferred by the plasmid. Two additional amino acid substitutions (K37E and D180N) are present in this *F. nucleatum* alanine 2,3-aminomutase (Fnaam2, SEQ ID NO: 21). The DNA sequence is shown in SEQ ID NO: 20. The improved mutant performed approximately three times better than the Fnaam mutant in liquid growth tests with 0 5 mg/mL of alpha-L-alanine.

### EXAMPLE 4

### Cloning and codon improvement of a Clostridium sticklandii lysine 2,3-aminomutose

This example describes methods used to clone a *C. sticklandii* lysine 2,3-aminomutase and optimize its codon useage for expression in *E. coli* Although the degradation pathway for lysine that involves lysine 2,3- and lysine 5,6-aminomutases has been described for C. *sticklandii,* the gene sequences are unknown.

*C. sticklandii* (ATCC 12662) was propagated in ATCC medium 1053 and genomic DNA was isolated as described for *F. nucleatum.* Three degenerate forward (F 1: 5'-YTWAGAATGGCWATWACWCC-3' (SEQ ID NO: 22); F2: 5'-AGAAARCARGCWATWCCWAC-3' (SEQ ID NO: 23); and F3: 5'-GGWYTWACWCAYAGATAYCC-3' (SEQ ID NO: 24)) and three degenerate reverse (R1: 5'-TAWGTWGTWATWACWCCYTC-3' (SEQ ID NO: 25); R2: 5'-TCWACWACRAAWGTWGGWAC-3' (SEQ ID NO: 26); and R3: 5'-CCWCCWCCWGGWGCRTCWAC-3' (SEQ ID NO: 27)) PCR primers were designed from the conserved protein regions of known lysine 2,3-aminomutase genes A C. *sticklandii* codon preference table was used to design primers from the protein sequences.

The primers were used in all logical combinations in PCR using Taq polymerase and 1 ng of *C. sticklandii* genomic DNA/µL reaction mix. PCR was conducted using a touchdown PCR program with 4 cycles at an annealing temperature of 56°C, 4 cycles at 54°C, 4 cycles at 52°C, and 20 cycles at 50°C Each cycle used an initial 45-second denaturing step at 94°C and a two minute extension at 72°C, and there was a final extension for 5 minutes at 72°C. The amount of PCR primer used in the reaction was increased three fold above typical PCR amounts due to the degeneracy in the 3' end of the primer. In addition, a separate PCR reaction containing each individual primer was made to identify PCR product resulting from single degenerate primers.

Twenty five µL of each PCR product was run on a 1 5% agarose gel. Strong bands were produced by primer combinations F1-R1(950 bp), F1-R3 (900 bp) and F3-R3 (730 bp), which were the approximate expected sizes based on genes from other species. Weaker bands were produced by primer combinations F3-R1 (800 bp), F1-R2 (900 bp), and F3-R2 (750 bp) No bands were obtained for the F2-R1, F2-R2, F2-R3 primer combinations or the individual primer controls. The F 1-R1 and F1-R3 fragments were isolated and purified using Qiagen Gel Extraction procedure (Qiagen Inc , Valencia, CA). Four µL of the purified band was ligated into TOPO 4 0 vector and transformed by a heat-shock method into TOP10 *E. coli* cells using a TOPO cloning procedure (Invitrogen, Carlsbad, CA). Transformations were plated on LB media containing 100 µg/ml of ampicillin and 50 µg/mL of X-gal (5-Bromo-4-Chloro-3-Indolyl-β-D-Galactopyranoside). Single, white colonies were placed in a small amount of buffer and an aliquot was used to start 5 mL cultures for plasmid DNA isolation. The remnant was heated at 95°C for 10 minutes and 2 µL was used in a PCR reaction to confirm the presence of the desired insert. Plasmid DNA was obtained, using a QiaPrep Spin Miniprep Kit, from multiple colonies showing the desired insert. The insert for the F1-R1 clones was sequencing with M13R and M13F primers Sequence analysis showed this fragment to be homologous to known lysine 2,3-aminomutase genes.

### Genome walking to obtain complete coding sequence.

Primers for conducting genome walking in both upstream and downstream directions were designed using sequence obtained for the F1-R1 fragment described above, avoiding the region corresponding to the degenerate primers. Primer sequences were 5'-CCTTTCAGTTGGAATTGAGCACTTTAGAAC-3'(SEQ ID NO: 28) for GSP1F, 5'-GATACTGCGTTCGTACATTTGTTGTGGATG-3'(SEQ ID NO: 29) for GSP2F, 5'-CGCTGCTCTATGTAGCTCTAAAGAAAGAG-3' (SEQ ID NO: 30) for GSP1R, and 5'-CAGCTTGCTTTCTTACAGGGTCATTTGG-3' (SEQ ID NO: 31) for GSP2R, where GSP1F and GSP2F are primers facing downstream, GSP1R and GSP2R are primers facing upstream, and GSP2F and GSP2R are primers nested inside of GSP1F and GSP1R, respectively Genome walking was conducted according to the manual for Clontech's Universal Genome Walking kit (ClonTech Laboratories, Inc., Palo Alto, CA), with the exception that the restriction enzymes used were *Hinc* II, *Ssp* I, *EcoR* V, *Pvu* II and *Dra* I.

First round PCR was conducted in a Perkin Elmer 9700 Thermocycler with an initial denaturation for 15 seconds at 94°C; 7 cycles consisting of 5 seconds at 94°C and 3 minutes at 70°C; and 32 cycles consisting of 5 seconds at 94°C and 3 minutes at 65°C, with a final extension at 65°C for 4 minutes. Second round PCR consisted of an initial denaturation for 15 seconds at 94°C; 5 cycles with 5 seconds at 94°C and 3 minutes at 70°C; 26 cycles with 5 seconds at 94°C and 3 minutes at 65°C; and a final extension at 65°C for 4 minutes

Twenty µL of PCR product from each round was run on a 1.5% agarose gel Amplification products were obtained with the *Ssp* I, *EcoR V, Pvu* II and *Dra* I libraries for the forward primers and *Ssp* I, *Hinc* II, Pvu II and *Dra* I libraries for the reverse primers. The second round products for the *Dra* I forward reaction (1.2 Kb) and *Hinc* II reverse reaction (13 Kb) were gel purified, cloned, and screened for insert size as described above. Plasmid DNA for multiple clones with the desired insert size was sequenced with M13R and M13F primers. The sequences were aligned with the sequence of the original gene fragment to determine the complete sequence of the *C. sticklandii* lysine 2,3-aminomutase homolog. The entire gene was then recloned in a vector using PCR amplification from genomic DNA Novel genes such as the C *sticklandii kam* gene (SEQ ID NO: 32, and its corresponding protein sequence shown in SEQ ID NO: 33) can be used as starting template for mutagenesis to obtain alanine 2,3-aminomutase activity.

The *C*. *sticklandii kam* gene shown in SEQ ID NO: 32 was partially codon optimized for expression in *E. coli.* Rare arginine codons were replaced with more preferred arginine codons by incorporation of primers containing the more preferred codons during amplification reactions, as described in Example 2. After two rounds of primer incorporation, the PCR product was cloned into the pPRO-Nde vector Sequencing revealed the best clone to have eight rare arginine codons, as compared to 22 in the wildtype gene. The partially optimized C *sticklandii kam* gene (Csco) is shown in SEQ ID NO: 34 (with the non-altered protein sequence shown in SEQ ID NO: 35).

### EXAMPLE 5

### in vitro mutagenesis of a Clostridium sticklandii lysine 2,3-aminomutase (kam gene)

This example describes methods used to mutagenize the partially optimized C. *sticklandii* kam gene (SEQ ID NO: 34) generated in EXAMPLE 4 to identify mutants having alanine 2,3-aminomutase activity.

Four mutations that had been obtained previously in *B. subtilis, P. gingivalis, or F. nucleatum* alanine 2,3-aminomutases (for example see SEQ ID NOS: 1-6 and 18-21 and WO 03/062173) were transferred by directed mutagenesis into the *C. sticklandii* Csco gene (SEQ ID NO: 35) These mutations included E28K, L93M, M126V, and D329H substitutions. The mutations were made using a Stratagene QuikChange^{®} Multi Site-Directed Mutagenesis Kit (Stratagene) The primers used to make these mutations were: CsE28K: 5'Phos/GAAATGGCAAGTAAGAAATCGTATAAAGACTGTTGAAGAACTTAA (SEQ ID NO: 36); CsL93M: 5'Phos/TCGCGCAGCGTCTGATATGGAAGACCCACTTCATG (SEQ ID NO: 37); CsM126V: 5'Phos/GACTGATCAATGTTCAGTATACTGCCGCCACTGTACTCGT (SEQ ID NO: 38); and CsD329H: 5'Phos/GTTCCTACATTTGTTGTGCATGCACCTGGTGGTG (SEQ ID NO: 39).

Liquid growth tests as described in EXAMPLE 2 of four clones having all four directed mutations in the Csco gene (SEQ ID NO: 41) demonstrated low levels of alanine 2,3-aminomutase activity After subculturing the growth test cultures, the Cscodm strain exhibited approximately three-fold more growth than the Csco parent strain, indicating a weak level of alanine aminomutase activity.

To introduce additional mutations into a Cscodm gene (SEQ ID NO: 40) *in vitro,* an error-prone PCR method was used as described above in Example 2, except that the PCR program consisted of an initial denaturation at 94°C for 2 minutes; 30 cycles of 94°C for 30 seconds, 55°C for 45 seconds, and 72°C for 2.25 minutes; and a final extension at 72°C for 7 minutes.

Following PCR, the PCR product was digested with *Not I* and *Nde I.* Equal amounts of DNA from each treatment were ligated into the vector pPRO-Nde, and transformed into *E. coli* Electromax^{™} DH10B^{™} cells Multiple transformations were plated on LBK25 media to obtain approximately 54,000 colonies Colonies were scrapped from plates and plasmid DNA prepared using a Qiagen MiniSpin Plasmid procedure. Plasmid DNA was precipitated with ammonium acetate and ethanol to increase its concentration before transformation into selection hosts.

### EXAMPLE 6

### Identification of Clones having Alanine 2,3-Aminumutase Activity

This example describes methods used to identify mutated lysine 2,3, aminomutase clones that have alanine 2,3 aminomutase activity.

The mutagenized Cscodm plasmid library generated above in EXAMPLE 5 was transformed into electrocompetent cells of a *ΔpanD*/*ΔgabT*/*ΔyeiA* strain of *E. coli* BW25113. The *gabT and yeiA* mutations were made to eliminate formation or processing of beta-alanine by cellular pathway Transformants were recovered one hour in SOC media, centrifuged, washed with 0.85% NaCl, and resuspended in 1 mL of NaCl. Half of the recovery was used to inoculate 25 mL of M9 minimal medium supplemented with 04% glucose, 100 µM IPTG, 20 µM ferric citrate, 2 mg/mL alpha-L-alanine, trace elements, and 25 µg/mL kanamycin (Sigma, St. Louis, MO),

After three days, grown culture was streaked to LBK media. Individual colonies were patched to both LBK and M9 minimal media Colonies that showed superior anaerobic growth on M9 media were tested in a liquid growth test using 16 mL M9 minimal media (as above) in a 2 mL tube Resuspended colonies were used to inoculate media both with (25µM) and without pantothenate Culture ODs were read when the pantothenate controls reached an OD₆₀₀ of approximately 0 6. As shown in table 1, three clones had a high ratio of growth without pantothenate to growth with pantothenate Plasmid DNA of these three clones was sequenced and used to retransform a *ΔpanD E. coli* strain Growth tests were repeated on the retransformed strains to ensure that the growth advantage was conferred by the plasmid rather than by a host effect.

**Table 1: Growth in the presence of C. sticklandii alanine, 2,3-aminomutase genes.**

| **Clone** | **Ratio** |
|---|---|
| Cscodm (SEQ ID NOS: 40 and 41) | 0.12 |
| Cscodm mut8 (SEQ ID NOS: 42 and 43) | 0.45 |
| Cscodm mut12 (SEQ ID NO: 44 and 45) | 0.68 |
| Cscodm mut15 (SEQ ID NO: 46 and 47) | 0.48 |

The three mutant C *sticklandii kam* gene sequences, which encode for alanine 2,3-aminomutases, are shown in SEQ ID NOS: 42, 44, and 45, and the corresponding amino acid sequences are shown in SEQ ID NOS: 43, 45, and 47 In addition to the four directed mutations (present in SEQ II3 NO: 41), there were one to four amino acid changes observed in the mutated sequences, as compared to the C *sticklandii kam* gene sequence (FIG. 5). These mutations include S9T, V30A, C50R, L51H, E69G, Q123L, Q139R, and K185R. However, all of these mutations may not be necessary to attain alanine 2,3-aminomutase activity

### EXAMPLE 7

### in vitro mutagenesis of a Porphyromonas gingivalis alanine 2,3-aminomutase

Mutagenic PCR was conducted on a previously described (WO 03/062173) *P gingivalis* alanine 2,3-aminomutase (SEQ ID NO: 5 with the corresponding protein sequence shown in SEQ ID NO: 6) using the methods described in Example 2, except that the PCR program consisted of an initial denaturation at 94°C for 2 minutes; 30 cycles of 94°C for 30 seconds, 55°C for 1 minute, and 72°C for 2 25 minutes; and a final extension at 72°C for 7 minutes

Following PCR, the PCR product was digested with *Not 1* and *Nde 1.* Equal amounts of DNA from each treatment were ligated into the vector pPRO-Nde, and transformed into *E*. *coli* Electromax™ DH10B™ cells Plasmid DNA was isolated from single colonies and sequenced to obtain an estimate of the mutation rate. The average mutation rate was 0.3% Multiple transformations were plated to LBK25 media to obtain approximately 80,000 colonies. Colonies were scrapped from plates and plasmid DNA prepared using a Qiagen MiniSpin Plasmid procedure Plasmid DNA was precipitated with ammonium acetate and ethanol to increase its concentration before transformation into selection hosts.

### EXAMPLE 8

### Selection and Identification of Clones having Improved Alanine 2,3-Aminomutase

The mutagenized Pgaam plasmid library generated above in EXAMPLE 7 was transformed into electrocompetent cells of a Δ*panD* strain of *E. coli* BW25113 Transformants were recovered one hour in SOC media, centrifuged, washed with 0 85% NaCl, and resuspended in 1 mL of NaCl 10 µL was used to inoculate 1.33 mL of M9 minimal medium supplemented with 0.4% glucose, 100 µM IPTG, 50 µM Fe(NH₄)₂(SO₄)₂, 2 mg/mL alpha-L-alanine, trace elements, and 25 µg/mL kanamycin (Sigma, St. Louis, MO) in a 1.8 mL glass tube After three days, 100 µL of grown culture was used to inoculate a fresh tube of the same media After overnight growth, culture was streaked out on M9 minimal media and placed in an anaerobic chamber. Six colonies that grew were patched to LBK and tested in a liquid growth test using 1.4 mL M9 minimal media (as above) in a 2 mL tube. Resuspended colonies were used to inoculate media both with (25 µM) and without pantothenate Culture ODs were read when the pantothenate controls reached an OD₆₀₀ of approximately 0.7. Clones were identified that had a high ratio of growth without pantothenate to growth with pantothenate.

As shown in Table 2, the Pgaam2 clone had a high ratio of growth without pantothenate to growth with pantothenate. Plasmid DNA of this clone was sequenced and used to retransform a Δ*panD E. coli* strain Growth tests were repeated on the retransformed strains to ensure that the growth advantage was conferred by the plasmid rather than by a host effect (Table 3, "retransformed").

**Table 2: Growth in the presence of P. gingivalis wild-type and mutated lysine 2,3-aminomutase.**

| **Clone** | **Ratio** |
|---|---|
| Pgkam (SEQ ID NO: 52) | 0.10 |
| Pgaam (SEQ ID NOS: 5 and 6) | 0.33 |
| Pgaam2 (SEQ ID NOS: 48 and 49) | 0.73 |
| Pgaam (retransformed) | 0.46 |
| Pgaam2 (retransformed) | 0.84 |

The amino acid substitutions E30K and 1192V are present in the *improved P. gingivalis* alanine aminomutase (Pgaam2, SEQ ID NO: 49) The DNA sequence is shown in SEQ ID NO: 48.

To increase the alanine 2,3-aminomutase activity of Pgaam2, (SEQ ID NO: 49), several negatively-charged amino acids were mutagenized. Since negatively-charged amino acids may interact with the positively-charged native substrate, lysine, elimination of some or all negative charges could increase activity with alanine as a substrate since it is smaller and uncharged Directed mutagenesis was done using a Stratagene QuikChange® Multi Site-Directed Mutagenesis Kit with the following modifications: a 50 µL reaction was made and was precipitated with NH₄OAc and ethanol after Dpn I treatment After resuspension in 10 µL of water, 2.5 µL was transformed into a Δ*panD E. coli* strain One third of the transformation recovery was used to inoculate 8 mL of M9 minimal media in a 14 mL tube The media was supplemented with 0.4% glucose, 100 µM IPTG, 100 mM MOPS pH 7.0, 50 µM ferric citrate, I mg/mL alpha-L-alanine, trace elements, and 25 µg/mL kanamycin After 5 days of growth, platings were made on M9 minimal media to obtain individual colonies. Individual colonies were patched to LBK25 media and tested in a liquid growth test using 1 4 mL M9 minimal media (as above, 1 mg/mL alpha-L-alanine) in a 2 mL tube Resuspended colonies were used to inoculate media both with (25 µM) and without pantothenate Culture ODs were read when the pantothenate controls reached an OD₆₀₀ of approximately 07 Plasmid DNA from a positive colony (Pgaam2 L26I) was retransformed into the Δ*panD* strain and the retransformed strain was retested in a liquid growth test (Table 3).

**Table 3: Growth in the presence of P. gingivalis alanine 2,3-aminomutase genes**

| **Clone** | **Ratio** |
|---|---|
| Pgaam2 (SEQ ID NOS: 48 and 49) | 0.21 |
| Pgaam2 L261 (SEQ ID NOS: 50 and 51) | 0.53 |

Sequence results showed that the mutation was at the nucleotide immediately downstream of the 3' end of one of the mutation primers. Thus, it may have been caused by an amplification error or error in the primer synthesis The DNA sequence of the improved P *gingivalis* alanine aminomutase (Pgaam2L26I) is shown in SEQ ID NO: 50 and the corresponding amino acid sequence shown in SEQ ID NO: 51

### Example 9

### In vitro assay of Alanine 2,3-Aminomutase activity

This example describes methods used to determine alanine 2,3-aminomutase activity for SEQ ID NO: 49. One skilled in the art will recognize that similar methods can be used to determine the alanine 2,3-aminomutase activity for any alanine 2,3-aminomutase protein disclosed herein, such as SEQ ID NO: 19, 21, 43, 45, 47, or 51, as well as variants, fragments, or fusions thereof that retain alanine 2,3-aminomutase activity.

Proteins with alanine 2,3-aminomutase activity were expressed in *E. coli* BL21(DE3) with a C-terminal strep-tag using the pASK-IBA3 vector (IBA, St. Louis, MO), using procedures described by the manufacturer. Cells carrying clones of strep-tag aminomutases were grown in Terrific Broth supplemented with 100 µg/mL ampicillin and 40 µg/mL ferric ammonium citrate, and expression induced at OD₆₀₀ = 1-2 with 0 2 µg/mL anhydrotetracycline The incubation temperature was reduced to 25°C and cells were harvested by centrifugation after overnight growth.

The cell pellet was resuspended in buffer (50 mM HEPPS pH 8, 25 µM pyridoxal phosphate, 0.1 mM alpha-L-alanine) at a ratio of 2 mL buffer/g cell paste and the cell suspension was sonicated 2 x 1.5 minutes at 9-15 W The homogenized cell suspension was centrifuged and the soluble cell free extract (CFE) was carefully decanted and reserved

Alanine 2,3-aminomutase activity was assayed based on the procedure as described by Chen et al (Biochem J 348:539-549, 2000) for lysine 2,3-aminomutase, except the assay contained alpha-L-alanine instead of lysine. 0.1 mL of CFE was pre-redueed by treatment with 0.4 mL Reductive Incubation Buffer (4 6 ml H₂O, 0.25 mL of 1 M HEPPS pH 8, 0 05 mL of 100 mM Fe(NH₄)₂(SO₄)₂, 0.05 mL of 50 mM pyridoxal phosphate, 0 05 mL of 100 mM dithiothreitol; all solutions were prepared anaerobically), and the preincubation allowed to proceed for 4 h at 37°C. The following components, prepared anaerobically, were added, in older, to the preincubated enzyme: 0.21 mL H₂O, 005 mL 1 M HEPPS pH 8, 0 02 mL 5-10 mM S-adenosylmethionne, 0.02 mL 100 mM Na dithionite, 0 2 mL 500 mM alpha-L-alanine The reaction was quenched at various time points by mixing with an equal volume of 90% formic acid, and the formation of beta-alanine monitored by HPLC using an Interaction Chromatography AA511 column and post-colunm derivatization with O-phthaldehyde Pickering Laboratories buffers Na 328 and Na 740 were used to develop the chromatogram, run at 0 5 ml/min flow, column temperature of 60°C, and chromatograms of reactions were compared with those of standard amounts of beta-alanine for quantitation

Using this assay, the specific activity of the Pgaam2 alanine 2,3-aminomutase (SEQ ID NO: 49) was determined to be 0 03 ± 0.01 units/mg protein (1 unit = production of 1 umol beta-alanine per minute)

### EXAMPLE 10

### Construction of E. coli ΔpanD::CAT strain

To identify genes encoding polypeptides that can perform the alanine 2,3-aminomutase, reaction, an efficient screen or selection for the desired activity is needed Therefore, a selection method was developed by recognizing that E coli uses beta-alanine for the synthesis of pantothenic acid which in turn is a component of coenzyme A (CoA) and of acyl carrier protein (ACP). CoA and ACP are the predominant acyl group carriers in living organisms, and are essential for growth. In *E*. *coli,* the primary route to beta-alanine is from aspartate in a reaction catalyzed by aspartate decarboxylase (E.C, 4.1.1.11), which is encoded by the *panD* gene (FIG. 3). A functional deletion mutation *of panD* results in beta-alanine auxotrophy and growth inhibition, which can alleviated by the exogenous addition of pantothenate or beta-alanine, or by the production of beta-alanine from another source,

Two *E*. *coli* strains were used in the screen, both of which are deficient in beta-alanine synthesis. The strain DV1 (#6865, *E coli* Genetic Stock Center, New Haven CT; Vallari and Rock, *J. Bacteriol* 164: 136.42, 1985) is an *E. coli* mutant made by chemical mutagenesis, which has host (chromosomal) mutations of both *the panF* and *panD* genes which renders both genes non-functional The *panF* gene encodes the uptake of pantothenate from the medium, and thus the combination of *panD* and *panF provides* a more stringent requirement for beta-alanine for growth Therefore, although the DV1 strain was known, its use for selecting cells having alanine 2,3-aminomutase activity was not previously known

The other selection strain, BW25113 ΔpanD::CAT, includes a deletion of the *panD* locus, to prevent revertants of the *panD* mutation which would be able to grow without exogenous beta-alanine This strain, which has an insertion of a chloramphenicol resistance marker conferred by the CAT gene into the panD locus, was constructed using the gene inactivation method of Datsenko and Wanner (Proc Natl. Acad Sci USA 97: 6640-5, 2000) using *E. coli* strains BW25113/pKD46 and BW 25141/pKD3 for the *E. Coli* Genetic Stock Center

The CAT gene of pKD3 was amplified using primers
TAICAATICGTIACAGGCGAIACATGGCACGCTICGGCGCGIGIAGGCTGGAGCTGCTTC (SEQ ID NO: 53) and
GATGICGCGGCTGGTGAGTAACCFAGCCGCAGGGATAACAACATATGAATATCCICCTTA G (SEQ ID NO: 54) The PCR reaction included 30 µl 10X concentrated PCR buffer (Roche Molecular Biochemicals), plasmid pKD.3, 0.2 mM each dNTP, 0.2 µM each primer, and 15 units Taq polymerase (Roche Molecular Biochemicals) in a final volume of 300 µl The PCR reaction was incubated at 95°C for 30 seconds followed by 30 cycles of 95°C for 30 seconds, 45°C for 30 seconds, 72°C for 1 min, then 72°C for 10 minutes The PCR product was precipitated with ethanol, digested with *DpnI,* purified with the QIAquick PCR Purification Kit (Qiagen), and transformed into BW25113/pKD46 expressing the recombination functions Transformants were plated on LB plates containing 25 µg/ml chloramphenicol and 5 µM beta-alanine.

Chloramphenicol-resistant transformations were single-colony purified on non-selective LB medium supplemented with 5 µM beta-alanine at 43°C, and single colonies tested for retention of chloramphenicol resistance, loss of ampicillin resistance (indicating curing of pKD46), and requirement for beta-alanine for growth on M9-glucose minimal medium Confirmation of correct insertion of the CAI gene into *the panD* locus was carried out by colony PCR of the resultant *ΔpanD::CAT strain* using primers that flank the insertion locus (TIACCGAGCAGCGITCAGAG, SEQ ID NO: 55; and CACCTGGCGGIGACAACCAT, SEQ ID NO: 56). While the wild-type panD locus is expected to yield a PCR product of 713 basepairs, the Δ*panD. CAT* construct yielded a 1215-basepair product A derivative of the Δ*panD:: CAT* strain, in which the inserted *CAT* gene is removed by the activity of the FLP recombinase encoded by plasmid pCP20, was constructed as described previously (Datsenko and Wanner, Proc. Natl. Acad Sci USA 97: 6640-5, 2000). This strain is referred to as Δ*panD*

A secondary route to beta-alanine exists in *E coli* based on the reductive pathway of uracil catabolism (West, Can J. Microbiol 44: 1106-9, 1998, FIG. 2). In this pathway, uracil is reduced to dihydrouracil by the enzyme dihydropyrimidine dehydrogenase (E.C. 1.3.1.2). Dihydrouracil is then converted by dihydropyrimidinase (E.C. 3.5 2 2) to N-carbamoyl-beta-alanine, which in turn is hydrolyzed by N-carbamoyl-beta-alanine amidohydrolase (E. C. 3 .5 1.6) to beta-alanine, CO₂, and NH₃. To prevent the formation of beta-alanine by this pathway, the gene encoding dihydropyrimidine dehydrogenase, *yeiA* (GenBank Accession No AAC75208), was insertionally deleted by the method of Datsenko and Wanner as described above The *CAT* gene of pKD3 was amplified using primers
GCGGCGIGAAGTTICCCAACCCGIICIGCCICICTICTICGGIAGGCIGGAGCIGCTTC (SEQ ID NO: 57), and
TTACAACGTTACCGGGTGTTCTTTCICGCCTTICTTAAACCAIATGAAIATCCICCTTAG (SEQ ID NO:58)

Chloramphenicol-resistant insertion mutants were isolated as described above, and the resistance marker transduced into the *ΔpanD* strain to generate the double mutant *ΔpanD*/*ΔyeiA*::*CAT.*
Electrocompetent cells of *E. coli* BW 25115 Δ*panD : CAT,* Δ*panD,* or *ΔpanD*/*ΔyeiA*::*CAT;* were generated and used as hosts for the transformation of libraries of mutant lysine 2,3-aminomutase DNAs as described in the EXAMPLES above

### EXAMPLE 11

### Selection for Alanine 2,3-Ammomutase Activity Without Using a Mutagenized Lysine 2,3-Aminomutase

An alternative method to identifying cells having alanine 2,3-aminomutase activity is to plate cells, such as the *DV1* or *ΔpanD*::CAT cells described in EXAMPLE 10, on the media described above, without transfecting them with a mutagenized lysine 2,3-aminomutase library Such cells are selected as described above, and verified for the presence of alanine 2,3-aminomutase activity as described in EXAMPLES 3, 5, 6, 8 and 9.

Cells can be mutagenized before plating, for example by exposing the cells to UV irradiation or chemicals (such as EMS) This permits isolation of mutants having mutations in one or more other genes which result in the cell having alanine 2,3-aminomutase activity

Alternatively, the cells can be unaltered before plating (such as not transformed, not mutagenized) This method permits isolation of naturally occurring strains having alanine 2,3-aminomutase activity.

### EXAMPLE 12

### Production of Pantothenate from Beta-Alanine

Pantothenate can be produced from beta-alanine by a polypeptides having alpha-ketopantoate hydroxymethyltansferase (E.C.2.1.2.11), alpha-ketopantoate reductase (E.C. 1.1.1.169), and pantothenate synthase (E.C 63 2.1) activity (FIG. 3)

Using the cloning methods described herein, alpha-ketopantoate hydroxymethyltransferase (E.C.2.1.2.11), alpha-ketopantoate reductase (E C. 1.1.1 169), and pantothenate synthase (E C, 6 3 2.1) polypeptides can be isolated, sequenced, expressed, and tested One skilled in the art will understand that similar methods can be used to obtain the sequence of any such polypeptides from any organism

### EXAMPLE 13

### Recombinant Expression

With publicly available enzyme cDNA and amino acid sequences, and the alanine 2,3-aminomutases disclosed herein, as well as variants, fragments and fusions thereof, the expression and purification of any protein, such as an alanine 2,3-aminomutase, by standard laboratory techniques is enabled. One skilled in the art will understand that enzymes and fragments thereof can be produced recombinantly in any cell or organism of interest, and purified prior to use, for example prior to production of 3-HP, pantothenate and derivatives thereof.

Methods for producing recombinant proteins are well known in the art Therefore, the scope of this disclosure includes recombinant expression of any protein or fragment thereof, such as alanine 2,3-aminomutase For example, see U S. Patent No: 5,342,764 to Johnson et al *;* U.S Patent No: 5,846,819 to Pausch et al.*;* U S Patent No: 5,876,969 to Fleet et al*,* and Sambrook et al. (Molecular Cloning A laboratory Manual, Cold Spring Harbor, New York, 1989, Ch 17)

Briefly, partial, full-length, or variant cDNA sequences, which encode for a protein, can be ligated into an expression vector, such as a bacterial expression vector. Proteins can be produced by placing a promoter upstream of the cDNA sequence Examples of promoters include, but are not limited to *lac, trp, tac, trc,* major operator and promoter regions of phage lambda, the control region of fd coat protein, the early and late promoters of SV40, promoters derived from polyoma, adenovirus, retrovirus, baculovirus and simian virus, the promoter for 3-phosphoglycerate kinase, the promoters of yeast acid phosphatase, the promoter of the yeast alpha-mating factors and combinations thereof

Vectors suitable for the production of intact native proteins include pKC30 (Shimatake and Rosenberg, 1981, Nature 292:128), pKK177-3 (Amann and Brosius, 1985, Gene 40:183) and pET-3 (Studier and Moffatt, 1986, J *Mol. Biol* 189:113). A DNA sequence can be transferred to other cloning vehicles, such as other plasmids, bacteriophages, cosmids, animal viruses and yeast artificial chromosomes (YACs) (Burke et al , 1987, Science 236:806-12). These vectors can be introduced into a variety of hosts including somatic cells, and simple or complex organisms, such as bacteria, fungi (Timberlake and Marshall, 1989, Science 244:1313-7), invertebrates, plants (Gasser and Fraley, 1989, Science 244:1293), and mammals (Pursel et al 1989, Science 244:1281-8), which are rendered transgenic by the introduction of the heterologous cDNA

For expression in mammalian cells, a cDNA sequence can be ligated to heterologous promoters, such as the simian virus SV40, promoter in the pSV2 vector (Mulligan and Berg, 1981, Proc. Natl. Acad. Sci. USA 78:2072-6), and introduced into cells, such as monkey COS-1 cells (Gluzman, 1981, Cell 23:175-82), to achieve transient or long-term expression, The stable integration of the chimeric gene construct may be maintained in mammalian cells by biochemical selection, such as neomycin (Southern and Berg, 1982, J Mol Appl. Genet 1:32741) and mycophoenolic acid (Mulligan and Berg, 1981, Proc. Natl. Acad. Sci. USA 78:2072-6)

The transfer of DNA into eukaryotic, such as human or other mammalian cells is a conventional technique The vectors are introduced into the recipient cells as pure DNA (transfection) by, for example, precipitation with calcium phosphate (Graham and vander Eb, 1973, Virology 52:466) strontium phosphate (Brash et al., 1987, Mol Cell Biol. 7:2013), electroporation (Neumann et al., 1982, EMBO J. 1:841), lipofection (Felgner et al, 1987, Proc Natl Acad Sci USA 84:7413), DEAE dextran (McCuthan et al, 1968, J. Natl. Cancer Inst. 41:351), microinjection (Mueller et al, 1978, Cell 15:579), protoplast fusion (Schafner, 1980, Proc. Natl Acad. Sci. USA 77:2163-7), or pellet guns (Klein et al, 1987, Nature 327:70) Alternatively, the cDNA can be introduced by infection with virus vectors, for example retroviruses (Bernstein et al., 1985, Gen. Engrg. 7:235) such as adenoviruses (Ahmad et al., 1986, J Virol. 57:267) or Herpes (Spaete et al, 1982, Cell 30:295)

### EXAMPLE 14

### Peptide Synthesis and Purification

The enzymes disclosed herein, such as the alanine 2,3-aminomutases and variants, fusions, and fragments, thereof can be chemically synthesized by any of a number of manual or automated methods of synthesis known in the art For example, solid phase peptide synthesis (SPPS) is carried out on a 0.25 millimole (mmole) scale using an Applied Biosystems Model 431A Peptide Synthesizer and using 9-fluorenylmethyloxycarbonyl (Fmoc) amino-terminus protection, coupling with dicyclohexylcarbadiimide/hydroxybenzotriazole or 2-(1H-benzo-triazol-1-yl)-1,1,3,3-tetramethyluronium hexafluorophosphate/hydroxybenzotriazole (HBIU/HOBI), and using p-hydroxymethylphenoxymethylpolystyrene (HMP) or Sasrin resin for carboxyl-terminus acids or Rink amide resin for carboxyl-terminus amides

Fmoc-derivatized amino acids are prepared from the appropriate precursor amino acids by tritylation and triphenylmethanol in trifluoroacetic acid, followed by Fmoc derivitization as described by Atherton et al (Solid Phase Peptide Synthesis, IRL Press: Oxford, 1989):

Sasrin resin-bound peptides are cleaved using a solution of 1% TFA in dichloromethane to yield the protected peptide, Where appropriate, protected peptide precursors are cyclized between the amino-and carboxyl-termini by reaction of the amino-terminal free amine and carboxyl-terminal free acid using diphenylphosphorylazide in nascent peptides wherein the amino acid sidechains are protected.

HMP or Rink amide resin-bound products are routinely cleaved and protected sidechain-containing cyclized peptides deprotected using a solution comprised of trifluoroacetic acid (TFA), optionally also comprising water, thioanisole, and ethanedithiol, in ratios of 100 : 5 : 5 : 2.5, for 0.5 - 3 hours at RI.

Crude peptides are purified by preparative high pressure liquid chromatography (HPLC), for example using a Waters Delta-Pak C18 column and gradient elution with 0 1% IFA in water modified with acetonitrile. After column elution, acetonitrile is evaporated from the eluted fractions, which are then lyophilized. The identity of each product so produced and purified may be confirmed by fast atom bombardment mass spectroscopy (FABMS) or electrospray mass spectroscopy (ESMS).

In view of the many possible embodiments to which the principles of our disclosure may be applied, it should be recognized that the illustrated embodiments are only particular examples of the disclosure and should not be taken as a limitation on the scope of the disclosure. Rather; the scope of the disclosure is in accord with the following claims We therefore claim as our invention all that comes within the scope and spirit of these claims

The invention will now be described by reference to the following clauses:
1. An isolated polypeptide comprising alanine 2,3-aminomutase activity, wherein the polypeptide comprises a mutated lysine 2,3-aminomutase amino acid sequence, and wherein the mutated lysine 2,3-aminomutase amino acid sequence comprises a P/S11I; N19Y; L/K/R/T26I; E/R30K; L/V32A; K36E; S/T/C52R; L/I53P/H; Y63F'; E/N/D71G; H/L/S85Q;Q/L/E86R; Q/L95M; K/M/Q125L; M128V; Y132H; Q/S141R; A/D/S/MI44G; D179N; K/Q187R; I192V; L228M; D331G/H; M/Q342I; or K/Q/I398E substitution, or combinations thereof, wherein numbering is based on a *Porphyromonas gingivalis* lysine 2,3 aminomutase
2. The isolated polypeptide of clause 1, wherein the mutated lysine 2,3-aminomutase amino acid sequence is a mutated *Bacillus subtilis, Clostridium sticklandii, Fusobacterium nucleatum,* or *Porphyromonas gingivalis* lysine 2,3-aminomutase
3. The isolated polypeptide of clause1, wherein the mutated lysine 2,3-aminomutase amino acid sequence comprises a N19Y, L/T53P/H, H/I/S85Q, D331G/H, and M/Q342T substitution
4. The isolated polypeptide of clause1, wherein the mutated lysine 2,3-aminomutase amino acid sequence comprises a N19Y, E/R30K, L/T53P/H, H/I/S85Q, I192V, D331G/H, and M/Q342T substitution
5. The isolated polypeptide of clause 1, wherein the mutated lysine 2,3-aminomutase amino acid sequence comprises a NI9Y, L/K/R/T26I; E/R30K, L/T53P/H, H/I/S85Q, I192V, D331G/H, and M/Q342T substitution
6. The isolated polypeptide of clause1, wherein the mutated lysine 2,3-aminomutase amino acid sequence comprises a E/R30K, Y63F, Q/L/E86R, Q/L95M, M128V, A/D/S/M144G, L228M, D331G/H, and K/Q/T398E substitution
7. The isolated polypeptide of clause1, wherein the mutated lysine 2,3-aminomutase amino acid sequence comprises a E/R30K, C52R, Q/L95M; M128V, and D331G/H substitution.
8. The isolated polypeptide of clause 1, wherein the mutated lysine 2,3-aminomutase amino acid sequence comprises a E/R30K, K36E, Y63F, Q/L/E86R, Q/L95M, M128V, A/D/S/M144G, D179N, L228M, D331G/H, and K/Q/I398E substitution.
9. The isolated polypeptide of clause1, wherein the mutated lysine 2,3-aminomutase amino acid sequence comprises a E/R30K, Q/L95M, M128V, and D331G/H substitution
10. The isolated polypeptide of clause1, wherein the mutated lysine 2,3-aminomutase amino acid sequence comprises a P/S11T, E/R30K, Q/L95M, M128V, Q/S141R, K/Q187R, and D331G/H substitution
11. The isolated polypeptide of clause 1, wherein the mutated lysine 2,3-aminomutase amino acid sequence comprises a E/R30K, L/V32A, L/T53P/H, E/N/D71G, Q/L95M; K/M/Q125L, M128V, and D331G/H substitution
12. The isolated polypeptide of Clause 1, wherein the mutated lysine 2,3-aminomutase amino acid sequence comprises a Q/L95M, M128V, and D331G/H substitution.
13. The isolated polypeptide of clause 1, wherein the mutated lysine 2,3-aminomutase amino acid sequence comprises a Q/L95M, M128V; Y132H, and D331G/H substitution
14. The isolated polypeptide of clause1, wherein the mutated lysine 2,3-aminomutase amino acid sequence comprises at least 3 of the substitutions.
15. The isolated polypeptide of clause1, wherein the mutated lysine 2,3-aminomutase amino acid sequence comprises 3-11 of the substitutions
16. The isolated polypeptide of clause 1, wherein the polypeptide comprises a sequence having at least 90% sequence identity to SEQ ID NO: 19, 21, 43,45,47,49, or 51
17. The isolated polypeptide of clause1, wherein the polypeptide comprises a sequence having at least 95% sequence identity to SEQ ID NO: 19, 21, 43, 45, 47, 49, or 51.
18. The isolated polypeptide of clause1, wherein the polypeptide comprises SEQ ID NO: 19, 21, 43, 45, 47, 49, or 51.
19. The polypeptide of clause 17, wherein the polypeptide comprises 1-10 conservative amino acid substitutions.
20. An isolated nucleic acid comprising a nucleic acid sequence that encodes the isolated polypeptide of clause1.
21. The isolated nucleic acid of clause 20 operably linked to a promoter sequence
22. The isolated nucleic acid of clause 20, wherein the nucleic acid comprises a sequence having at least 90% identity to SEQ ID NO: 18, 20, 42, 44, 46, 48, or 50
23. The isolated nucleic acid of clause20, wherein the nucleic acid comprises a sequence having at least 95% identity to SEQ ID NO: 18, 20, 42, 44, 46, 48, or 50
24. The isolated nucleic acid of clause 22, wherein the nucleic acid sequence includes one or more substitutions which results in 1-10- conservative amino acid substitutions
25. The isolated nucleic acid of clause 20, wherein the nucleic acid comprises SEQ ID NO: 18, 20, 42, 44, 46, 48, or 50
26. A vector comprising the isolated nucleic acid of clause 20
27. A recombinant nucleic acid comprising the isolated nucleic acid of clause20.
28. A cell transformed with the recombinant nucleic acid of clause 27.
29. The cell of clause 28, wherein the cell is a prokaryotic cell.
30. The cell of clause 29, wherein the prokaryotic cell is a *Lactobacillus, Lactococcus, Bacillus,* or *Escherichia* cell.
31. The cell of clause 28, wherein the cell is a plant cell, bacterial cell, yeast cell, or fungal cell.
32. A plant comprising the cell of clause 31
33. A transgenic plant comprising the recombinant nucleic acid of clause27.
34. The cell of clause 28, wherein the cell comprises alanine 2,3-aminomutase activity and produces beta-alanine from alpha-alanine.
35. The cell of clause 28, wherein the isolated nucleic acid sequence comprises a sequence having at least 90% identity to SEQ ID NO: 18, 20, 42, 44, 46, 48, or 50.
36. The cell of clause 28, wherein the isolated nucleic acid sequence comprises SEQ ID NO: 18, 20, 42,44,46,48, or 50.
37. The cell of clause28, wherein the cell produces 3-hydroxypropionic acid (3-HP)
38. The cell of clause37, wherein the cell further comprises:
   pyruvate/2-oxoglutarate aminotransferase activity;
   beta-alanine/2-oxoglutarate aminotransferase activity; and
   3-hydroxypropionate dehydrogenase activity.
39. The cell of clause38, wherein the cell further comprises lipase or esterase activity.
40. The cell of clause 39, wherein the cell produces an ester of 3-HP
41. The cell of clause 40, wherein the ester of 3-HP is methyl 3-hydroxypropionate, ethyl 3-hydroxypropionate, propyl 3-hydroxypropionate, butyl 3-hydroxypnopionate, or 2-ethylhexyl 3-hydroxypropionate.
42. The cell of clause38, wherein the cell further comprises poly hydroxyacid synthase activity
43. The cell of clause42, wherein the cell produces polymerized 3-HP.
44. The cell of clause 38, wherein the cell further comprises a nucleic acid molecule encoding a peptide having alcohol dehydrogenase activity, a nucleic acid molecule encoding a peptide having aldehyde dehydrogenase activity or both
45. The cell of clause 44, wherein the cell produces 1,3-propanediol.
46. The cell of clause 28, wherein the cell further comprises:
   alpha-ketopantoate hydroxymethyltransferase activity;
   alpha-ketopantoate reductase activity; and
   pantothenate synthase activity.
47. The cell of clause46, wherein the cell produces pantothenate.
48. The cell of clause46, wherein the cell further comprises:
   pantothenate kinase activity;
   4'-phosphopantethenoyl-1-cysteine synthetase activity;
   4'-phosphopantothenoylcysteine decarboxylase activity;
   ATP:4'-phosphopantetheine adenyltransferase activity; and
   dephospho-CoA kinase activity.
49. The cell of clause 48, wherein the cell produces coenzyme A (CoA)
50. A transformed cell comprising at least one exogenous nucleic acid molecule, wherein the at least one exogenous nucleic acid molecule comprises a nucleic acid sequence that encodes the polypeptide of clause1
51. The transformed cell of clause50, wherein the cell produces beta-alanine from alpha-alanine.
52. The cell of clause 51, wherein the cell produces 3-HP, 1,3-propanediol, pantothenate, CoA, or combinations thereof.
53. A method of producing a polypeptide comprising alanine 2,3-aminomutase activity, comprising culturing the cell of clause 28 under conditions that allow the cell to produce the polypeptide comprising alanine 2,3-aminomutase activity.
54. A method for making beta-alanine from alpha-alanine, comprising culturing the cell of clause28 under conditions that allow the cell to make beta-alanine from alpha-alanine.
55. The method of cfause54, wherein the cell comprises at least one exogenous nucleic acid molecule that encodes an alanine 2,3-aminomutase, wherein the alanine 2,3-aminomutase is capable of producing the beta-alanine from the alpha-alanine.
56. The method of clause 54, wherein the cell is a prokaryotic cell.
57. The method of clause 56, wherein the cell comprises a functional deletion of *panD*.
58. A method for making 3-HP, comprising culturing the cell of clause38 under conditions wherein the cell produces the 3-HP.
59. The method of clause58, wherein the cell comprises at least one exogenous nucleic acid that encodes an alanine 2,3-aminomutase such that the 3-HP is produced from beta-alanine, wherein the alanine 2,3-aminomutase produces beta-alanine from alpha-alanine.
60. A method for making an ester of 3-HP, comprising culturing the cell of clause39 under conditions wherein the cell produces the ester of 3-HP.
61. A method for making polymerized 3-HP, comprising culturing the cell of clause 42 under conditions wherein the cell produces the polymerized 3-HP.
62. A method for making 1,3-propanediol, comprising culturing the cell of clause 44 under conditions wherein the cell produces the 1,3-propanediol.
63. A method for making pantothenate, comprising culturing the cell of clause46 under conditions wherein the cell produces the pantothenate.
64. A method for making CoA comprising culturing the cell of clause 48 under conditions wherein the cell produces the CoA.
65. A method for making 3-HP, comprising:
   purifying beta-alanine from the cell of clause 28;
   contacting the beta-alanine with a polypeptide comprising beta-alanine/2-oxoglutarate aminotransferase activity to form 3-oxopropionate; and
   contacting the 3-oxopropionate with a polypeptide comprising 3-hydroxypropionate dehydrogenase activity to make 3-HP.
66. A method for making 3-P, comprising:
   transfecting the cell of clause 28 with a nucleic acid molecule encoding a polypeptide comprising pyruvate/2-oxoglutarate aminotransferase activity, with a nucleic acid molecule encoding a polypeptide comprising beta-alanine/2-oxoglutarate aminotransferase activity, and with a nucleic acid molecule encoding a polypeptide comprising 3-hydroxypropionate dehydrogenase activity; and
   culturing the transfected cell to allow the transfected cell to make 3-HP.
67. A method for making 1,3-propanediol from 3-HP, comprising:
   making 3-HP using the method of clause 65;
   contacting the 3-HP with a polypeptide comprising alcohol dehydrogenase activity and a polypeptide comprising aldehyde dehydrogenase activity to make 1,3-propanediol
68. A method for making 1,3-propanediol, comprising:
   transfecting the cell of clause 28 with a nucleic acid molecule encoding a polypeptide comprising pyruvate/2-oxoglutarate aminotransferase activity, with a nucleic acid molecule encoding a polypeptide comprising beta-alanine/2-oxoglutarate aminotransferase activity, with a nucleic acid molecule encoding a polypeptide comprising 3-hydroxypropionate dehydrogenase activity, with a nucleic acid encoding a polypeptide comprising aldehyde dehydrogenase activity, and with a nucleic acid encoding a polypeptide comprising alcohol dehydrogenase activity; and
   culturing the transfected cell to allow the transfected cell to make 1,3-propanediol.
69. A method for making pantothenate, comprising:
   purifying beta-alanine from the cell of clause28; and
   contacting the beta-alanine with alpha-ketopantoate hydroxymethyltransferase, alpha-ketopantoate reductase, and pantothenate synthase to make pantothenate.
70. A method for making pantothenate, comprising:
   transfecting the cell of clause28 with a nucleic acid molecule encoding a polypeptide comprising alpha-ketopantoate hydroxymethyltransferase activity, a nucleic acid molecule encoding a polypeptide comprising alpha-ketopantoate reductase activity, and a nucleic acid molecule encoding a polypeptide comprising pantothenate synthase activity; and
   culturing the transfected cell to allow the transfected cell to make pantothenate.
71. A method for making CoA, comprising:
   purifying beta-alanine from the cell of clause 28; and
   contacting the beta-alanine with alpha-ketopantoate hydroxymethyltransferase, alpha-ketopantoate reductase, and pantothenate synthase to make pantothenate; and contacting the pantothenate with pantothenate kinase, 4'-phosphopantethenoyl-1-cysteine synthetase, 4'-phosphopantothenoylcysteine decarboxylase, ATP:4-phosphopantetheine adenyltransferase, and dephospho-CoA kinase to make CoA.
72. A method for making CoA, comprising:
   transfecting the cell of clause 28 with a nucleic acid molecule encoding a polypeptide comprising alpha ketopantoate hydroxymethyltransferase activity, a nucleic acid molecule encoding a polypeptide comprising alpha-ketopantoate reductase activity, a nucleic acid molecule encoding a polypeptide comprising pantothenate synthase activity, a nucleic acid molecule encoding a polypeptide comprising pantothenate kinase activity, a nucleic acid molecule encoding a polypeptide comprising 4-phosphopantethenoyl-1-cysteine synthetase activity, a nucleic acid molecule encoding a polypeptide comprising 4'-phosphopantothenoylcysteine decarboxylase activity, a nucleic acid molecule encoding a polypeptide comprising ATP:4'-phosphopantetheine adenyltransferase activity, and a nucleic acid molecule encoding a polypeptide comprising dephospho-CoA kinase activity; and
   culturing the transfected cell to allow the transfected cell to make pantothenate.
73. A specific binding agent that specifically binds to the polypeptide of clause 1.

## Claims

1. An isolated polypeptide comprising alanine 2,3-aminomutase activity, wherein the polypeptide comprises a mutated lysine 2,3-aminomutase amino acid sequence, and wherein the mutated lysine 2,3-aminomutase amino acid sequence comprises a E/R30K substitution, wherein numbering is based on a *Porphyromonas gingivalis* lysine 2,3 aminomutase.

2. The isolated polypeptide of claim 1, wherein:
(a) the mutated lysine 2,3-aminomutase amino acid sequence is a mutated *Bacillus subtilis, Clostridium sticklandii, Fusobacterium nucleatum,* or *Porphyromonas gingivalis* lysine 2,3-aminomutase; preferably, wherein the mutated lysine 2, 3-aminomutase amino acid sequence further comprises a N19Y, L/T53P/H, H/I/S85Q, I192V, D331G/H, and M/Q342T substitution; or
(b) the polypeptide comprises a sequence having at least 60%, at least 70%, at least 80%, at least 90% or at least 95% sequence identity to SEQ ID NO: 19, 21, 43, 45, 47, 49, or 51; preferably wherein the polypeptide comprises SEQ ID NO: 19, 21, 43, 45, 47, 49, or 51; more preferably, wherein the polypeptide comprises 1-10 conservative amino acid substitutions.

3. An isolated nucleic acid comprising a nucleic acid sequence that encodes the isolated polypeptide of claim 1; preferably, wherein the isolated nucleic acid is operably linked to a promoter sequence; more preferably, wherein the nucleic acid comprises a sequence having at least 60%, at least 70%, at least 80%, at least 90% or at least 95% sequence identity to SEQ ID NO: 18, 20, 42, 44, 46, 48, or 50; yet more preferably, wherein the nucleic acid comprises SEQ ID NO: 18, 20, 42, 44, 46, 48, or 50.

4. The isolated nucleic acid of claim 3, wherein the nucleic acid sequence includes one or more substitutions which results in 1-10 conservative amino acid substitutions.

5. A vector comprising the isolated nucleic acid of claims 3 or 4.

6. A recombinant nucleic acid comprising the isolated nucleic acid of claims 3 or 4.

7. A cell transformed with the recombinant nucleic acid of claim 6; preferably, wherein the cell is a prokaryotic cell, and/or wherein the prokaryotic cell is a *Lactobacillus, Lactococcus, Bacillus,* or *Escherichia* cell; or, wherein the cell is a plant cell, bacterial cell, yeast cell, or fungal cell.

8. A plant comprising the cell of claim 7, wherein the cell is a plant cell, bacterial cell, yeast cell, or fungal cell.

9. The cell of claim 7, wherein:
(a) the cell comprises alanine 2,3-aminomutase activity and produces beta-alanine from alpha-alanine; or, wherein the cell produces 3-hydroxypropionic acid (3-HP), an ester of 3-HP, and/or polymerized 3-HP; preferably, wherein the cell has:
pyruvate/2-oxoglutarate aminotransferase activity;
beta-alanine/2-oxoglutarate aminotransferase activity; and
3-hydroxypropionate dehydrogenase activity, and/or lipase or esterase activity,
and/or poly hydroxyacid synthase activity; more preferably, wherein the cell further comprises a nucleic acid molecule encoding a peptide having alcohol dehydrogenase activity, a nucleic acid molecule encoding a peptide having aldehyde dehydrogenase activity or both; or
(b) the cell further comprises:
alpha-ketopantoate hydroxymethyltransferase activity;
alpha-ketopantoate reductase activity; and
pantothenate synthase activity, and/or
pantothenate kinase activity;
4'-phosphopantethenoyl-1-cysteine synthetase activity;
4'-phosphopantothenoylcysteine decarboxylase activity;
ATP:4'-phosphopantetheine adenyltransferase activity; and
dephospho-CoA kinase activity.

10. A transformed cell comprising at least one exogenous nucleic acid molecule, wherein the at least one exogenous nucleic acid molecule comprises a nucleic acid sequence that encodes the polypeptide of claim 1; preferably, wherein the cell produces beta-alanine from alpha-alanine, and/or wherein the cell produces 3-HP, 1,3-propanediol, pantothenate, CoA, or combinations thereof.

11. A method of producing a polypeptide comprising alanine 2,3-aminomutase activity, comprising culturing the cell of claim 7 under conditions that allow the cell to produce the polypeptide comprising alanine 2,3-aminomutase activity.

12. A method for making beta-alanine from alpha-alanine, comprising culturing the cell of claim 7 under conditions that allow the cell to make beta-alanine from alpha-alanine; preferably, wherein the cell comprises at least one exogenous nucleic acid molecule that encodes an alanine 2,3-aminomutase, wherein the alanine 2,3-aminomutase is capable of producing the beta-alanine from the alpha-alanine; more preferably, wherein the cell is a prokaryotic cell; yet more preferably, wherein the cell comprises a functional deletion of *panD.*

13. A method for making 3-HP and/or an ester of 3-HP and/or polymerized 3-HP comprising culturing the cell of claim 7 under conditions wherein the cell produces the 3-HP, and/or wherein the cell produces an ester of 3-HP and/or wherein the cell produces polymerized 3-HP; preferably, wherein the cell comprises at least one exogenous nucleic acid that encodes an alanine 2,3-aminomutase such that the 3-HP is produced from beta-alanine, wherein the alanine 2,3-aminomutase produces beta-alanine from alpha-alanine.

14. A method for making 3-HP, comprising:
(a) purifying beta-alanine from the cell of claim 7;
contacting the beta-alanine with a polypeptide comprising beta-alanine/2-oxoglutarate aminotransferase activity to form 3-oxopropionate; and contacting the 3-oxopropionate with a polypeptide comprising 3-hydroxypropionate dehydrogenase activity to make 3-HP; or
(b) transfecting the cell of claim 7 with a nucleic acid molecule encoding a polypeptide comprising pyruvate/2-oxoglutarate aminotransferase activity, with a nucleic acid molecule encoding a polypeptide comprising beta-alanine/2-oxoglutarate aminotransferase activity, and with a nucleic acid molecule encoding a polypeptide comprising 3-hydroxypropionate dehydrogenase activity; and
culturing the transfected cell to allow the transfected cell to make 3-HP.

15. A monoclonal or polyclonal antibody that specifically binds to a polypeptide of claim 1.
